# EUROPEAN PATENT SPECIFICATION

(11) **EP 2 176 250 B1**
(45) Date of publication and mention of the grant of the patent: **26.10.2011**
(21) Application number: 08784892.5
(22) Date of filing: 18.07.2008
(51) Int. Cl.: C07D 401/04, C07D 401/14, C07D 417/14, C07D 453/02, A61K 31/44, A61P 3/04, A61P 3/10, A61P 15/00, A61P 35/00, A61P 25/00, A61P 25/22, A61P 25/24

(54) **SUBSTITUTED HETEROARYLPIPERIDINE DERIVATIVES AS MELANOCORTIN-4 RECEPTOR MODULATORS**
SUBSTITUIERTE HETEROARYLPIPERIDINDERIVATE ALS MODULATOREN DES MELANOCORTIN-4-REZEPTORS
DÉRIVÉS D'HÉTÉROARYLPIPÉRIDINE SUBSTITUÉS UTILISÉS COMME MODULATEURS DU RÉCEPTEUR 4 DE LA MÉLANOCORTINE

(30) Priority: 19.07.2007 US 950676 P; 19.07.2007 EP 07014219
(43) Date of publication of application: 21.04.2010
(73) Proprietor: Santhera Pharmaceuticals (Schweiz) AG, 4410 Liestal (CH)
(72) Inventor: HERZNER, Holger, 79595 Rümmingen (DE); SOEBERDT, Michael, 79618 Rheinfelden (DE); WEYERMANN, Philipp, CH-4450 Sissach (CH); NORDHOFF, Sonja, CH-4144 Arlesheim (CH); FEURER, Achim, 79424 Auggen (DE); DEPPE, Holger, CH-4052 Basel (CH); SIENDT, Hervé, F-68220 Ranspach-le-Haut (FR); TERINEK, Miroslav, CH-4416 Bubendorf (CH); RUMMEY, Christian, CH-4058 Basel (CH); HENNEBÖHLE, Marco, 79618 Rheinfelden (DE)
(74) Representative: Grünecker, Kinkeldey, Stockmair & Schwanhäusser
(86) International application number: PCT/EP2008/005913
(87) International publication number: WO 2009/010299

(56) References cited:
- WO-A-2004/024720
- WO-A-2005/047253
- WO-A-2007/115798

## Description

### Field of the Invention

The present invention relates to substituted heteroarylpiperidine derivatives as melanocortin-4 receptor modulators. Depending on the structure and the stereochemistry the compounds of the invention are either selective agonists or selective antagonists of the human melanocortin-4 receptor (MC-4R). The agonists can be used for the treatment of disorders and diseases such as obesity, diabetes and sexual dysfunction, whereas the antagonists are useful for the treatment of disorders and diseases such as cancer cachexia, muscle wasting, anorexia, amyotrophic lateral sclerosis, anxiety and depression. Generally all diseases and disorders where the regulation of the MC-4R is involved can be treated with the compounds of the invention.

### Background of the Invention

Melanocortins (MCs) stem from pro-opiomelanocortin (POMC) via proteolytic cleavage. These peptides, adrenocorticotropic hormone (ACTH), α-melanocyte-stimulating hormone (α-MSH), β-MSH and γ-MSH, range in size from 12 to 39 amino acids. The most important endogenous agonist for central MC-4R activation appears to be the tridecapeptide α-MSH. Among MCs, it was reported that α-MSH acts as a neurotransmitter or neuromodulator in the brain. MC peptides, particularly α-MSH, have a wide range of effects on biological functions including feeding behavior, pigmentation and exocrine function. The biological effects of α-MSH are mediated by a sub-family of 7-transmembrane G-protein-coupled receptors, termed melanocortin receptors (MC-Rs). Activation of any of these MC-Rs results in stimulation of cAMP formation.

To date, five distinct types of receptor subtype for MC (MC-1 R to MC-5R) have been identified and these are expressed in different tissues.

MC-1 R was first found in melanocytes. Naturally occurring inactive variants of MC-1 R in animals were shown to lead to alterations in pigmentation and a subsequent lighter coat color by controlling the conversion of phaeomelanin to eumelanin through the control of tyrosinase. From these and other studies, it is evident that MC-1 R is an important regulator of melanin production and coat color in animals and skin color in humans.

The MC-2R is expressed in the adrenal gland representing the ACTH receptor. The MC-2R is not a receptor for α-MSH but is the receptor for the adrenocorticotropic hormone I (ACTH I).

The MC-3R is expressed in the brain (predominately located in the hypothalamus) and peripheral tissues like gut and placenta, and knock-out studies have revealed that the MC-3R may be responsible for alterations in feeding behavior, body weight and thermogenesis.

The MC-4R is primarily expressed in the brain. Overwhelming data support the role of MC-4R in energy homeostasis. Genetic knock-outs and pharmacologic manipulation of MC-4R in animals have shown that agonizing the MC-4R causes weight loss and antagonizing the MC-4R produces weight gain (A. Kask et al., "Selective antagonist for the melanocortin-4 receptor (HS014) increases food intake in free-feeding rats," Biochem. Biophys. Res. Commun., 245: 90-93 (1998)).

MC-5R is ubiquitously expressed in many peripheral tissues including white fat, placenta and a low level of expression is also observed in the brain. However its expression is greatest in exocrine glands. Genetic knock-out of this receptor in mice results in altered regulation of exocrine gland function, leading to changes in water repulsion and thermoregulation. MC-5R knockout mice also reveal reduced sebaceous gland lipid production (Chen et al., Cell, 91: 789-798 (1997)).

Attention has been focused on the study of MC-3R and MC-4R modulators and their use in treating body weight disorders, such as obesity and anorexia. However, evidence has shown that the MC peptides have potent physiological effects besides their role in regulating pigmentation, feeding behavior and exocrine function. In particular, α-MSH recently has been shown to induce a potent anti-inflammatory effect in both acute and chronic models of inflammation including inflammatory bowel-disease, renal ischemia/reperfusion injury and endotoxin-induced hepatitis. Administration of α-MSH in these models results in substantial reduction of inflammation-mediated tissue damage, a significant decrease in leukocyte infiltration and a dramatic reduction in elevated levels of cytokines and other mediators to near baseline levels. Recent studies have demonstrated that the anti-inflammatory actions of α-MSH are mediated by MC-1 R. The mechanism by which agonism of MC-1 R results in an anti-inflammatory response is likely through inhibition of the pro-inflammatory transcription activator, NF-κB. NF-κB is a pivotal component of the pro-inflammatory cascade, and its activation is a central event in initiating many inflammatory diseases. Additionally, anti-inflammatory actions of α-MSH may be, in part, mediated by agonism of MC-3R and/or MC-5R.

A specific single MC-R that may be targeted for the control of obesity has not yet been identified, although evidence has been presented that MC-4R signaling is important in mediating feeding behavior (S.Q. Giraudo et al., "Feeding effects of hypothalamic injection of melanocortin-4 receptor ligands," Brain Research, 80: 302-306 (1998)). Further evidence for the involvement of MC-Rs in obesity includes: 1) the agouti (A^{vy}) mouse which ectopically expresses an antagonist of the MC-1R, MC-3R and MC-4R is obese, indicating that blocking the action of these three MC-R's can lead to hyperphagia and metabolic disorders; 2) MC-4R knockout mice (D. Huszar et al., Cell, 88: 131-141 (1997)) recapitulate the phenotype of the agouti mouse and these mice are obese; 3) the cyclic heptapeptide melanotanin II (MT-II) (a non-selective MC-1 R, -3R, -4R, and -5R agonist) injected intracerebroventricularly (ICV) in rodents, reduces food intake in several animal feeding models (NPY, ob/ob, agouti, fasted) while ICV injected SHU-9119 (MC-3R and 4R antagonist; MC-1 R and -5R agonist) reverses this effect and can induce hyperphagia; 4) chronic intraperitoneal treatment of Zucker fatty rats with an α-NDP-MSH derivative (HP-228) has been reported to activate MC-1R, -3R, -4R, and -5R and to attenuate food intake and body weight gain over a 12 week period (I. Corcos et al., "HP-228 is a potent agonist of melanocortin receptor-4 and significantly attenuates obesity and diabetes in Zucker fatty rats", Society for Neuroscience Abstracts, 23: 673 (1997)).

MC-4R appears to play a role in other physiological functions as well, namely controlling grooming behavior, erection and blood pressure. Erectile dysfunction denotes the medical condition of inability to achieve penile erection sufficient for successful intercourse. The term "impotence" is often employed to describe this prevalent condition. Synthetic melanocortin receptor agonists have been found to initiate erections in men with psychogenic erectile dysfunction (H. Wessells et al., "Synthetic Melanotropic Peptide Initiates Erections in Men With Psychogenic Erectile Dysfunction: Double-Blind, Placebo Controlled Crossover Study", J. Urol., 160: 389-393, (1998)). Activation of melanocortin receptors of the brain appears to cause normal stimulation of sexual arousal. Evidence for the involvement of MC-R in male and/or female sexual dysfunction is detailed in WO 00/74679.

Diabetes is a disease in which a mammal's ability to regulate glucose levels in the blood is impaired because the mammal has a reduced ability to convert glucose to glycogen for storage in muscle and liver cells. In Type I diabetes, this reduced ability to store glucose is caused by reduced insulin production. "Type II diabetes" or "Non-Insulin Dependent Diabetes Mellitus" (NIDDM) is the form of diabetes which is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle, and inadequate insulin repression of lipolysis in adipose tissue and of glucose production and secretion in liver. When these cells become desensitized to insulin, the body tries to compensate by producing abnormally high levels of insulin and hyperinsulemia results. Hyperinsulemia is associated with hypertension and elevated body weight. Since insulin is involved in promoting the cellular uptake of glucose, amino acids and triglycerides from the blood by insulin sensitive cells, insulin insensitivity can result in elevated levels of triglycerides and LDL which are risk factors in cardiovascular diseases. The constellation of symptoms which includes hyperinsulemia combined with hypertension, elevated body weight, elevated triglycerides and elevated LDL, is known as Syndrome X. MC-4R agonists might be useful in the treatment of NIDDM and Syndrome X.

Among MC receptor subtypes, the MC4 receptor is also of interest in terms of the relationship to stress and the regulation of emotional behavior, as based on the following findings. Stress initiates a complex cascade of responses that include endocrine, biochemical and behavioral events. Many of these responses are initiated by release of corticotropin-releasing factor (CRF) (M.J. Owen and C.B. Nemeroff, "Physiology and pharmacology of corticotrophin releasing factor." Pharmacol. Rev. 43: 425-473 (1991)). In addition to activation of the brain CRF system, there are several lines of evidence that melanocortins (MCs), which stem from proopiomelanocortin by enzymatic processing, mediate important behavioral and biochemical responses to stress and, consequently, stress-induced disorders like anxiety and depression (Shigeyuki Chaki et al, "Anxiolytic-Like and Antidepressant-Like Activities of MCL0129 (1-[(S)-2-(4-Fluorophenyl)-2-(4-isopropylpiperadin-1-yl)ethyl]-4- [4-(2-methoxynaphthalen-1-yl)butyl]piperazine), a Novel and Potent Nonpeptide Antagonist of the Melanocortin-4 Receptor", J. Pharm. Exp. Ther. 304(2), 818-826 (2003)).

Chronic diseases, such as malignant tumors or infections, are frequently associated with cachexia resulting from a combination of a decrease in appetite and a loss of lean body mass. Extensive loss of lean body mass is often triggered by an inflammatory process and is usually associated with increased plasma levels of cytokines (e.g. TNF-α), which increase the production of α-MSH in the brain. Activation of MC4 receptors in the hypothalamus by α-MSH reduces appetite and increases energy expenditure. Experimental evidence in tumor bearing mice suggests that cachexia can be prevented or reversed by genetic MC4 receptor knockout or MC4 receptor blockade. The increased body weight in the treated mice is attributable to a larger amount of lean body mass, which mainly consists of skeletal muscle (D.L. Marks et al. "Role of the central melanocortin system in cachexia." Cancer Res. 61: 1432-1438 (2001)).

Clinical observations indicate, that progression of amytrophic lateral sclerosis (ALS) might be inversely correlated with body weight (e.g. Ludolph AC, Neuromuscul Disord. (2006) 16 (8):530-8). Accordingly, MC-4R inhibitors could be used to treat ALS patients.

Modulators of the melanocortin receptor are already known from the literature. WO 2004/024720 A1 describes piperazine urea derivatives which are selective agonists of the human melanocortin-4 receptor and as such they are claimed to be useful in the treatment of prevention of obesity-related disorders.

WO 2005/047253 A1 describes 4,4-disubstituted piperidine derivatives which are postulated to function as melanocortin receptor agonists.

Substituted piperidine derivatives are also described in DE 103 00973 which relates to carboxylic acids and esters having a piperidine ring or a piperazine ring as the central core of the molecule and wherein the core is further substituted in the para-position by a 5-7-membered heterocycle, a phenyl ring, a pyridine ring or a thiazole ring. Said rings are optionally substituted by an ester group. The compounds are used in the preparation of a medicament for the treatment of headaches, non-insulin dependent diabetes mellitus (NIDDM), cardiovascularic diseases, morphintolerance, diseases of the skin, inflammations, allergic rhinitis, asthma, diseases with vascular dilatation and, consequently, with reduced blood circulation in tissues, acute or preemptive treatment of menopausal hot flashes of women with an estrogen deficiency or for the treatment of pain.

WO 2007/115798 A1 relates to substituted phenylpiperidine derivatives which act as melanocortin-4 receptor modulators and, thus, are useful in the treatment of diseases and disorders where the regulation of the MC-4R is involved.

In view of the unresolved deficiencies in treatment of various diseases and disorders as discussed above, it is an object of the present invention to provide novel substituted heteroarylpiperidine derivatives with improved ability to cross the blood brain barrier, which are useful as melanocortin-4 receptor modulators to treat cancer cachexia, muscle wasting, anorexia, anxiety, depression, obesity, diabetes, sexual dysfunction, amyotrophic lateral sclerosis and other diseases with MC-4R involvement.

### Summary of the Invention

The present invention relates to substituted heteroarylpiperidine derivatives of structural formula (I) 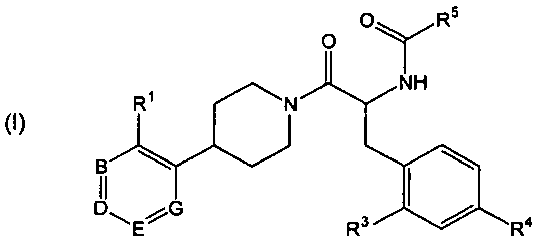 wherein R¹, R³, R⁴, R⁵, B, D, E and G are defined as described below.

The heteroarylpiperidine derivatives of structural formula (I) are effective as melanocortin receptor modulators and are particularly effective as selective melanocortin-4 receptor (MC-4R) modulators. They are therefore useful for the treatment of disorders where the activation or inactivation of the MC-4R are involved. Agonists can be used for the treatment of disorders and diseases such as obesity, diabetes and sexual dysfunction, whereas the antagonists are useful for the treatment of disorders and diseases such as cancer cachexia, muscle wasting, anorexia, amyotrophic lateral sclerosis (ALS), anxiety and depression.

Thus, the present inventions relates to compounds of formula (I) for the treatment and/or prophylaxis of cancer cachexia, muscle wasting, anorexia, amytrophic lateral sclerosis (ALS), anxiety, depression, obesity, diabetes mellitus, male or female sexual dysfunction and erectile dysfunction.

In a further aspect, the invention relates to the use of a compound of formula (I) for the preparation of a medicament for the treatment and/or prophylaxis of cancer cachexia, muscle wasting, anorexia, amytrophic lateral sclerosis (ALS), anxiety, depression, obesity, diabetes mellitus, male or female sexual dysfunction and erectile dysfunction.

The present invention also relates to pharmaceutical compositions comprising the compounds of the present invention and a pharmaceutically acceptable carrier.

### Detailed Description of the Invention

The present invention relates to substituted heteroarylpiperidine derivatives useful as melanocortin receptor modulators, in particular, selective MC-4R agonists and MC-4R antagonists.

The compounds of the present invention are represented by structural formula (I) 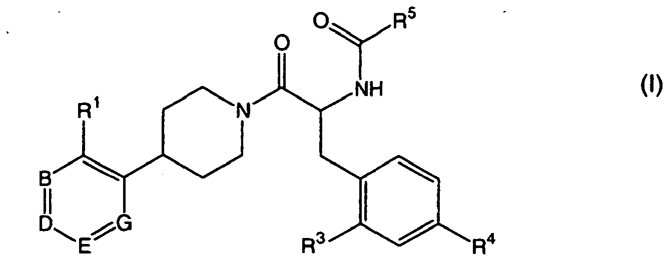 and the enantiomers, diastereomers, tautomers, solvates and pharmaceutically acceptable salts thereof,
wherein
- R¹ is: -N(R¹⁰)-(C(A⁶)₂)ₘ-T
-(C(R⁶)₂)ₗ-T, or
-O-C(R⁶)₂)ₘ-T;
- R⁶ is: independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
- T is: NR⁷R⁸, 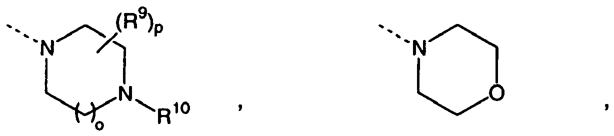 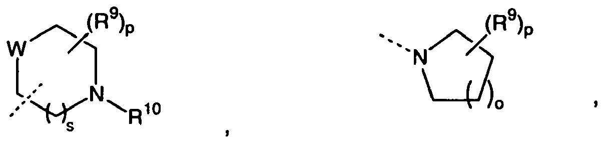 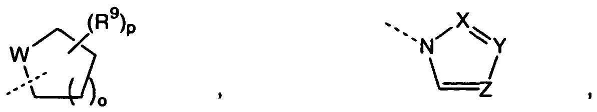 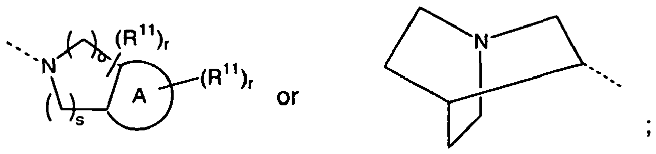
- R⁷ and R⁸: are independently from each other selected from H,
C₁₋₆-alkyl,
C₂₋₆-alkenyl,
C₂₋₆-alkinyl, and
C₂₋₆-alkylene-O-C₁₋₆-alkyl,
wherein each alkyl, alkenyl and alkinyl is optionally substituted by one or more halogen atoms, CN or OH;
- R⁹ is: independently selected from halogen,
CN, OH,
C₁₋₆-alkyl, optionally substituted with 1 to 3 substituents selected from halogen, CN and OH, and
O-C₁₋₆-alkyl, optionally substituted with 1 to 3 substituents selected from halogen, CN and OH,
C₁₋₆-alkylene-O-C₁₋₆-alkyl, optionally substituted with 1 to 3 substituents selected from halogen, CN and OH, or
NR¹²R¹³;
- R¹⁰ is: H, or C₁₋₆-alkyl;
- R¹¹ is: independently selected from halogen, CN,
OH,
C₁₋₆-alkyl, optionally substituted with 1 to 3 substituents selected from halogen, CN and OH,
C₂₋₆-alkenyl,
C₂₋₆-alkinyl,
O-C₁₋₆-alkyl, optionally substituted with 1 to 3 substituents selected from halogen, CN and OH,
C₁₋₆-alkylene-O-C₁₋₆-alkyl, optionally substituted with 1 to 3 substituents selected from halogen, CN and OH,
C₀₋₆-alkyl-C₃₋₆-cycloalkyl,
-OC(O)C₁₋₆-alkyl,
-NH₂,
-NH(C₁₋₆-alkyl), and
-N(C₁₋₆-alkyl)₂;
- R¹² and R¹³: are independently from each other selected from C₁₋₆-alkyl, optionally substituted with OH,
C₂₋₆-alkenyl,
C₂₋₆-alkinyl,
C₂₋₆-alkylene-O-C₁₋₆-alkyl, and
C₂₋₆-alkylene-N-(C₁₋₆-alkyl)₂;
- W is: CH, O or NR¹⁰;
- X is: CH or N;
- Y is: CH or N;
- Z is: CH or N;
- A is: a 3-7-membered saturated, unsaturated or aromatic ring containing 0-2 nitrogen atoms;
- B is: CR² or N;
- G is: CR² or N;
- D is: CR² or N;

- E is: CR² or N;
with the proviso that one or two of the variables B, G, D and E must be N;
- R² is: independently selected from
H,
F,
Cl,
CH₃,
OCH₃, and CF₃;
- R³ is: H,
Cl,
F, or
CH₃;
- R⁴ is: Cl, F or
CH₃;
- R⁵ is: 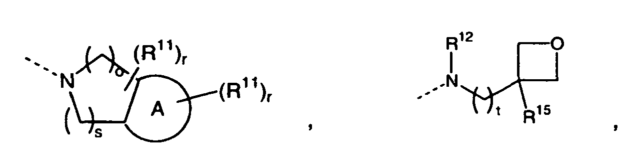 morpholine, optionally substituted by 1 to 3 same or different substituents R¹⁴, 4 to 7 membered, saturated or partially unsaturated heterocycle containing in the ring one nitrogen atom and optionally a further heteroatom selected from O, N and S, wherein heterocycle is optionally substituted by 1 to 4 same or different substituents R¹¹, or
NR¹²R¹³;
- R¹⁴ is: C₁₋₆-alkyl,
C₁₋₆-alkylene-O-C₁₋₆-alkyl,
C₁₋₆-alkylene-OH, C₁₋₆-alkylene-NH₂,
C₁₋₆-alkylene-NH-C₁₋₆-alkyl, or
C₁₋₆-alkylene-N(C₁₋₆-alkyl)₂;
- R¹⁵ is: H or C₁₋₆-alkyl;
- I is: 0, 1, 2, 3, or 4;
- m is: 0, 1, 2, 3, or 4;
- o is: 0, 1, or 2;
- p is: 0, 1, 2, 3, or 4;
- r is: 0, 1, 2, 3, or 4;
- s is: 1, or 2 and
- t is: 0 or 1.

In a preferred embodiment, the compounds of formula (I) are defined as follows:
- R¹ is: -N(R¹⁰)-(C(R⁶)₂)ₘ-T
-(C(R⁶)₂)ₗ-T, or
-O-(C(R⁶)₂)ₘ-T;
- R⁶ is: independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
- T is: NR⁷R⁸,
morpholine, 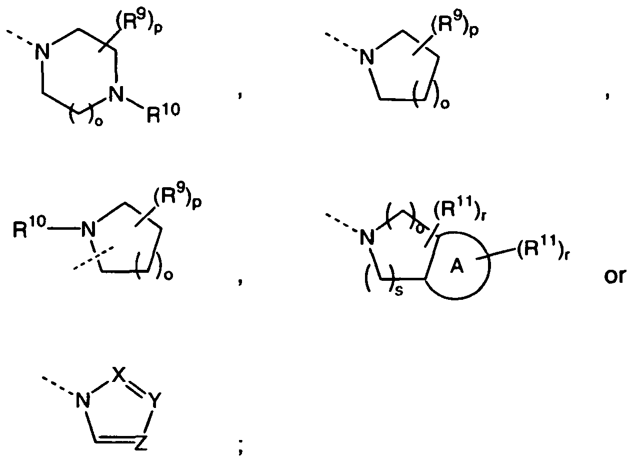
- R⁷ and R⁸: are independently from each other selected from H,
C₁₋₆-alkyl,
C₂₋₆-alkenyl,
C₂₋₆-alkinyl, and
C₂₋₆-alkylene-O-C₁₋₆-alkyl,
wherein each alkyl, alkenyl and alkinyl is optionally substituted by one or more halogen atoms, CN or OH;
- R⁹ is: independently selected from
halogen,
CN,
OH,
C₁₋₆-alkyl, optionally substituted with 1 to 3 substituents selected from halogen, CN and OH, and
O-C₁₋₆-alkyl, optionally substituted with 1 to 3 substituents selected from halogen, CN and OH,
C₁₋₆-alkylene-O-C₁₋₆-alkyl optionally substituted with 1 to 3 substituents selected from halogen, CN and OH;
- R¹⁰ is: H, or C₁-C₆-alkyl;
- R¹¹ is: independently selected from
halogen,
CN,
OH,
C₁₋₆-alkyl, optionally substituted with 1 to 3 substituents selected from halogen, CN and OH,
O-C₁₋₆-alkyl, optionally substituted with 1 to 3 substituents selected from halogen, CN and OH,
C₁₋₆-alkylene-O-C₁₋₆-alkyl, optionally substituted with 1 to 3 substituents selected from halogen, CN and OH,
-NH₂,
-NH(C₁₋₆-alkyl), and
-N(C₁₋₆-alkyl)₂;
- X is: CH or N;
- Y is: CH or N;
- Z is: CH or N;
- A is: a 3-7-membered saturated, unsaturated or aromatic ring containing 0-2 nitrogen atoms;
- B is: CR² or N;
- G is: CR² or N;
- D is: CR² or N;
- E is: CR² or N;
with the proviso that one or two of the variables B, G, D and E must be N;
- R² is: independently selected from
H,
F,
Cl,
CH₃,
OCH₃, and CF₃;
- R³ is: H,
Cl,
F, or
CH₃;
- R⁴ is: Cl or F;
- R⁵ is: 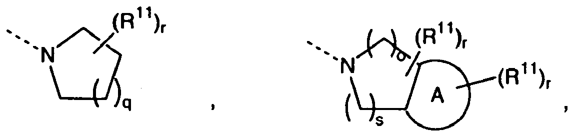 morpholine, optionally substituted by 1 to 3, same or different substituents R¹⁴, or
NR¹²R¹³;
- R¹² and R¹³: are independently from each other selected from
C₁₋₆-alkyl,
C₂₋₆-alkenyl,
C₂₋₆-alkinyl,
C₂₋₆-alkylene-O-C₁₋₆-alkyl, and
C₂₋₆-alkylene-N-(C₁₋₆-alkyl)₂;
- R¹⁴ is: C₁₋₆-alkyl,
C₁₋₆-alkylene-O-C₁₋₆-alkyl,
C₁₋₆-alkylene-OH,
C₁₋₆-alkylene-NH₂,
C₁₋₆-alkylene-NH-C₁₋₆-alkyl, or
C₁₋₆-alkylene-N(C₁₋₆-alkyl)₂;
- l is: 0, 1, 2, 3, or 4;
- m is: 0, 1, 2, 3, or 4;
- o is: 0, 1, or 2;
- p is: 0, 1, 2, 3, or 4;
- q is: 0, 1, 2, or 3;
- r is: 0, 1, 2, 3, or 4 and
- s is: 1, or 2.

Preferably, the compounds according to formula (I) adopt the structural conformation of the following stereoisomer formula (I'): 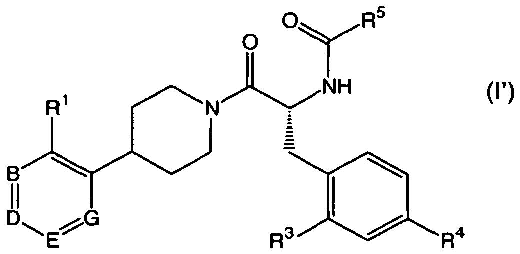 wherein B, G, D, E, R¹, R³, R⁴ and R⁵ are as defined above.

The moiety 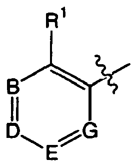 in general formula (I) is selected from the following structures:

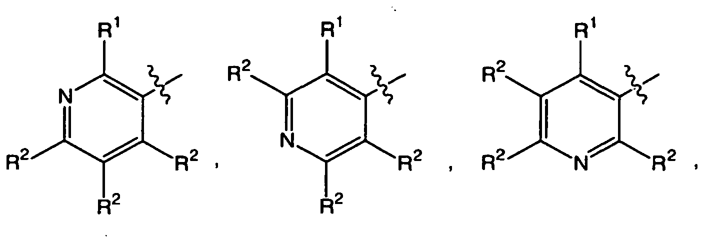

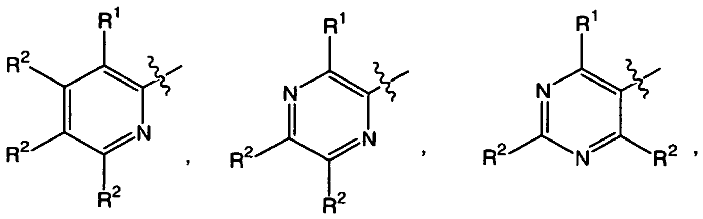

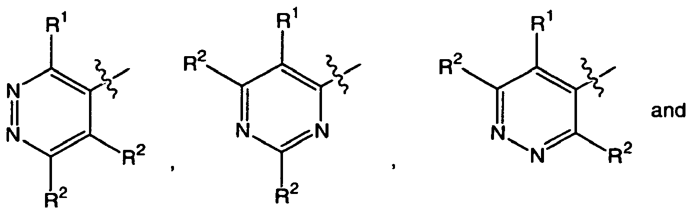

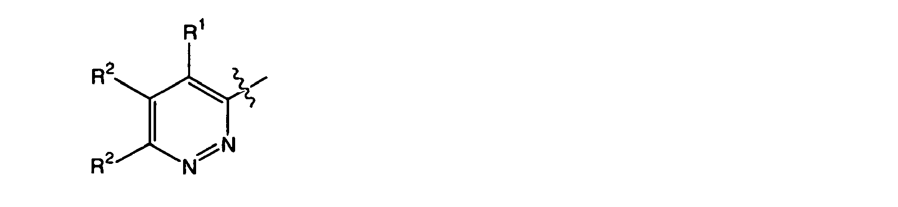

Therein, the variant R² is defined as above. In a preferred embodiment of the present invention, R² represents H, Cl, F or CH₃. More preferred, R² represents H or CH₃.

Preferred embodiments of the moiety 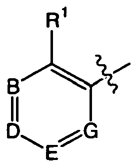 are the following structures:

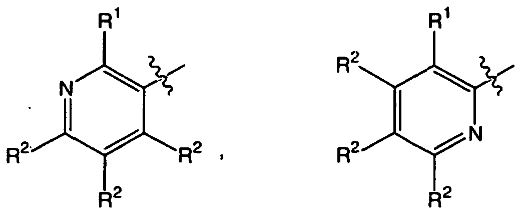

In a preferred embodiment of the present invention, the variant R¹ represents -N(R¹⁰)-(C(R⁶)₂)ₘ-T, -(C(R⁶)₂)ₗ-T, or -O-(C(R⁶)₂)ₘ-T wherein R⁶ and R¹⁰ are preferably independently selected from the group consisting of H and C₁₋₆-alkyl.

It is further preferred that R³ represents H, Cl, or CH₃, more preferably Cl. In an alternative embodiment, R³ preferably represents F.

Preferably, R⁴ represents Cl.

In a preferred embodiment of the present invention, the variant R⁵ represents 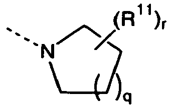 wherein r preferably is 0 or 1 and q preferably assumes the number 1 or 2.

In a further preferred embodiment at least one of R⁷ and R⁸ is selected from the group consisting of C₁₋₆-alkyl, C₂₋₆-alkenyl, C₂₋₆-alkinyl and C₂₋₆-alkylene-O-C₁₋₆-alkyl, more preferably from C₂₋₆-alkenyl, C₂₋₆-alkinyl and C₂₋₆-alkylene-O-C₁₋₆-alkyl.

It is preferred that R⁹ is independently selected from the group consisting of halogen, CN, OH, C₁₋₆-alkyl optionally substituted with 1 to 3 substituents selected from halogen, CN and OH, and O-C₁₋₆-alkyl optionally substituted with 1 to 3 substituents selected from halogen, CN and OH.

The variant I is preferably selected from 2 or 3.

The variant m is preferably selected from 2, 3 or 4, more preferably from 2 or 3.

As regards compounds of formula (I), T is preferably selected from the group consisting of the following radicals:

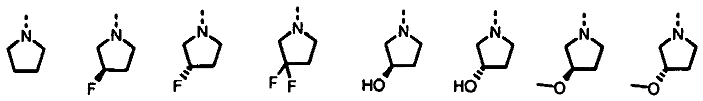

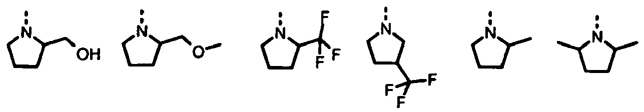

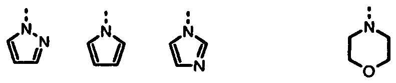

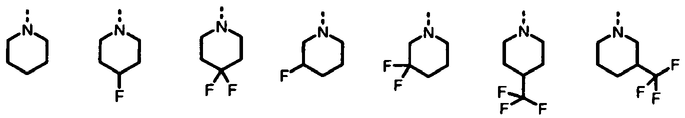

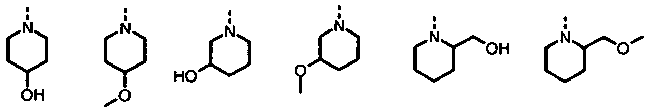

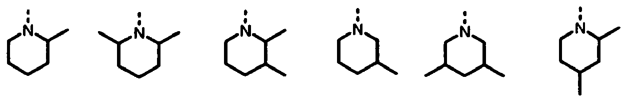

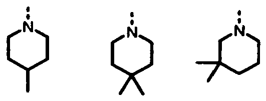

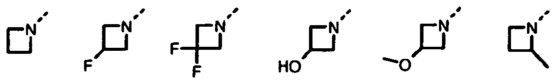

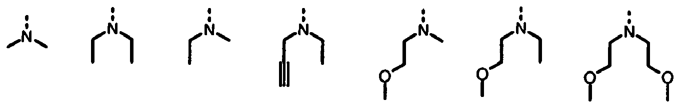

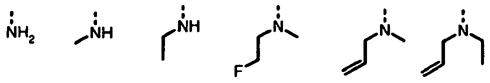

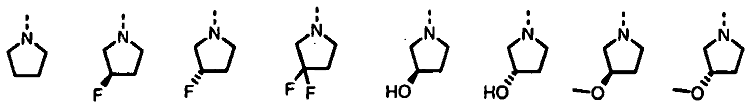

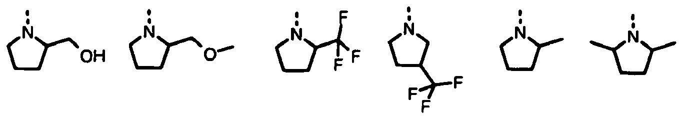

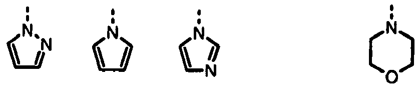

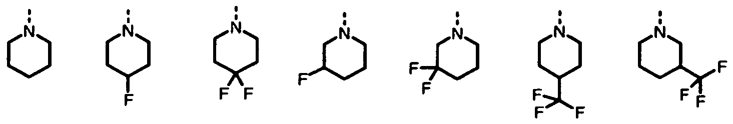

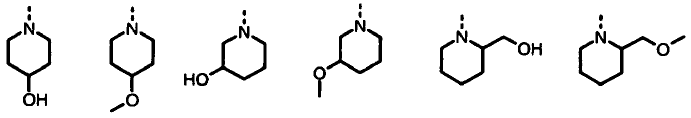

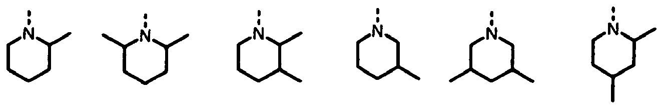

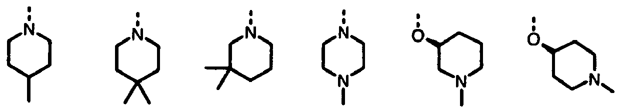

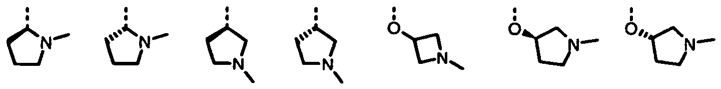

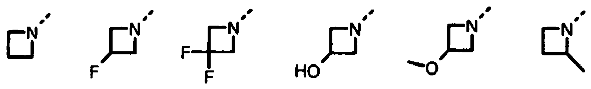

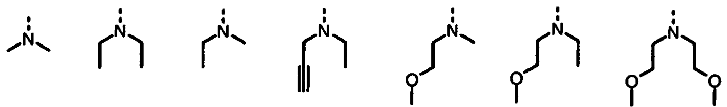

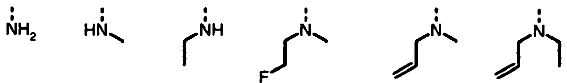

In a further preferred embodiment, R⁵ is preferably selected from the group consisting of

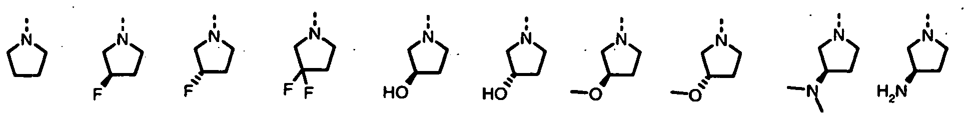

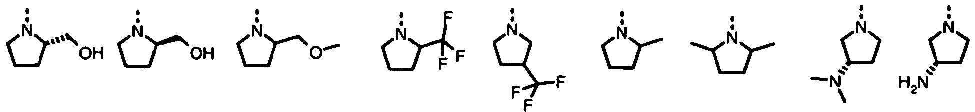

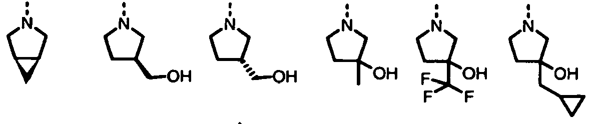

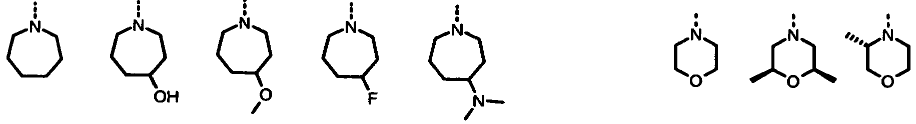

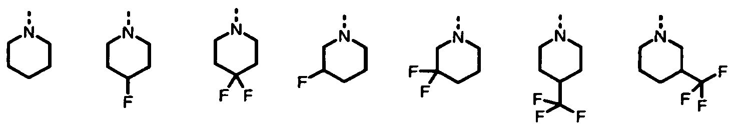

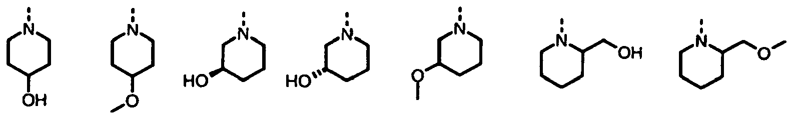

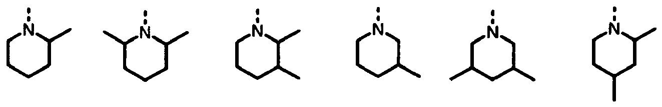

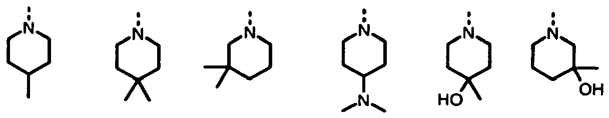

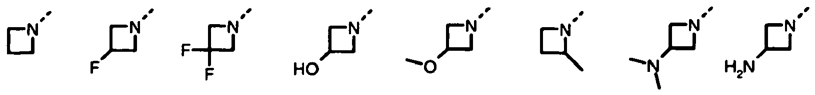

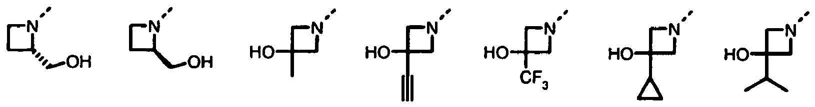

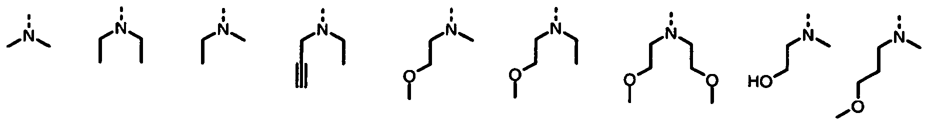

Compounds of the formula (I) in which some or all of the above-mentioned groups have the preferred or more preferred meanings are also an object of the present invention.

In the above and the following, the employed terms have the meaning as described below:

Alkyl is a straight chain or branched alkyl having 1 to 6 carbon atoms, such as methyl, ethyl, n-propyl, isopropyl, n-butyl, isobutyl, tert-butyl, n-pentyl, isopentyl, neopentyl, or hexyl.

Alkenyl is a straight chain or branched alkyl having 2 to 6 carbon atoms and which contains at least one carbon-carbon double bond, such as vinyl, allyl, 1-propenyl, 2-butenyl, 2-methyl-2-butenyl, isopropenyl, pentenyl, or hexenyl.

Alkinyl is a straight chain or branched alkyl having 2 to 6 carbon atoms and which contains at least one carbon-carbon triple bond, such as ethinyl, 1-propinyl, 1-butinyl, 2-butinyl, pentinyl or hexinyl.

A 3-7-membered, saturated, unsaturated or aromatic ring containing 0-2 nitrogen atoms encompasses a 3-7-membered saturated carbocycle such as cyclopropyl, cyclobutyl, cyclopentyl, cyclohexyl or cycloheptyl. Said term further encompasses 3-7-membered unsaturated carbocycles such as cyclobutenyl, cyclopentenyl, cyclohexenyl, cycloheptenyl, cyclohexa-1,4-diene or cycloheptadienes, or aromatic rings such as benzene. Nitrogen-containing, 3-7-membered, saturated, unsaturated or aromatic heterocycles are further encompassed by the above term. Examples thereof include azetidine, pyrrolidine, piperidine, azepane, piperazine, pyridine, pyrimidine, pyrazine, pyrrole, imidazole, and pyrazole.

The compounds of structural formula (I) are effective as melanocortin receptor modulators and are particularly effective as selective modulators of MC-4R. They are therefore useful for the treatment and/or prevention of disorders responsive to the activation and inactivation of MC-4R, such as cancer cachexia, muscle wasting, anorexia, amyotrophic lateral sclerosis, anxiety, depression, obesity, diabetes, sexual dysfunction and other diseases with MC-4R involvement.

The compounds of structural formula (I) are particularly useful as antagonists of MC-4R. Thus, they are preferably used for the preparation of a medicament for the treatment and/or prevention of cancer cachexia, muscle wasting, anorexia, amyotrophic lateral sclerosis, anxiety and depression.

### Optical Isomers - Diastereomers - Geometric Isomers - Tautomers

Compounds of structural formula (I) contain one or more asymmetric centers and can occur as racemates and racemic mixtures, single enantiomers, diastereomeric mixtures and individual diastereomers. The present invention is meant to comprehend all such isomeric forms of the compounds of structural formula (I).

Compounds of structural formula (I) may be separated into their individual diastereoisomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the general formula (I) may be obtained by stereospecific synthesis using optically pure starting materials or reagents of known absolute configuration.

### Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, p-toluenesulfonic, trifluoroacetic acid and the like. Particularly preferred are citric, fumaric, hydrobromic, hydrochloric, maleic, phosphoric, sulfuric and tartaric acids.

It will be understood that, as used herein, references to the compounds of formula (I) are meant to also include the pharmaceutically acceptable salts.

### Utility

The compounds of formula (I) are melanocortin receptor modulators and as such are useful in the treatment, control or prevention of diseases, disorders or conditions responsive to the inactivation of one or more of the melanocortin receptors including, but not limited to, MC-1 R, MC-2R, MC-3R, MC-4R or MC-5R. Such diseases, disorders or conditions include, but are not limited to, cancer cachexia, muscle wasting, anorexia, anxiety, depression, obesity (by reducing appetite, increasing metabolic rate, reducing fat intake or reducing carbohydrate craving), diabetes mellitus (by enhancing glucose tolerance, decreasing insulin resistance) and male and female sexual dysfunction (including impotence, loss of libido and erectile dysfunction).

The compounds of formulas (I) can be further used in the treatment, control or prevention of hypertension, hyperlipidemia, osteoarthritis, cancer, gall bladder disease, sleep apnea, compulsion, neuroses, insomnia/sleep disorder, substance abuse, pain, fever, inflammation, immune-modulation, rheumatoid arthritis, skin tanning, acne and other skin disorders, neuroprotective and cognitive and memory enhancement including the treatment of Alzheimer's disease.

### Administration and Dose Ranges

Any suitable route of administration may be employed for providing a mammal, especially a human with an effective dosage of a compound of the present invention. For example, oral, rectal, topical, parenteral, ocular, pulmonary, nasal and the like may be employed. Dosage forms include tablets, troches, dispersions, suspensions, solutions, capsules, creams, ointments, aerosols and the like. Preferably compounds of formula (I) are administered orally or topically.

The effective dosage of active ingredient employed may vary depending on the particular compound employed, the mode of administration, the condition being treated and the severity of the condition being treated. Such dosage may be ascertained readily by a person skilled in the art.

When treating cancer cachexia, muscle wasting, amyotrophic lateral sclerosis or anorexia generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating obesity, in conjunction with diabetes and/or hyperglycemia, or alone, generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating diabetes mellitus and/or hyperglycemia, as well as other diseases or disorders for which compounds of formula (I) are useful, generally satisfactory results are obtained when the compounds of the present invention are administered at a daily dosage of from about 0.001 milligram to about 100 milligram per kilogram of animal body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

For the treatment of sexual dysfunction, compounds of the present invention are given in a dose range of 0.001 milligram to about 100 milligram per kilogram of body weight, preferably as a single dose orally or as a nasal spray.

### Formulation

The compounds of formula (I) are preferably formulated into a dosage form prior to administration. Accordingly the present invention also includes a pharmaceutical composition comprising a compound of formula (I) and a suitable pharmaceutical carrier.

The present pharmaceutical compositions are prepared by known procedures using well-known and readily available ingredients. In making the formulations of the present invention, the active ingredient (a compound of formula (I)) is usually mixed with a carrier, or diluted by a carrier, or enclosed within a carrier, which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

### Preparation of Compounds of the Invention

When describing the preparation of the present compounds of formula (I), the terms "A moiety", "B moiety" and "C moiety" are used below. This moiety concept is illustrated below:

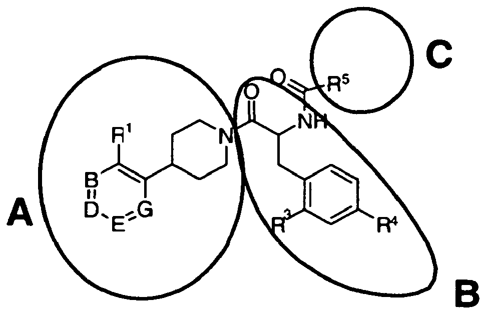

The preparation of the compounds of the present invention may be carried out via sequential or convergent synthetic routes. The skilled artisan will recognize that, in general, the A and B moieties of a compound of formula (I) are connected via amide bonds. The skilled artist can, therefore, readily envision numerous routes and methods of connecting the two moieties via standard peptide coupling reaction conditions.

The phrase "standard peptide coupling reaction conditions" means coupling a carboxylic acid with an amine using an acid activating agent such as EDCI, dicyclohexylcarbodiimide and benzotriazol-1-yloxytris(dimethylamino)-phosphonium hexafluorophosphate, in a inert solvent such as DCM, in the presence of a catalyst such as HOBt. The uses of protective groups for amine and carboxylic acids to facilitate the desired reaction and minimize undesired reactions are well documented. Conditions required to remove protecting groups which may be present can be found in Greene et al., Protective Groups in Organic Synthesis, John Wiley & Sons, Inc., New York, NY 1991.

Protecting groups like Z, Boc and Fmoc are used extensively in the synthesis, and their removal conditions are well known to those skilled in the art. For example, removal of Z groups can he achieved by catalytic hydrogenation with hydrogen in the presence of a noble metal or its oxide, such as palladium on activated carbon in a protic solvent, such as ethanol. In cases where catalytic hydrogenation is contraindicated by the presence of other potentially reactive functionality, removal of Z can also be achieved by treatment with a solution of hydrogen bromide in acetic acid, or by treatment with a mixture of TFA and dimethylsulfide. Removal of Boc protecting groups is carried out in a solvent such as methylene chloride, methanol or ethyl acetate with a strong acid, such as TFA or HCl or hydrogen chloride gas.

The B and C moieties of a compound of formula (I) are linked together via a urea function. The skilled artist can, therefore, readily envision numerous routes and methods of connecting the two moieties using different well known methods.

The compounds of formula (I), when existing as a diastereomeric mixture, may be separated into diastereomeric pairs of enantiomers by fractional crystallization from a suitable solvent such as methanol, ethyl acetate or a mixture thereof. The pair of enantiomers thus obtained may be separated into individual stereoisomers by conventional means by using an optically active acid as a resolving agent. Alternatively, any enantiomer of a compound of the formula (I) may be obtained by stereospecific synthesis using optically pure starting materials or reagents of known configuration.

The compounds of formula (I) of the present invention can be prepared according to the procedures of the following schemes and examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described previously. The free amine bases corresponding to the isolated salts can be generated by neutralization with a suitable base, such as aqueous sodium hydrogencarbonate, sodium carbonate, sodium hydroxide and potassium hydroxide, and extraction of the liberated amine free base into an organic solvent followed by evaporation. The amine free base isolated in this manner can be further converted into another pharmaceutically acceptable salt by dissolution in an organic solvent followed by addition of the appropriate acid and subsequent evaporation, precipitation or crystallization. All temperatures are degrees Celsius.

In the schemes, preparations and examples below, various reagent symbols and abbreviations have the following meanings:

| | |
|---|---|
| Ac₂O | acetic anhydride |
| AcOH | acetic acid |
| Boc | tert-butoxycarbonyl |
| Bu | butyl |
| BuLi | butyllithium |
| DBU | 1,8-diazabicyclo[5.4.0]undec-7-ene |
| DCC | N,N'-dicyclohexylcarbodiimide |
| DCE | 1,2-dichloroethane |
| DCM | dichloromethane |
| DEAD | diethyl azodicarboxylate |
| DIAD | diisopropyl azodicarboxylate |
| DIC | N,N'-diisopropylcarbodiimide |
| DIEA | ethyl-diisopropylamine |
| DMA | N,N-dimethylacetamide |
| DMAP | 4-dimethylaminopyridine |
| DMF | N,N-dimethylformamide |
| DMS | dimethylsulfide |
| DMSO | dimethylsulfoxide |
| dppf | 1,1'-bis(diphenylphosphino)-ferrocen |
| EDCI | 1-(3-dimethylaminopropyl)-3-ethylcarbodiimide hydrochloride |
| Et₂O | diethyl ether |
| EtOAc | ethyl acetate |
| EtOH | ethanol |
| Fmoc | 9-fluorenylmethyloxycarbonyl |
| h | hour(s) |
| HOBt | 1-hydroxybenzotriazole hydrate |
| HTMP | 2,2,6,6-tetramethylpiperidine |
| MeCN | acetonitrile |
| MeOH | methanol |
| mp. | melting point |
| NMM | N-methylmorpholine |
| PG | protecting group |
| PPh₃ | triphenylphosphine |
| RT | room temperature |
| TEA | triethylamine |
| TFA | trifluoroacetic acid |
| THF | tetrahydrofurane |
| Z | benzyloxycarbonyl |
| Z-OSu | N-(benzyloxycarbonyloxy)succinimide |

The following amino acid derivatives were custom synthesized by PepTech Corporation, 20 Mall Road, Suite 460, Burlington, MA 01803 USA: D-2-chloro-4-fluorophenylalanine methyl ester hydrochloride, D-4-chloro-2-fluorophenylalanine methyl ester hydrochloride, and D-2,4-difluoro-phenylalanine methyl ester hydrochloride. D-2-Chloro-4-methylphenylalanine methyl ester hydrochloride and D-4-chloro-2-methylphenylalanine methyl ester hydrochloride were obtained from NetChem, Inc., 100 Jersey Ave, Suite A211, New Brunswick, NJ 08901 USA.

Cis-3-aza-bicyclo[3.1.0]hexane hydrochloride was prepared as described in US4,183,857.

2-Fluoro-3-iodo-pyrazine was prepared as described in Tetrahedron 1998, 54, 4899-4912.

4-Fluoro-3-iodo-pyridine was prepared as described in Tetrahedron 1993, 49, 49-64.

### Reaction scheme 1:

### Directed ortho-metallation

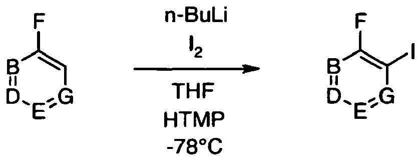

The starting material for the synthesis of heteroarylpiperidines, ortho-fluoro-iodopyridines, -pyridazines and -pyrazines, can be prepared as shown in Reaction scheme 1. A fluoro-substituted heteroaryl can be metallated with an amide prepared from a reagent such as n-butyllithium and an amine such as diisopropylamine or 2,2,6,6-tetramethylpiperidine at an appropriate temperature in a suitable solvent such as THF. The resulting lithio derivatives can subsequently be reacted with iodine to yield the desired compounds.

### Reaction scheme 2:

### Acetylation

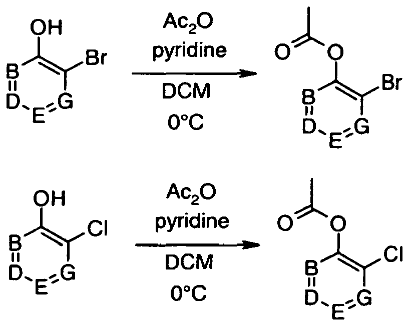

Another starting material for the synthesis of heteroarylpiperidines, ortho-acetoxy-bromopyridines, -pyridazines and -pyrazines, can be prepared as shown in Reaction scheme 2. A bromo- or chloroheteroaryl containing a hydroxy group in ortho-position to the bromo atom can be reacted with an acetylating reagent such as acetic anhydride in the presence of a suitable base such as pyridine in an appropriate solvent like DCM at a suitable temperature.

### Reaktion scheme 3:

### Negishi Coupling Reaction of Bromo- or Iodosubstituted Heteroaryls

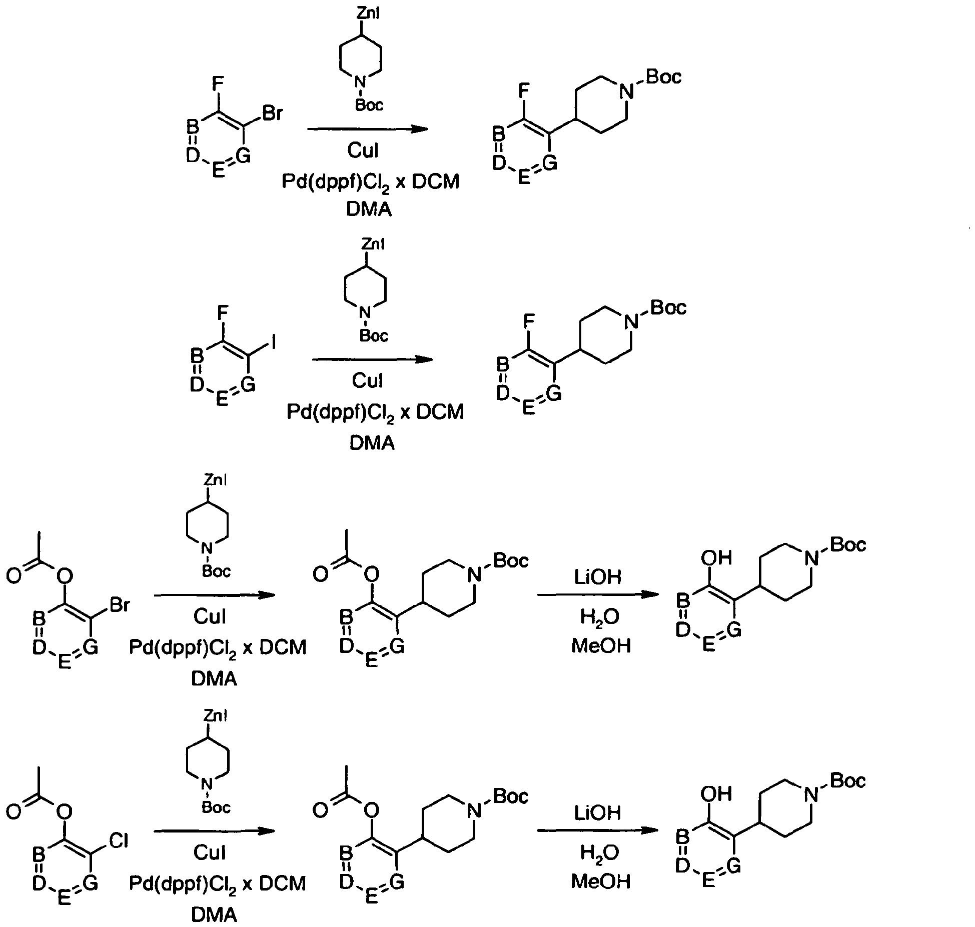

As shown in Reaction scheme 3, ortho-fluoro-bromopyridines, pyridazines and -pyrazines can be subjected to a Negishi coupling with (1-tert-butoxycarbonylpiperidin-4-yl)(iodo)zinc (J. Org. Chem. 2004, 69, 5120-5123) in the presence of copper(I) iodide and dichloro(1,1'-bis(diphenyl-phosphino)-ferrocene)palladium(II) DCM adduct in an inert solvent such as DMA to yield the resulting arylpiperidine. The same product is obtained, when ortho-fluoro-iodoopyridines, -pyridazines and -pyrazines are used as starting material.

The Negishi coupling can alternatively be performed using the ortho-acetoxysubstituted bromopyridines, -pyridazines and -pyrazines from Reaction scheme 2 as starting material. The free alcohol is obtained by saponification of the acetic acid ester with a base such as lithium hydroxide in a suitable solvent such as mixture of water and methanol. Usage of ortho-acetoxy-chloropyridines, -pyridazines and -pyrazines as starting materials results in formation of the same products.

### Reaktion scheme 4:

### Negishi Coupling Reaction of Heteroaryls with a Carboxaldehyde or Benzylamine Function

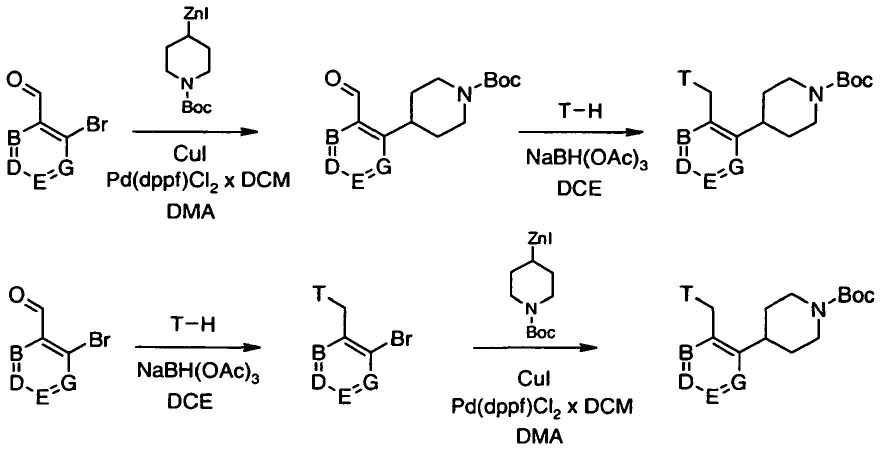

Optionally substituted ortho-carboxy-bromopyridines, pyridazines and -pyrazines can be subjected to a Negishi coupling as depicted in Reaction scheme 4. Reaction with (1-tert-butoxycarbonylpiperidin-4-yl)(iodo)zinc in the presence of copper(I) iodide and dichloro(1,1'-bis(diphenyl-phosphino)-ferrocene)palladium(II) DCM adduct in an inert solvent such as DMA leads to the resulting arylpiperidine. Reductive amination with a capping group T-H in the presence of a reducing agent such as sodium triacetoxyborohydride in a suitable solvent such as 1,2-dichloroethane leads to the Boc-protected A-moiety.

Alternatively, optionally substituted ortho-carboxy-bromopyridines, pyridazines and -pyrazines can first be subjected to a reductive amination step before the Negishi coupling is performed.

The reaction sequences described above can also be performed using optionally substituted ortho-carboxy-chloropyridines, pyridazines and -pyrazines.

### Reaktion scheme 5:

### Aminoalcohol Preparation

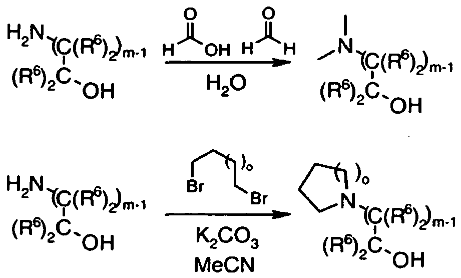

N-substituted amino alcohols HO(C(R⁶)₂)ₘ-T can be obtained as described in Reaction scheme 5. Reaction of an optionally substituted amino alcohol HO(C(R⁶)₂)ₘNH₂ with a mixture of formic acid and formaldehyde in a suitable solvent such as water at a given temperature results in formation of the corresponding N,N-dimethylated amino alcohols. Cyclic analogs of such amino alcohols can be obtained by reacting optionally substituted amino alcohol HO(C(R⁶)₂)ₘNH₂ with α,ω-dibromoalkanes in the presence of a base such as potassium carbonate in an appropriate solvent like acetonitrile.

### Reaktion scheme 6:

### Epoxides as Aminoalcohol Precursor

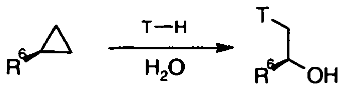

As shown in Reaction scheme 6, optionally substituted epoxides can be reacted in a regioselective way with an appropriate amine T-H in a suitable solvent like water to form α-substituted β-aminoalcohols.

### Reaktion scheme 7:

### Nucleophilic Aromatic Substitution Reaction

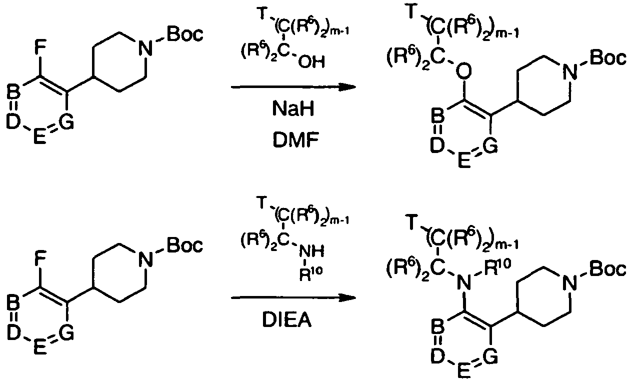

As shown in Reaction scheme 7, fluoro-substituted pyridyl-, pyridazyl- and pyrazinylpiperidines can be subjected to a nucleophilic aromatic substitution reaction with a ω-T-capped alkylalcohol in the presence of a base such as sodium hydride in a solvent such as DMF at a suitable temperature to obtain the Boc-protected A moiety. Alternatively, the fluoro-substituted heteroarylpiperidines can also be reacted with a ω-T-capped primary or secondary alkylamine in the presence of a base like BuLi or DIEA in an appropriate solvent like THF or without a solvent at a suitable temperature to obtain the Boc-protected A moiety.

### Reaction scheme 8

### Synthesis of A Moieties Containing Cyclic Amines

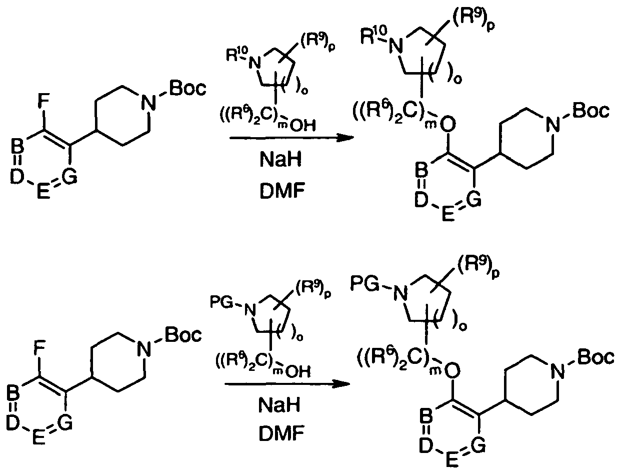

As shown in Reaction scheme 8, the intermediate product from Reaction scheme 3, optionally substituted fluoropyridines, -pyridazines and -pyrazines, can also be subjected to a nucleophilic aromatic substitution reaction with an alcohol which contains a cyclic tertiary amine moiety, in the presence of a base such as sodium hydride in a suitable solvent such as DMF to give the Boc-protected A moieties.

Similarly, an alcohol containing a protected cyclic secondary amine moiety can be introduced as building block using the conditions described above. The protecting group has to be orthogonal to the Boc-protecting group used for protection of the piperidine. After coupling of the A moiety with the B-C moiety this protecting group can be removed using standard methods.

### Reaction scheme 9:

### Alternative Synthesis of A Moieties with Alkylether Spacer (R¹ = -O(C(R⁶)₂)ₘ-T

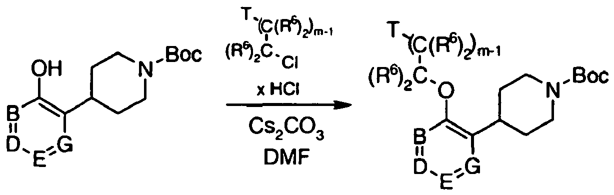

The synthesis of A Moieties bearing an alkylether spacer (R¹ = -O(C(R⁶)₂)ₘ-T) can alternatively be performed as depicted in Reaction scheme 9. The Boc-protected piperidine is reacted with an alkylchloride or alkylbromide bearing the capping group T in the presence of a base such as Cs₂CO₃ or NaH in an appropriate solvent such as DMF to give the Boc-protected A moiety.

### Reaction scheme 10:

### Synthesis of A Moieties with Alkylether Spacer (R¹ = -O(C(R⁶)₂)ₘ-T) Using Mitsunobu Conditions

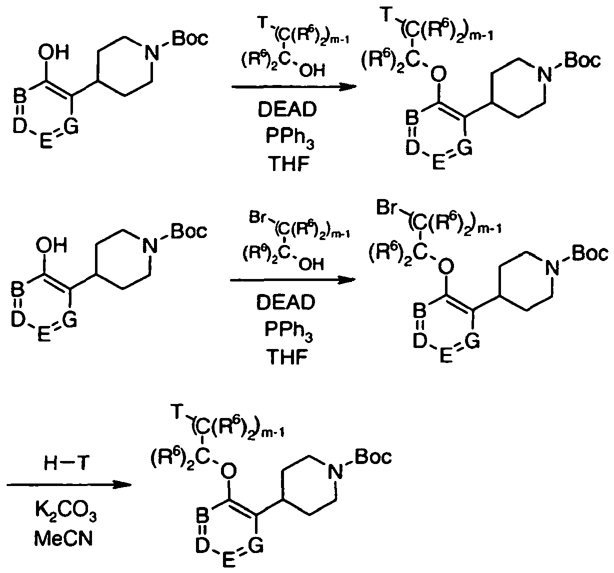

As shown in Reaction scheme 10, the intermediate product from Reaction scheme 3, optionally substituted (hydroxyheteraoaryl)piperidines, can also be alkylated with an ω-T-capped alkylalcohol in the presence of a reagent such as DEAD or DIAD and a phosphine such as PPh₃ in a suitable solvent such as THF to give the Boc-protected A moieties.

Similarly, the same intermediate can be reacted with an ω-bromo alkylalcohol, using the reaction conditions described above, to give access to the corresponding ether which subsequently can be used to alkylate the capping group T in the presence of a suitable base such as K₂CO₃ or NaH, in an appropriate solvent such as MeCN, THF, or DMF, at a suitable temperature, to yield the Boc-protected A moieties.

### Reaction scheme 11:

### Synthesis of 5-piperidin-4-yl-pyrimidine-derived A moieties

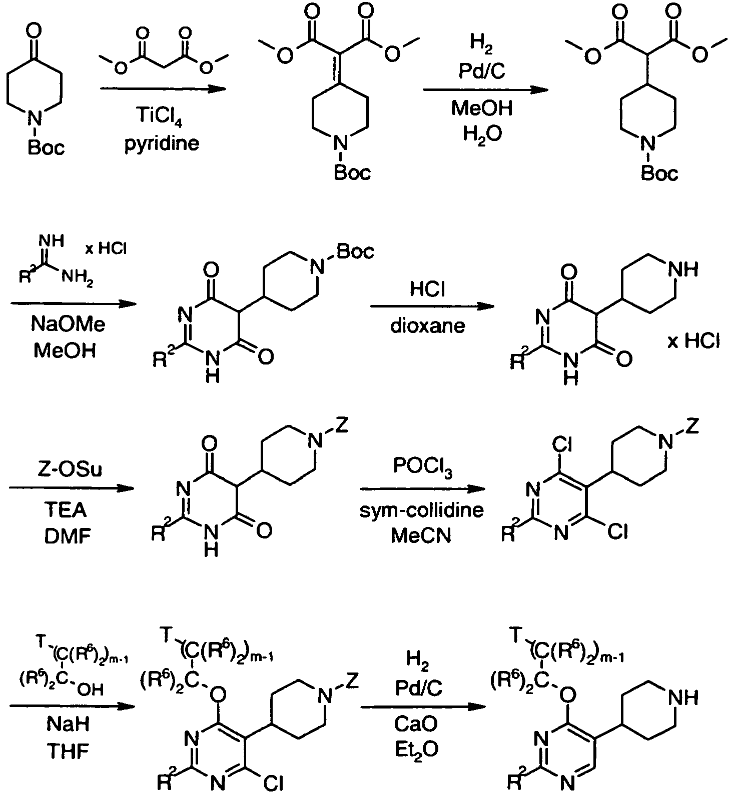

5-Piperidin-4-yl-pyrimidine-derived A moieties can be synthesized as shown in Reaction scheme 11. Boc-4-piperidone can be reacted with a malonic acid diester in the presence of a reagent such as titanium tetrachloride and a base such as pyridine at an appropriate temperature. The product of this reaction can be hydrogenated using a catalyst such as 10% palladium on charcoal in a suitable solvent such as a mixture of water and methanol to yield the corresponding Boc-2-piperidin-4-yl-malonic acid diester. Reaction with an amidine hydrochloride in the presence of a base such as sodium methoxide in a solvent like methanol leads to optionally substituted 1H-pyrimidine-4,6-dione which after exchanging the Boc protecting group to a Z protecting group can be converted to the 4,6-dichloro-pyrimidine using a reagent such as POCl₃ in the presence of a suitable base such as sym-collidine in an appropriate solvent like acetonitrile. Optionally substituted 4,6-dichloro-pyrimidine can be reacted with an ω-T-capped alkylalcohol in the presence of a base such as sodium hydride in a suitable solvent like THF. Subsequent hydrogenation using a catalyst such as 10% palladium on charcoal in the presence of calcium oxide in an appropriate solvent like diethyl ether yields the 5-piperidin-4-yl-pyrimidine-derived A moieties.

### Reaction scheme 12

### A Moiety Deprotection

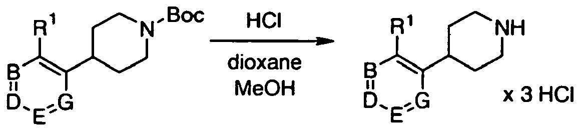

Generally, the starting material of Boc-protected heteroarylpiperidine (A moiety) can be deprotected in the presence of TFA/CH₂Cl_{2,} HCl/EtOAc, HCl/dioxane or HCl in MeOH/dioxane with or without a cation scavenger, such as dimethyl sulfide (DMS) before being subjected to the coupling procedure. It can be converted to the free base before being subjected to the coupling procedure or in some cases used as the salt.

### Reaction scheme 13

### Synthesis of Amines H-R⁵ using Grignard Reactions or Rupert's Reagent

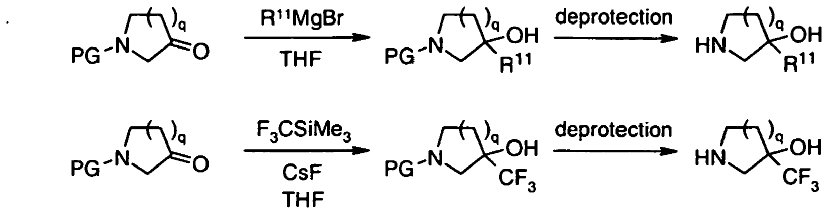

Reaction Scheme 13 shows the synthesis of amines H-R⁵ bearing a hydroxy substituent and one additional substituent at the same carbon atom. Such amines can be synthesized using oxo-substituted amines protected with a suitable protecting group. Suitable protecting group includes, but is not limited to, Boc, Z, benzyl, and benzhydryl. Protected oxo-substituted amines can be reacted with Grignard reagents in an inert solvent like THF or diethyl ether at an appropriate temperature to yield the corresponding substituted alcohols.

Alternatively, reaction of protected oxo-substituted amines with Rupert's reagent (trifluoromethyltrimethylsilane) in the presence of a reagent such as cesium fluoride in an appropriate solvent like THF at a given temperature leads to the trifluoromethylated alcohols.

Protecting groups of protected amines H-R⁵ obtained via the synthetic methods described above can be removed using standard conditions.

### Reaction scheme 14:

### B-C Moiety Formation

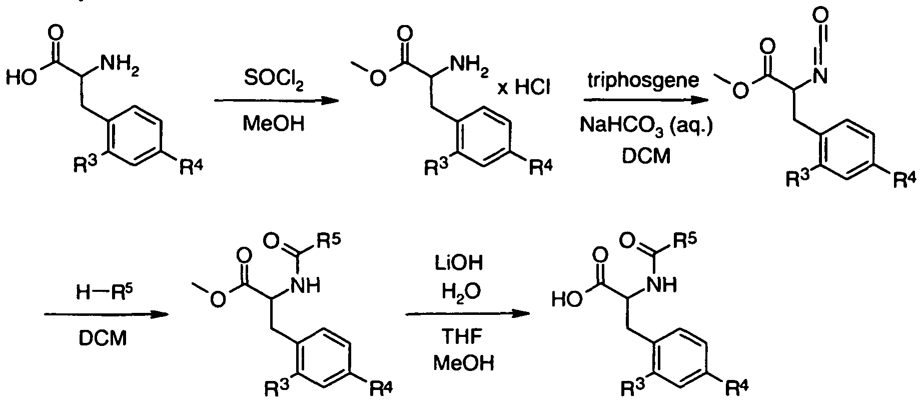

The B-C moieties can be synthesized as shown in Reaction scheme 14. Optionally substituted phenylalanine can be converted to the corresponding methyl ester hydrochloride using an activating reagent such as thionyl chloride or oxalyl chloride in methanol. Amino acid methyl ester hydrochloride can be reacted with a reagent such as triphosgene in the presence of a base such as NaHCO₃ (aq.) in a suitable solvent such as DCM to yield the isocyanate which can subsequently be reacted with an amine R⁵-H in a suitable solvent such as DCM or DMF. When R⁵-H is used in form of a hydrochloride, a suitable base such as DIEA is used in addition to liberate the free amine R⁵-H. The ester function can be hydrolyzed with a base such as LiOH in a suitable solvent or solvent mixture such as water/THF/methanol to give access to the B-C-moiety.

### Reaction scheme 15:

### B-C Moiety Solid-Phase Synthesis

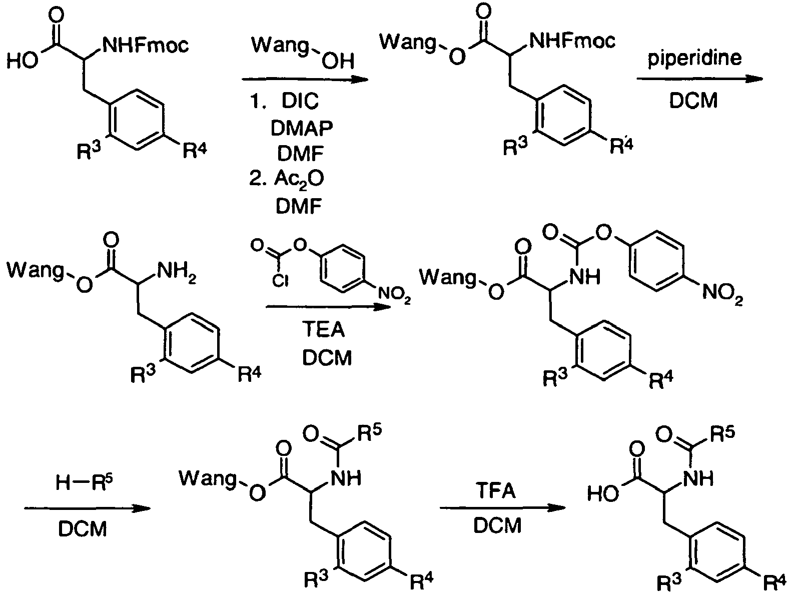

Alternatively, B-C-moieties can also be synthesized on solid phase as shown in Reaction scheme 15. Wang resin can be loaded with optionally substituted Fmoc-protected phenylalanine using a reagent such as DIC or DCC in the presence of a base such as DMAP in a suitable solvent such as DMF or DCM. Capping of unreacted OH-groups on the solid-support can be accomplished by subsequent reaction with acetic anhydride in an appropriate solvent like DMF or DCM. After removing the Fmoc-protecting group with a base such as piperidine or diethylamine in a solvent like DCM, the free amine can be converted to an activated carbamate with p-nitrophenyl chloroformate in the presence of a base such as TEA in an appropriate solvent like DCM. Reaction of said p-nitrophenyl carbamate with an amine R⁵-H in a suitable solvent such as DCM yields the desired B-C moiety. When R⁵-H is used in form of a hydrochloride, a suitable base such as DIEA is used in addition to liberate the free amine R⁵-H. Cleavage from solid support can be achieved by treament with TFA in DCM. B-C-moities obtained by this route can either be purified or directly be coupled with an appropriate A moiety.

### Reaction scheme 16:

### Coupling of A Moiety with B-C Moiety and Salt Formation

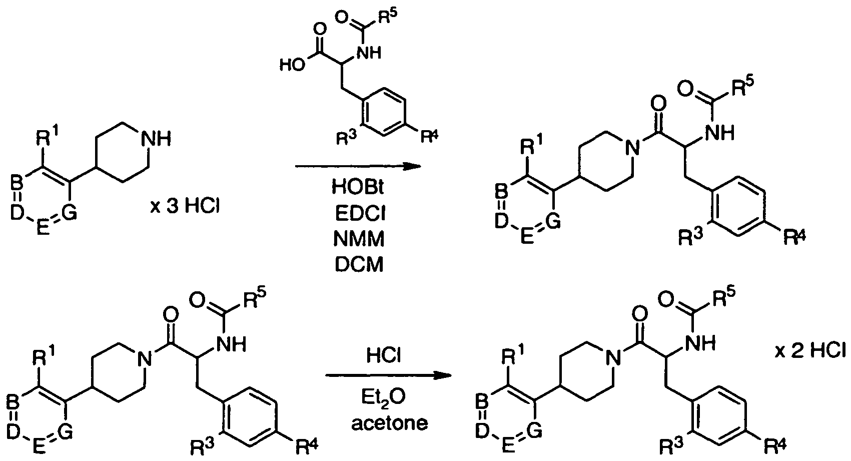

As shown in Reaction scheme 16, A moieties can be coupled with B-C moieties in the presence of EDCI/HOBt, a base such as N-methylmorpholine (NMM) and a solvent such as dichloromethane (DCM). A suitable solvent, such as DCM, DMF, THF or a mixture of the above solvents, can be used for the coupling procedure. A suitable base includes triethylamine (TEA), diisopropylethylamine (DIEA), N-methylmorpholine (NMM), collidine or 2,6-lutidine. A base may not be needed when EDCI/HOBt is used.

Generally after the reaction is completed, the reaction mixture can be diluted with an appropriate organic solvent, such as EtOAc, DCM or Et₂O, which is then washed with aqueous solutions, such as water, HCl, NaHSO₄, bicarbonate, NaH₂PO₄, phosphate buffer (pH 7), brine or any combination thereof. The reaction mixture can be concentrated and then be partitioned between an appropriate organic solvent and an aqueous solution. The reaction mixture can be concentrated and subjected to chromatography without aqueous workup.

The product can be transferred to a pharmaceutically acceptable salt such as a hydrochloride, using HCl in a solvent or solvent mixture such as diethyl ether/acetone.

### Reaction scheme 17:

### Urea Formation via Nitrophenylformate Intermediate

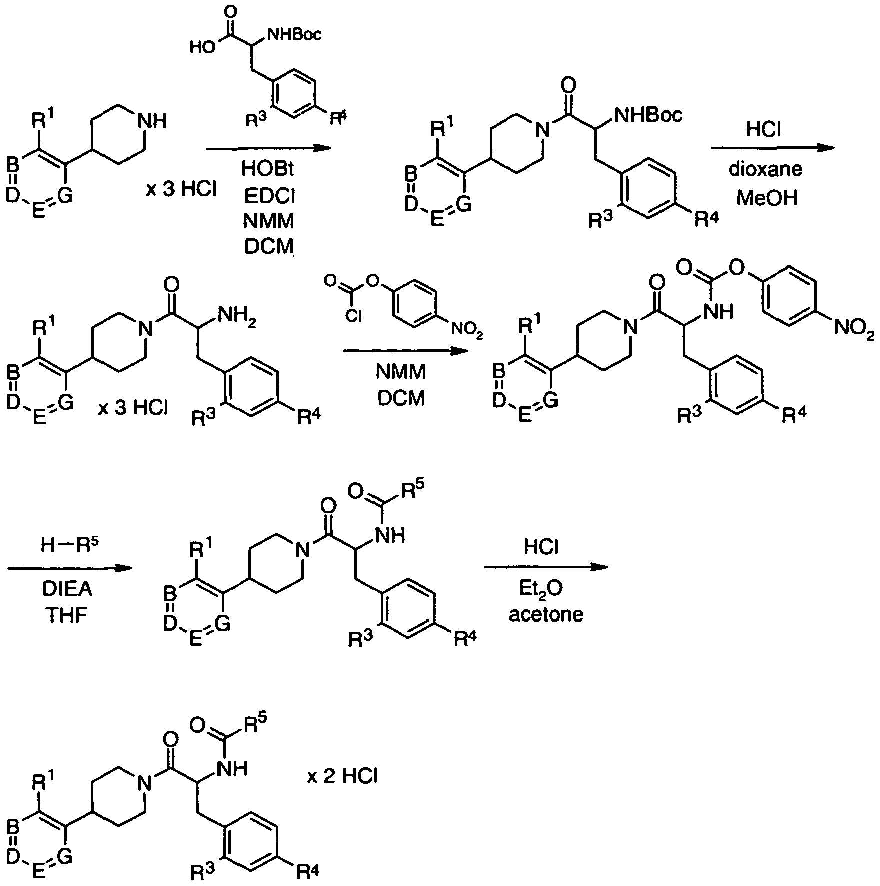

The three moieties can also be combined stepwise, as shown in Reaction scheme 17. An appropriate A moiety is coupled to a Boc-protected B moiety in the presence of EDCI/HOBt, a base such as N-methylmorpholine (NMM) and a solvent such as dichloromethane (DCM) followed by Boc deprotection with the aid of hydrogen chloride in a mixture of dioxane and methanol. The product can be reacted with 4-nitrophenyl chloroformate in the presence of a base such as NMM in an appropriate solvent such as DCM to yield the 4-nitrophenyl carbamate which subsequently can be treated with an amine H-R⁵ in the presence of a base such as DIEA in an appropriate solvent such as THF to give access to the target compound. The final product can be converted to a pharmaceutically acceptable salt as described above.

### Reaction scheme 18:

### Urea Formation Using 1-Methyl-3-(amino-1-carbonyl)-3H-imidazol-1-ium iodide as Reagent

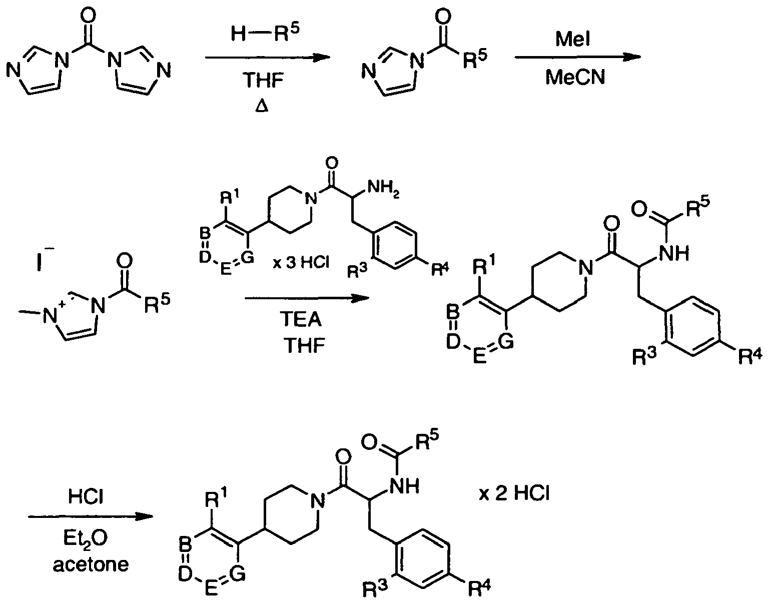

As shown in Reaction scheme 18 1,1'-carbonyldiimidazole can be reacted with an amine in an appropriate solvent such as THF at a suitable temperature. The product of this reaction is further reacted with methyl iodide in a suitable solvent such as acetonitrile to yield the 1-methyl-3-(amino-1-carbonyl)-3H-imidazol-1-ium iodide. This activated species is reacted with a deprotected A-B moiety in the presence of a base such as triethylamine in a suitable solvent such as THF to yield the final product. The final product can be converted to a pharmaceutically acceptable salt as described above.

### Analytical LC-MS

The compounds of the present invention according to formula (I) were analyzed via analytical LC-MS. The conditions used in the analysis are summarized below.

### Analytical conditions summary:

LC10Advp-Pump (Shimadzu) with SPD-M10Avp UV/Vis diode array detector and QP2010
MS-detector in ESI+ modus with UV-detection at 214 and 254 nm,
Column: Waters XTerra MS C18, 3.5 µm, 2.1 * 100 mm,
linear gradient with acetonitrile in water (0.15% HCOOH)
Flow rate of 0.4 ml/min;

| | |
|---|---|
| Mobile Phase A: | water (0.15% HCOOH + 5% acetonitrile) |
| Mobile Phase B: | acetonitrile (0.15% HCOOH + 5% water) |

### Gradient A:

start concentration 1% acetonitrile (0.15% HCOOH)

| | | |
|---|---|---|
| 9.00 min | B.Conc | 30 |
| 10.00 min | B.Curve | 3 |
| 12.00 min | B.Conc | 99 |
| 15.00 min | B.Conc | 99 |
| 15.20 min | B.Conc | 1 |
| 18.00 min | Pump | STOP |

### Gradient B:

start concentration 10% acetonitrile (0.15% HCOOH)

| | | |
|---|---|---|
| 10.00 min | B.Conc | 60 |
| 11.00 min | B.Curve | 2 |
| 12.00 min | B.Conc | 99 |
| 15.00 min | B.Conc | 99 |
| 15.20 min | B.Conc | 10 |
| 18.00 min | Pump | STOP |
| 0.00 min | 5% B | |
| 5.00 min | 95% B | |
| 5.10 min | 99% B | |
| 6.40 min | 99% B | |
| 6.50 min | 5% B | |
| 8.00 min | Pump | STOP |

### Gradient D:

linear gradient from 5% to 80% acetonitrile in water (0.15% HCOOH)

| | |
|---|---|
| 0.00 min | 5% B |
| 5.00 min | 80% B |
| 5.10 min | 99% B |
| 6.40 min | 99% B |
| 6.50 min | 5% B |
| 8.00 min | Pump STOP |

### Gradient E:

linear gradient from 1% to 70% acetonitrile in water (0.15% HCOOH)

| | |
|---|---|
| 0.00 min | 1% B |
| 5.00 min | 70% B |
| 5.10 min | 99% B |
| 6.40 min | 99% B |
| 6.50 min | 5% B |
| 8.00 min | Pump STOP |

The following tables describe detailed examples of the invention which can be prepared according to the Reaction schemes 1 to 18.

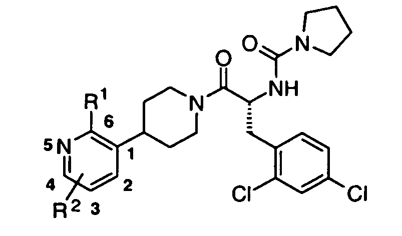

**Table 1:**

| | | | | HPLC | | MS | |
|---|---|---|---|---|---|---|---|
| No. | salt | R¹ | R² | t_{R} (min) | method | MW (calc.) free base | [M+H]⁺ (found) |
| 1 | 2 x HCl | 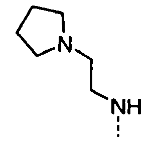 | 4-Me | 4.36 | B | 600 | 601 |
| 2 | 2 x HCl | 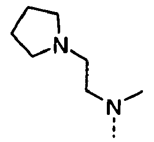 | 4-Me | 5.34 | B | 614 | 615 |
| 3 | 2x HCOOH | 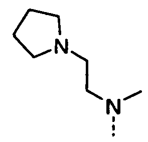 | 3-Me | 3.99 | B | 614 | 615 |
| 4 | 2 x HCl | 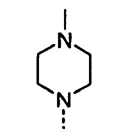 | 4-Me | 5.60 | B | 586 | 587 |
| 5 | 2x HCOOH | 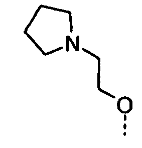 | H | 5.80 | B | 587 | 590 |
| 6 | 2 x HCl | 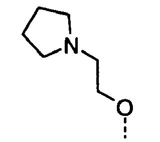 | 4-Me | 6.48 | B | 601 | 604 |
| 7 | 2x HCOOH | 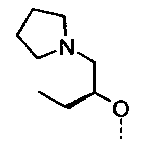 | H | 6.32 | B | 615 | 616 |
| 8 | 2x HCOOH | 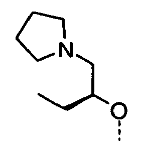 | 4-Me | 6.74 | B | 629 | 630 |
| 9 | 2x HCOOH | 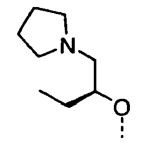 | 3-Me | 6.90 | B | 629 | 630 |
| 10 | 2 x HCl | 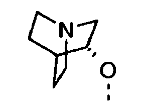 | H | 6.14 | B | 599 | 602 |

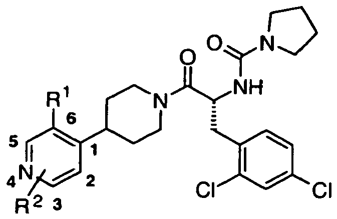

**Table 2:**

| | | | | HPLC | | MS | |
|---|---|---|---|---|---|---|---|
| No. | salt | R¹ | R² | t_{R} (min) | method | MW (calc.) free base | [M+H]⁺ (found) |
| 11 | 2 x HCl | 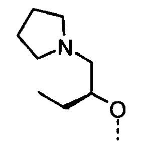 | H | 4.04 | B | 615 | 618 |

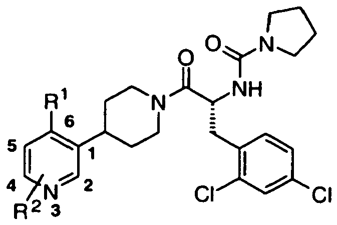

**Table 3:**

| | | | | HPLC | | MS | |
|---|---|---|---|---|---|---|---|
| No. | salt | R¹ | R² | t_{R} (min) | method | MW (calc.) free base | [M+H]⁺ (found) |
| 12 | 2 x HCl | 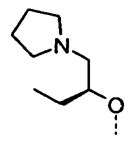 | H | 3.64 | B | 615 | 618 |
| 13 | 2x HCOOH | 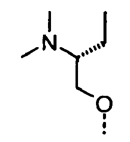 | H | 6.43 | A | 589 | 590 |

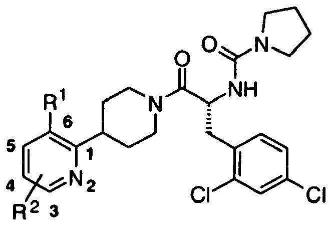

**Table 4:**

| | | | | HPLC | | MS | |
|---|---|---|---|---|---|---|---|
| No. | salt | R¹ | R² | t_{R} (min) | method | MW (calc.) free base | [M+H]⁺ (found) |
| 14 | 2 x HCl | 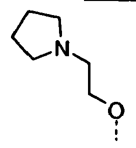 | H | 7.48 | A | 587 | 588 |
| 15 | 2 x HCl | 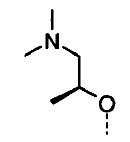 | H | 4.18 | B | 575 | 576 |
| 16 | 2 x HCl | 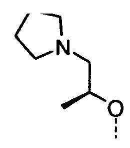 | H | 4.47 | B | 601 | 602 |
| 17 | 2 x HCl | 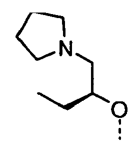 | H | 4.73 | B | 615 | 618 |
| 18 | - | 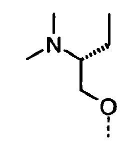 | H | 8.99 | A | 589 | 590 |
| 19 | 2 x HCl | 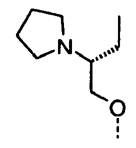 | H | 4.66 | B | 615 | 617 |
| 20 | - | 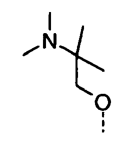 | H | 5.15 | A | 589 | 590 |
| 21 | 2 x HCl | 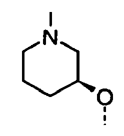 | H | 4.28 | B | 587 | 588 |
| 22 | 2x HCOOH | 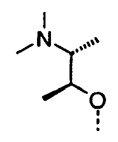 | H | 8.72 | A | 589 | 590 |
| 23 | 2 x HCl | 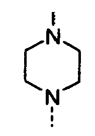 | H | 3.85 | B | 572 | 573 |
| 24 | 2x HCOOH | 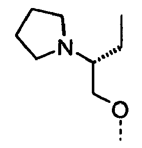 | 4-Me | 7.84 | A | 629 | 630 |
| 25 | HCOOH | 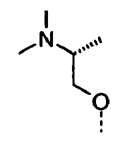 | H | 7.76 | A | 575 | 576 |
| 26 | 2 x HCl | 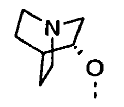 | H | 4.49 | B | 599 | 600 |
| 27 | HCOOH | 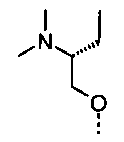 | 4-Me | 8.93 | A | 603 | 604 |
| 28 | 2x HCOOH | 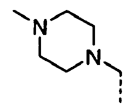 | H | 3.81 | B | 586 | 294* |
| 29 | 2x HCOOH | 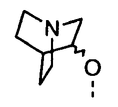 | H | 4.54 | B | 599 | 600 |
| 30 | HCOOH | 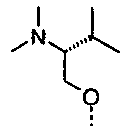 | H | 4.75 | B | 603 | 604 |
| 31 | 2 x HCl | 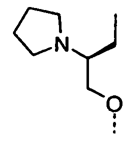 | H | 2.82 | C | 615 | 618 |
| 32 | 2 x HCl | 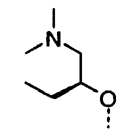 | H | 2.95 | D | 589 | 590 |
| 33 | 2 x HCl | 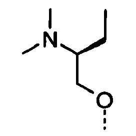 | H | 4.90 | B | 589 | 590 |
| 34 | 2 x HCl | 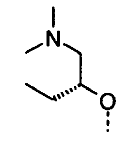 | H | 5.09 | B | 589 | 590 |
| 35 | HCOOH | 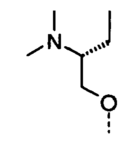 | 5-Me | 4.88 | B | 603 | 604 |
| 36 | HCOOH | 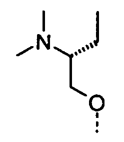 | 4-OMe | 5.41 | B | 619 | 620 |
| 37 | 2x HCOOH | 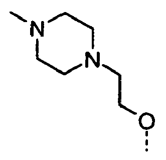 | H | 3.33 | B | 616 | 617 |
| 38 | 2 x HCl | 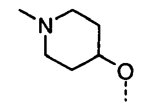 | H | 3.72 | B | 587 | 588 |
| 39 | HCOOH | 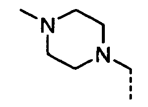 | 4-Cl | 5.96 | B | 620 | 621 |
| 40 | 2 x HCl | 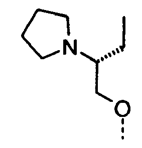 | 4-Cl | 6.78 | B | 649 | 652 |
| 41 | 2x HCOOH | 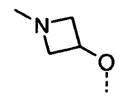 | H | 8.53 | A | 559 | 560 |
| 42 | 2x HCOOH | 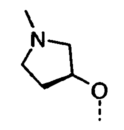 | H | 4.12 | B | 573 | 574 |
| 43 | 2x HCOOH | 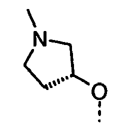 | H | 4.31 | B | 573 | 574 |
| 44 | 2x HCOOH | 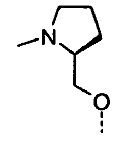 | H | 4.45 | B | 587 | 588 |
| 45 | 2x HCOOH | 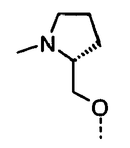 | H | 4.33 | B | 587 | 588 |
| 46 | HCOOH | 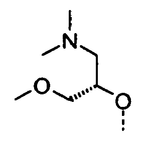 | H | 4.68 | B | 605 | 606 |
| 47 | HCOOH | 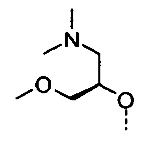 | H | 4.45 | B | 605 | 606 |
| 48 | 2x HCOOH | 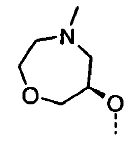 | H | 4.29 | B | 603 | 604 |
| 49 | 2x HCOOH | 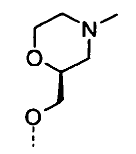 | H | 3.80 | B | 603 | 604 |
| 50 | HCOOH | 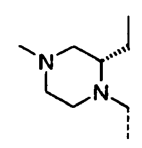 | H | 4.20 | B | 614 | 615 |
| 51 | HCOOH | 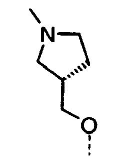 | H | 4.19 | B | 587 | 588 |
| 52 | 2x HCOOH | 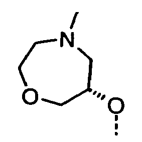 | H | 4.61 | B | 603 | 604 |
| 53 | 2x HCOOH | 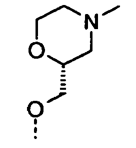 | H | 3.91 | B | 603 | 604 |
| 54 | 2 x HCl | 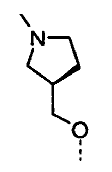 | H | 4.59 | B | 587 | 588 |
| 55 | HCOOH | 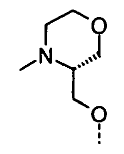 | H | 4.70 | B | 603 | 604 |
| 56 | 2x HCOOH | 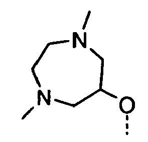 | H | 4.13 | B | 616 | 617 |
| 57 | 2x HCOOH | 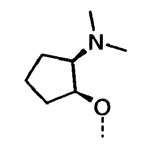 | H | 4.50 | B | 601 | 603 |
| 58 | HCOOH | 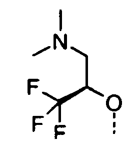 | H | 5.84 | B | 629 | 630 |
| 59 | HCOOH | 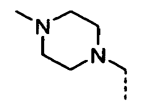 | 4-F | 5.74 | B | 604 | 605 |
| 60 | 2 x HCl | 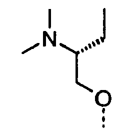 | 4-F | 5.88 | B | 607 | 608 |
| 61 | HCOOH | 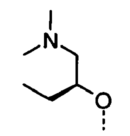 | 4-F | 6.41 | B | 607 | 608 |
| 62 | - | 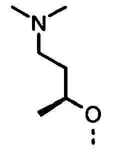 | H | 4.21 | B | 589 | 590 |

| | | | | | | | |
|---|---|---|---|---|---|---|---|
| * [M+2H]²⁺ | | | | | | | |

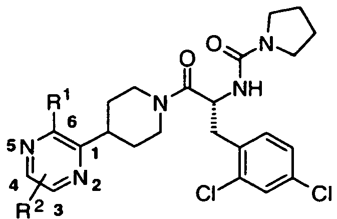

**Table 5:**

| | | | | HPLC | | MS | |
|---|---|---|---|---|---|---|---|
| No. | salt | R¹ | R² | t_{R} (min) | method | MW (calc.) free base | [M+H]⁺ (found) |
| 63 | 2 x HCI | 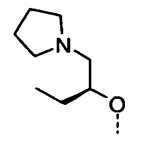 | H | 6.11 | B | 616 | 621 |

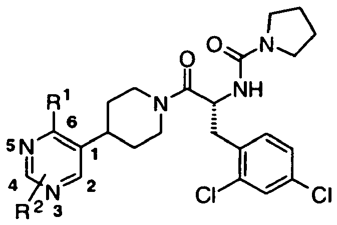

**Table 6:**

| | | | | HPLC | | MS | |
|---|---|---|---|---|---|---|---|
| No. | salt | R¹ | R² | t_{R} (min) | method | MW (calc.) free base | [M+H]⁺ (found) |
| 64 | HCOOH | 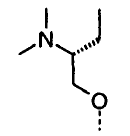 | 4-Me | 3.93 | B | 604 | 605 |

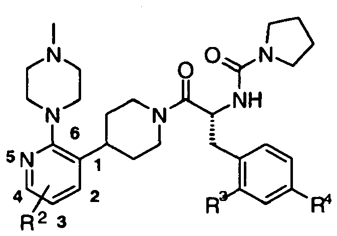

**Table 7:**

| | | | | | HPLC | | MS | |
|---|---|---|---|---|---|---|---|---|
| No. | salt | R² | R³ | R⁴ | t_{R} (min) | method | MW (calc.) free base | [M+H]⁺ (found) |
| 65 | 2 x HCl | 4-Me | F | CI | 5.33 | B | 570 | 571 |
| 66 | 2 x HCl | 4-Me | Cl | F | 5.06 | B | 570 | 571 |
| 67 | 2 x HCl | 4-Me | F | F | 4.51 | B | 554 | 555 |

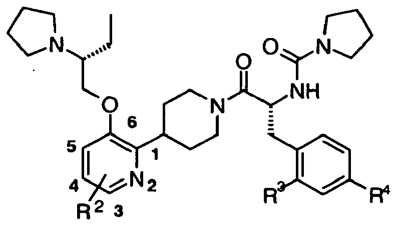

**Table 8:**

| | | | | | HPLC | | MS | |
|---|---|---|---|---|---|---|---|---|
| No. | salt | R² | R³ | R⁴ | t_{R} (min) | method | MW (calc.) free base | [M+H]⁺ (found) |
| 68 | HCOOH | H | Me | Cl | 4.45 | B | 595 | 596 |
| 69 | HCOOH | H | Cl | Me | 4.33 | B | 595 | 596 |
| 70 | HCOOH | H | Me | Me | 4.00 | B | 575 | 576 |
| 71 | 2x HCOOH | 4-Cl | Me | Me | 6.83 | B | 609 | 611 |

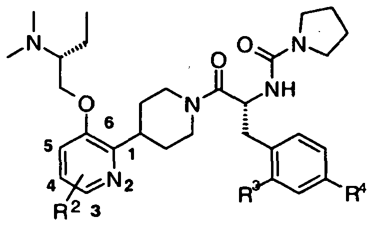

**Table 9:**

| | | | | | HPLC | | MS | |
|---|---|---|---|---|---|---|---|---|
| No. | salt | R² | R³ | R⁴ | t_{R} (min) | method | MW (calc.) free base | [M+H]⁺ (found) |
| 72 | HCOOH | 4-Me | F | Cl | 8.35 | A | 587 | 588 |
| 73 | HCOOH | 4-Cl | Me | Me | 5.91 | B | 583 | 585 |

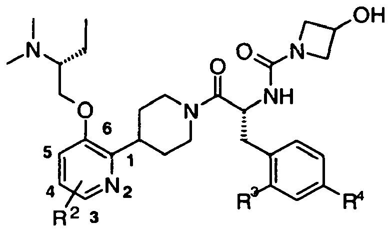

**Table 10:**

| | | | | | HPLC | | MS | |
|---|---|---|---|---|---|---|---|---|
| No. | salt | R² | R³ | R⁴ | t_{R} (min) | method | MW (calc.) free base | [M+H]⁺ (found) |
| 74 | HCOOH | 4-Cl | Me | Me | 5.04 | B | 585 | 586 |
| 75 | HCOOH | 4-F | Cl | Cl | 5.13 | B | 609 | 610 |

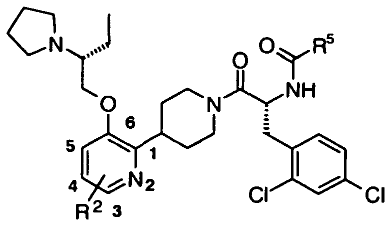

**Table 11:**

| | | | | HPLC | | MS | |
|---|---|---|---|---|---|---|---|
| No. | salt | R⁵ | R² | t_{R} (min) | method | MW (calc.) free base | [M+H]⁺ (found) |
| 76 | HCOOH | 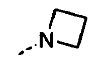 | H | 4.83 | B | 601 | 602 |
| 77 | HCOOH | 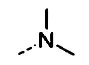 | H | 4.59 | B | 589 | 590 |
| 78 | - | 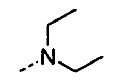 | H | 5.51 | B | 617 | 618 |
| 79 | 2 x HCI | 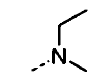 | H | 3.59 | E | 603 | 604 |
| 80 | 2 x HCI | 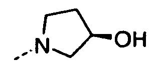 | H | 8.12 | A | 631 | 632 |
| 81 | - | 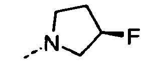 | H | 5.02 | B | 633 | 634 |
| 82 | - | 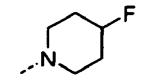 | H | 5.47 | B | 647 | 648 |
| 83 | - | 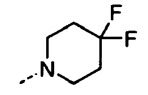 | H | 5.74 | B | 665 | 666 |
| 84 | 2x HCOOH | 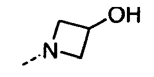 | H | 3.63 | B | 617 | 618 |
| 85 | HCOOH | 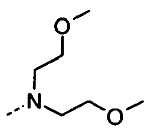 | H | 5.18 | B | 677 | 678 |
| 86 | 2 x HCl | 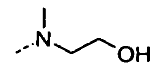 | H | 4.66 | E | 619 | 620 |
| 87 | 2 x HCl | 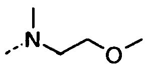 | H | 3.52 | E | 633 | 634 |
| 88 | - | 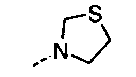 | H | 5.09 | B | 633 | 634 |
| 89 | 2 x HCl | 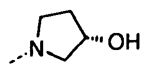 | H | 2.97 | E | 631 | 632 |
| 90 | HCOOH | 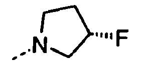 | H | 4.79 | B | 633 | 634 |
| 91 | 2 x HCl | 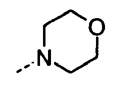 | H | 2.70 | C | 631 | 634 |
| 92 | 2 x HCl | 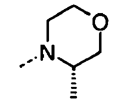 | H | 2.79 | C | 645 | 646 |
| 93 | 2 x HCl | 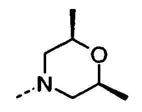 | H | 2.90 | C | 659 | 660 |
| 94 | 2 x HCl | 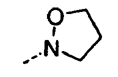 | H | 2.83 | C | 617 | 618 |
| 95 | 3 x HCl | 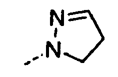 | H | 2.70 | C | 614 | 615 |
| 96 | 2x HCOOH | 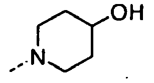 | H | 6.69 | B | 645 | 646 |
| 97 | 2 x HCl | 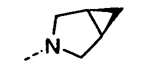 | H | 2.86 | C | 627 | 628 |
| 98 | HCOOH | 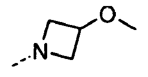 | H | 4.14 | B | 631 | 632 |
| 99 | - | 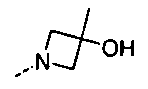 | H | 3.39 | E | 631 | 632 |
| 100 | HCOOH | 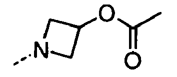 | H | 8.93 | A | 659 | 660 |

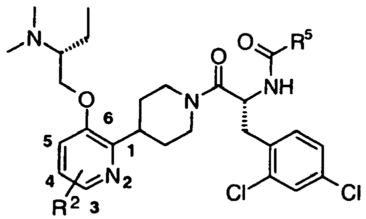

**Table 12:**

| | | | | HPLC | | MS | |
|---|---|---|---|---|---|---|---|
| No. | salt | R⁵ | R² | t_{R} (min) | method | MW (calc.) free base | [M+H]⁺ (found) |
| 101 | 2 x HCl | 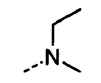 | H | 7.83 | B | 577 | 578 |
| 102 | HCOOH | 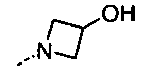 | H | 3.46 | B | 591 | 592 |
| 103 | 2 x HCl | 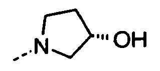 | H | 4.10 | B | 605 | 606 |
| 104 | 2 x HCl | 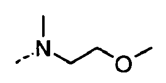 | H | 4.50 | B | 607 | 609 |
| 105 | HCOOH | 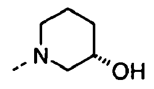 | H | 8.17 | A | 619 | 620 |
| 106 | HCOOH | 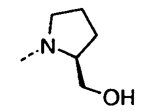 | H | 8.54 | A | 619 | 620 |
| 107 | HCOOH | 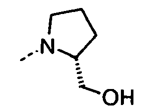 | H | 4.16 | B | 619 | 620 |
| 108 | 2x HCOOH | 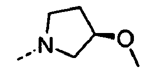 | H | 4.05 | B | 619 | 620 |
| 109 | 2x HCOOH | 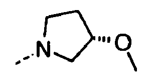 | H | 4.00 | B | 619 | 620 |
| 110 | 2 x HCl | 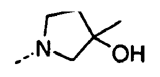 | H | 7.85 | A | 619 | 620 |
| 111 | HCOOH | 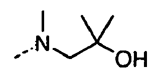 | H | 4.18 | B | 621 | 622 |
| 112 | HCOOH | 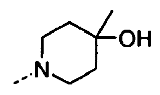 | H | 3.87 | B | 633 | 634 |
| 113 | HCOOH | 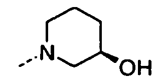 | H | 4.03 | B | 619 | 620 |
| 114 | HCOOH | 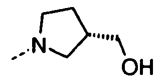 | H | 7.77 | A | 619 | 620 |
| 115 | HCOOH | 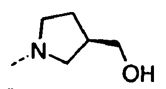 | H | 7.66 | A | 619 | 620 |
| 116 | HCOOH | 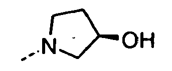 | H | 3.40 | B | 605 | 606 |
| 117 | HCOOH | 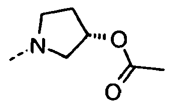 | H | 4.58 | B | 647 | 648 |
| 118 | HCOOH | 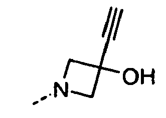 | H | 3.79 | B | 615 | 616 |
| 119 | HCOOH | 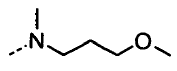 | H | 4.54 | B | 621 | 622 |
| 120 | HCOOH | 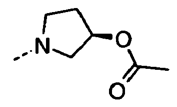 | H | 4.35 | B | 647 | 648 |
| 121 | HCOOH | 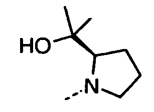 | H | 4.88 | B | 647 | 648 |
| 122 | HCOOH | 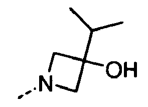 | H | 4.63 | B | 633 | 634 |
| 123 | 2x HCOOH | 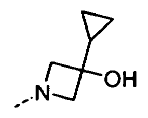 | H | 4.44 | B | 631 | 632 |
| 124 | - | 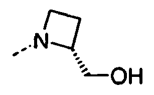 | H | 4.14 | B | 605 | 606 |
| 125 | HCOOH | 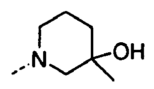 | H | 4.41 | B | 633 | 634 |
| 126 | - | 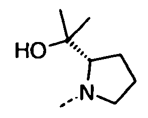 | H | 5.31 | B | 647 | 648 |
| 127 | - | 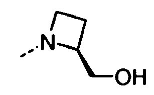 | H | 4.05 | B | 605 | 606 |
| 128 | - | 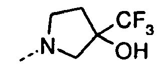 | H | 5.15 | B | 673 | 674 |
| 129 | - | 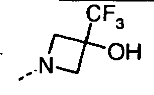 | H | 9.04 | B | 559 | 560 |
| 130 | HCOOH | 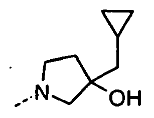 | H | 4.92 | B | 659 | 660 |
| 131 | - | 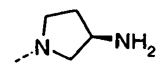 | H | 3.00 | B | 604 | 605 |
| 132 | 2x HCOOH | 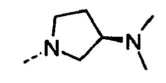 | H | 7.43 | A | 632 | 633 |
| 133 | 2x HCOOH | 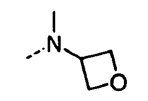 | H | 6.94 | B | 605 | 606 |
| 134 | 2x HCOOH | 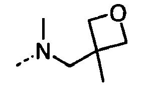 | H | 7.06 | B | 633 | 634 |

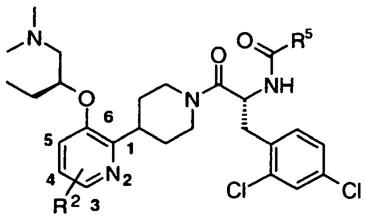

**Table 13:**

| | | | | HPLC | | MS | |
|---|---|---|---|---|---|---|---|
| No. | salt | R⁵ | R² | t_{R} (min) | method | MW (calc.) free base | [M+H]⁺ (found) |
| 135 | 2 x HCl | 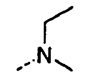 | H | 7.76 | B | 577 | 578 |
| 136 | HCOOH | 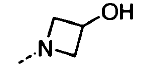 | H | 3.65 | B | 591 | 592 |
| 137 | 2 x HCl | 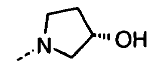 | H | 4.12 | B | 605 | 606 |
| 138 | 2 x HCl | 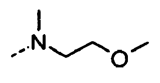 | H | 4.88 | B | 607 | 608 |
| 139 | HCOOH | 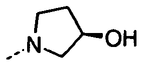 | H | 3.56 | B | 605 | 606 |

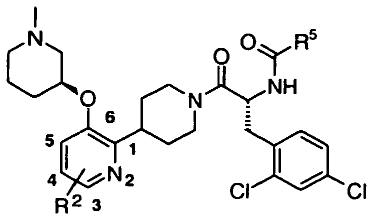

**Table 14:**

| | | | | HPLC | | MS | |
|---|---|---|---|---|---|---|---|
| No. | salt | R⁵ | R² | t_{R} (min) | method | MW (calc.) free base | [M+H]⁺ (found) |
| 140 | HCOOH | 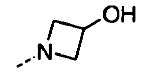 | H | 3.17 | B | 589 | 591 |
| 141 | 2 x HCl | 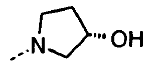 | H | 3.52 | B | 603 | 604 |
| 142 | 2 x HCl | 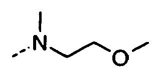 | H | 3.87 | B | 605 | 606 |
| 143 | HCOOH | 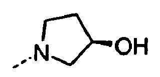 | H | 3.18 | B | 603 | 604 |

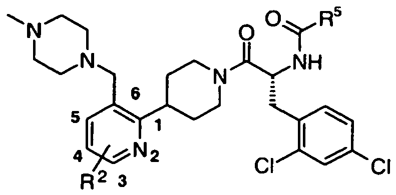

**Table 15:**

| | | | | HPLC | | MS | |
|---|---|---|---|---|---|---|---|
| No. | salt | R⁵ | R² | t_{R} (min) | method | MW (calc.) free base | [M+H]⁺ (found) |
| 144 | HCOOH | 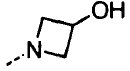 | 4-F | 4.43 | B | 606 | 607 |

The following examples are provided to illustrate the invention and are not limiting the scope of the invention in any manner.

### Common Intermediates:

### 1-tert-Butoxycarbonylpiperidin-4-yl)(iodo)zinc:

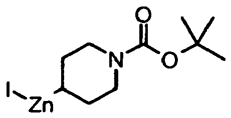

**Zinc activation.** A schlenk flask was charged with Celite (1.28 g) and dried by heating *in vacuo*. Then zinc dust (6.51 g) and dry *N,N*-dimethylacetamide (15 ml) were added. The mixture was stirred at room temperature while a 7:5 v/v mixture of chlorotrimethylsilane (1.14 ml) and 1,2-dibromoethane (0.80 ml) as solution in *N,N*-dimethylacetamide (1 ml) was added at a rate to maintain the temperature below 65°C (~15 min). The resulting slurry was aged for 15 min.

**Zink insertion.** A solution of Boc-4-iodopiperidine (24.8 g) in *N,N*-dimethylacetamide (39 ml) was slowly added to the mixture described above at a rate to maintain a temperature below 65°C (~20 min). The resulting reaction mixture was then aged for 30 min at room temperature. The suspension was filtered through a frit under argon to remove all solids. The resulting pale yellow solution was stored at room temperature under argon and was directly used for the coupling reactions.

### 3-Fluoro-3',4',5',6'tetrahydro-2'H-[2,4']bipyridinyl-1'-carboxylic acid tert-butyl ester:

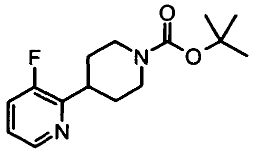

A 500 ml flask was charged with 2-bromo-3-fluoropyridine (10.0 g), [1,1'-bis(diphenylphosphino)ferrocene]dichloropalladium(II) complex with dichloromethane (1.39 g), copper(I) iodide (0.65 g), and dry *N,N*-dimethylacetamide (80 ml). The resulting mixture was degassed with alternating vacuum/argon purges. Then 1-tert-butoxycarbonylpiperidin-4-yl)(iodo)zinc (79.7 mmol) was added. The mixture was degassed once again and then heated to 80°C overnight. The main part of *N,N-*dimethylacetamide was then evaporated and the remainder was taken up in a mixture of EtOAc and water (500 ml each). This was then filtered through Celite and transferred into a separatory funnel. The phases were separated and the water layer was extracted with EtOAc (3 x 250 ml). The combined organic layer was washed with water and brine (500 ml each), dried (Na₂SO₄), filtered and concentrated *in vacuo*. The crude product was purified by chromatography to furnish the desired compound in form of a brown solid.

### (S)-1-Pyrrolidin-1-yl-butan-2-ol:

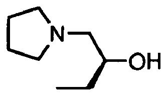

Pyrrolidine (5.76 ml) was added to a mixture of (S)-(-)-1,2-epoxybutane (5.00 ml) and water (25 ml). The reaction mixture was vigorously stirred overnight at room temperature. More water (25 ml) was added, and the organic materials were extracted with diethyl ether (2 x 100 ml). The combined organic layer was dried (Na₂SO₄), filtered and concentrated *in vacuo*. In order to get rid of volatile impurities several co-evaporations with ether and toluene were carried out (each with 2 x 10 ml) to furnish the desired compound in form of a colorless liquid.

### (R)-2-Pyrrolidin-1-yl-butan-1-ol:

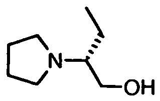

To a solution of (R)-(-)-2-amino-1-butanol (5.00 g) and 1,4-dibromobutane (6.61 ml) in CH₃CN (60 ml) was added K₂CO₃ (15.48 g) and the resulting suspension was stirred at reflux temperature for 19 hours. The reaction mixture was evaporated *in vacuo* and the residue was divided between EtOAc and water. The organic layer was washed with water and brine. The aqueous layers were extracted with EtOAc. The combined organic layer was dried over Na₂SO₄ and evaporated *in vacuo* to dryness. The crude product was purified by Kugelrohr distillation (12 mbar, 106-150°C) to yield a clear colorless oil.

### (S)-1-Dimethylamino-butan-2-ol:

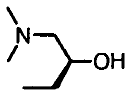

A mixture of (*S*)-(-)-1,2-epoxybutane (9.66) and water (40 ml) was placed in a 100 ml flask. Dimethylamine (40% solution in water, 20.3 ml) was added in one portion and the reaction mixture was cooled for a few minutes with an ice bath and stirred overnight at room temperature. Solid NaCI was added until saturation and the mixture was extracted with DCM (3 x 50 ml). The combined organic layer was washed with water and brine (30 ml each), dried (Na₂SO₄), filtered and carefully evaporated (20 mbar / 25°C) to yield a colorless liquid.

### (R)-2-Dimethylamino-butan-1-ol:

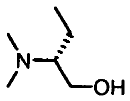

(R)-(-)-2-Amino-1-butanol (5.00 g) was added dropwise to cooled formic acid (11.0 ml). Formaldehyde (36.5% aq., 10.73 ml) was added and the mixture was stirred at 0°C for 1 h. It was then heated to 90°C for 4 h. The reaction was evaporated *in vacuo* and the oily residue was partitioned between DCM (150 ml) and 1 N NaOH. The organic phase was washed with 1 N Na₂CO₃ and brine. The DCM phase was dried over MgSO₄, filtered and evaporated to yield a yellow oil. It was purified by Kugelrohr distillation (60 mbar, 85-130°C). The product was obtained in form of a colorless oil.

### 2-Dimethylamino-2-methyl-propan-l-ol:

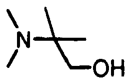

A mixture of 2-amino-2-methyl-1-propanol (5.00 g), formaldehyde solution, 36.5% in water (10.73 ml) and formic acid (11.00 ml) in water (20 ml) was stirred at 0°C for 1 h and then heated to 90°C for 4 h. The reaction mixture was concentrated *in vacuo*, the residue diluted with water (20 ml) and made alkaline (pH 14) by addition of 1 N NaOH. Furthermore, solid NaCl was added until saturation. The aqueous solution was extracted with dichloromethane (3 x 50 ml) and the combined organic layer was dried (Na₂SO₄) and concentrated *in vacuo*. Distillation under reduced pressure (40 mbar) afforded the desired compound as colorless oil.

### (S)-1-Methyl-piperidin-3-ol:

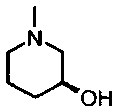

A mixture of (S)-3-hydroxypiperidine hydrochloride (4.00 g), formaldehyde solution, 36.5% in water (3.73 ml) and formic acid (2.19 ml) in water (20 ml) was kept under reflux overnight. The reaction mixture was concentrated *in vacuo*, the residue diluted with water (20 ml) and made alkaline (pH 14) by addition of 1 N NaOH. Furthermore, solid NaCI was added until saturation. The aqueous solution was extracted with dichloromethane (3 x 50 ml) and the combined organic layer was dried (Na₂SO₄) and concentrated *in vacuo*. Distillation under reduced pressure (10 mbar) afforded the desired compound as colorless liquid.

### 3-((R)-2-Dimethylamino-butoxy)-3',4',5',6'-tetrahydro-2'H-[2,4']bipyridinyl-1'-carboxylic acid tert-butyl ester:

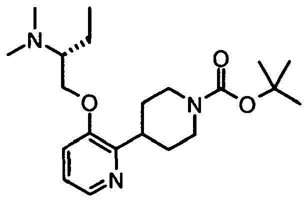

(R)-2-Dimethylamino-butan-1-ol (1.004 g) was added under argon at 0°C to a suspension of sodium hydride (60% in mineral oil, 240 mg) in DMF (30 ml). The cooling bath was removed and the mixture was stirred at room temperature for 2 h. Then 3-fluoro-3',4',5',6'-tetrahydro-2'H-[2,4']bipyridinyl-1'-carboxylic acid tert-butyl ester (1.200 g) was added and the reaction mixture was heated to 120°C (oil bath temperature). After being stirred at 120°C overnight the reaction mixture was concentrated *in vacuo*. The remaining dark brown oil was dissolved in ethyl acetate (150 ml) and the mixture was washed with 1 N Na₂CO₃ (2 x 60 ml). The combined aqueous layer was re-extracted with EtOAc (30 ml) and the combined organic layer was washed with brine (50 ml), dried (MgSO₄), filtered and evaporated to yield a dark brown oil. It was purified by column chromatography.

### [(R)-1-(1',2',3',4',5',6'-Hexahydro-[2,4']bipyridinyl-3-yloxymethyl)-propyl]-dimethyl-amine:

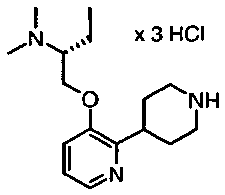

3-((R)-2-Dimethylamino-butoxy)-3',4',5',6'-tetrahydro-2'H-[2,4']bipyridinyl-1'-carboxylic acid tert-butyl ester (788 mg) was dissolved in a mixture of dioxane (15 ml) and methanol (4 ml). 4 N HCI in dioxane (15 ml) was added and the reaction was stirred at room temperature for 1 h. Evaporation of all volatiles including co-evaporation with toluene (4 x 30 ml) gave a beige semisolid, which was dried further in high vacuum overnight. The remaining solid was triturated with 10 ml of dry diethyl ether. The solvent was decanted and the product was dried *in vacuo*.

### Synthesis of B-C Moieties:

### B-C Moiety 1:

### Intermediate A1):

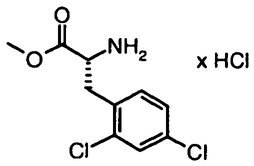

To a suspension of D-2,4-dichlorophenylalanine (10.00 g) in methanol (100 ml) was dropwise added thionylchloride (9.39 ml). During the course of the addition a clear solution was formed and the reaction started to reflux. The reaction mixture was kept under reflux for 2 h. After cooling to room temperature the mixture was evaporated to dryness at 40°C. The crude product was triturated in diethyl ether, and the insoluble compound was filtered off, washed with diethyl ether, and finally dried *in vacuo* at room temperature over P₂O₅ overnight. The product was obtained in form of colorless needles.

### Intermediate B1):

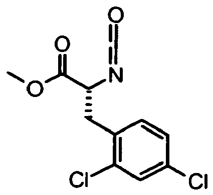

A 350 ml three-necked, flat-bottomed flask was equipped with a mechanical stirrer and charged with DCM (80 ml), saturated aqueous sodium bicarbonate solution (80 ml), and intermediate A1) (5.69 g). The biphasic mixture was cooled in an ice bath and stirred mechanically while triphosgene (1.96 g) was added in a single portion. The reaction mixture was stirred in the ice bath for 45 min and then poured into a 250 ml separatory funnel. The organic layer was collected, and the aqueous layer was extracted with three 20 ml portions of DCM. The combined organic layer was washed with water, dried over Na₂SO₄, filtered, and evaporated *in vacuo* to dryness to yield the crude product as a semisolid. The residue was purified by Kugelrohr distillation (200-240°C, 0.04-0.08 mbar). The product was obtained as clear colorless oil.

### Intermediate C1):

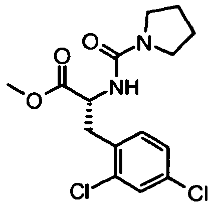

To an ice cold solution of intermediate B1) (4.99 g) in DCM (50 ml) was added pyrrolidine (4.56 ml). After 10 minutes the ice bath was removed and stirring was continued for 4 h. The reaction mixture was evaporated *in vacuo.* The residue was redissolved in EtOAc and the organic layer was washed with 1 N HCI, water, sat. Na₂CO₃, water and brine. All the aqueous layers were extracted with EtOAc. The combined organic layer was dried over Na₂SO₄ and evaporated *in vacuo* to dryness.

### B-C Moiety 1:

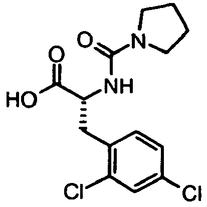

Intermediate C1) (6.28 g) was dissolved in MeOH (100 ml) and THF (30 ml) at 0°C. A solution of lithium hydroxide monohydrate (1.53 g) in water (30 ml) was added dropwise over the course of 5 min. The mixture was stirred at 0°C for 60 min and then acidified by adding 0.5 M HCI. The reaction mixture was extracted two times with EtOAc. The combined organic layer was washed two times with water and with brine, dried over Na₂SO₄ and evaporated *in vacuo*. The solid residue was triturated in Et₂O, then filtered off and washed with Et₂O. The product was obtained as a white solid.

### B-C Moiety 2:

### Intermediate A2):

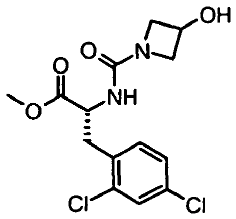

To an ice cold solution of intermediate B1) (200 mg) in anhydrous DMF (2.5 ml) was added a solution of 3-hydroxyazetidine hydrochloride (160 mg) and triethylamine (205 µl) in anhydrous DMF (2.5 ml) under Ar. The mixture was left stirring at 0°C for 4 h 30 min. The reaction mixture was evaporated *in vacuo*. The residue was diluted with cold EtOAc (75 ml), the organic phase was washed with 0.1 M HCI (2 x 25 ml) and brine. The aqueous phase was re-extracted with EtOAc and the organic phase was dried over Na₂SO₄, filtered and evaporated. A colorless oil was obtained which was dried under high vacuum.

### B-C Moiety 2:

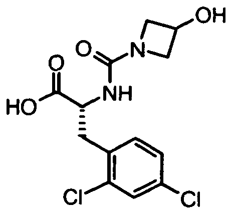

Intermediate A2) (255 mg) was dissolved in MeOH (1.5 ml) and THF (4.0 ml) at 0°C. A solution of lithium hydroxyde monohydrate (62 mg) in H₂O (1.5 ml) was added. The mixture was stirred at 0°C for 4 h 30 min. The reaction mixture was neutralized by addition of 1 N HCI and the solvents were evaporated *in vacuo*. The aqueous phase was then acidified with 1 N HCI (pH - 1-2). The aqueous phase was extracted with EtOAc (~1 x 20 ml). The organic layer was washed with water and brine. The aqueous phases were re-extracted with EtOAc, the combined organic layer was dried over Na₂SO₄ and evaporated *in vacuo* to yield a colorless solid.

### B-C Moiety 3:

### Intermediate A3):

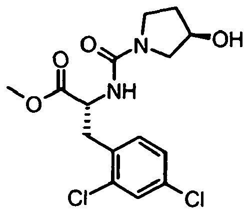

(R)-3-Pyrrolidinol (1.43 g) was added to a ice cold solution of intermediate B1) (1.50 g) in CH₂Cl₂ (25 ml). After 2 hours, the ice bath was removed and stirring continued for 2 hours. The reaction mixture was evaporated *in vacuo*. The residue was redissolved in EtOAc and the organic layer was washed with 1 N HCI, water, sat. NaHCO₃, water and brine. All the aqueous layers were extracted with EtOAc. The combined organic layer was dried over MgSO₄, filtered and evaporated *in vacuo* to dryness.

### B-C Moiety 3:

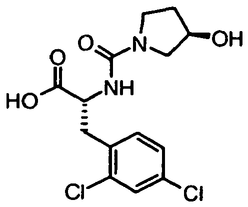

Intermediate A3) (1.78 g) was dissolved in MeOH (35 ml) and THF (9.5 ml) at 0°C. A solution of the lithium hydroxide monohydrate (0.41 g) in H₂O (9.5 ml) was added dropwise over the course of 5 minutes. The mixture was stirred at 0°C for 2 hours. The reaction mixture was acidified by adding 0.5 M HCI and was then extracted twice with EtOAc. The organic layers were washed twice with water and brine. The combined organic layer was dried over Na₂SO₄ and evaporated *in vacuo.* The solid residue was triturated in Et₂O, then filtered off and washed with Et₂O to yield a colorless, crystalline solid.

### B-C Moiety 4:

### Intermediate A4):

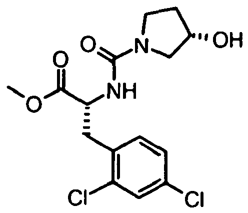

(S)-3-Pyrrolidinol (1.43 g) was added to a ice cold solution of the isocyanate (1.50 g) in CH₂Cl₂ (25 ml). After 20 minutes the ice bath was removed and stirring continued for 4 hours. The reaction mixture was evaporated *in vacuo*. The residue was redissolved in EtOAc and the organic layer was washed with 1 N HCI, water, sat. NaHCO₃ and brine. Each aqueous layer was re-extracted with EtOAc. The combined organic layer was dried over MgSO₄, filtered and evaporated *in vacuo* to dryness. The product was obtained in form of a colorless, stable foam.

### B-C Moiety 4:

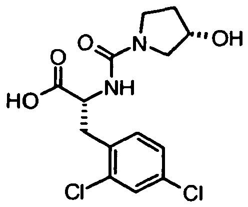

Intermediate A4) (1.97 g) was dissolved in MeOH (35 ml) and THF (9.5 ml) at 0°C. A solution of the lithium hydroxide monohydrate (0.45 g) in H₂O (9.5 ml) was added dropwise over the course of 5 minutes. The mixture was stirred at 0°C for 2 hours. The reaction mixture was acidified by adding 0.5 M HCI and was then extracted twice with EtOAc. The organic layers were washed with water and brine. The combined organic layer was dried over Na₂SO₄ and evaporated *in vacuo*. The residue was taken up in diethyl ether and evaporated again. The remaining colorless foam was dried *in vacuo.*

### Synthesis of Example 2:

### Intermediate 2a):

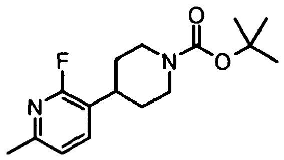

A flame dried Schlenk flask was charged with 3-bromo-2-fluoro-6-picoline (434 mg), [1,1'-bis(diphenylphosphino)ferrocene]dichloropalladium(II) complex with dichloromethane (56 mg), copper(I) iodide (26 mg), and dry *N,N*-dimethylacetamide (3 ml). The resulting mixture was degassed with alternating vacuum/argon purges. Then 1-tert-butoxycarbonylpiperidin-4-yl)(iodo)zinc (3.19 mmol, prepared as described above) was added. The mixture was degassed once again and then heated to 80°C overnight. The main part of *N,N*-dimethylacetamide was then evaporated and the remainder was taken up in a mixture of EtOAc and water (50 ml each). The mixture was then filtered through Celite and transferred into a separatory funnel. The phases were separated and the aqueous layer was extracted with EtOAc (2 x 50 ml). The combined organic layer was washed with water and brine (100 ml each), dried (Na₂SO₄), filtered and concentrated *in vacuo*. The crude product was purified by column chromatography to furnish the desired compound in form of a colorless oil.

### Intermediate 2b):

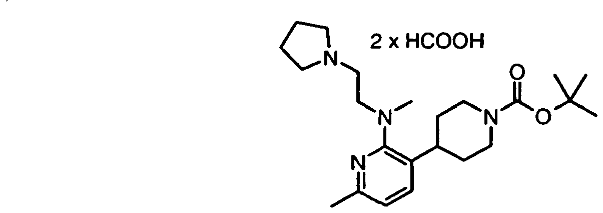

In a sealed tube were placed intermediate 2a) (0.55 g), methyl-(2-pyrrolidin-1-yl-ethyl)-amine (2.10 g), and N,N-diisopropylethylamine (286 µl). The reaction mixture was heated to 150°C for 4 d. The main part of the volatiles was evaporated and the remainder taken up in EtOAc (100 ml). The solution was washed with NaHCO₃ (2 x 25 ml) and the combined water layer was re-extracted with EtOAc (25 ml). The organic layers were merged and washed with brine (25 ml), dried (Na₂SO₄), filtered and evaporated. The crude product was purified by column chromatography to isolate unreacted starting material and a mixed fraction containing the desired product. The desired compound was finally isolated by preparative HPLC under acidic conditions in form of a brownish resin.

### Intermediate 2c):

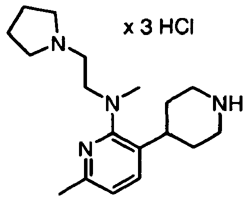

To Boc-protected intermediate 2b) (69 mg) in a mixture of DCM (1 ml) and MeOH (1 ml) was added hydrogen chloride, 4.0 M sol. in 1,4-dioxane (2 ml) and the solution was stirred at room temperature for 1 h. Evaporation of all volatiles and co-evaporation with toluene (2 x 5 ml), and acetone (5 ml) led to a beige solid, which was dried further in a desiccator over Sicapent overnight.

### Example 2:

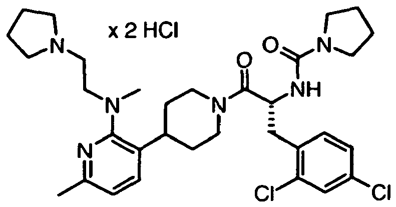

Intermediate 2c) (68 mg) and B-C Moiety 1 (60 mg), 1-hydroxybenzotriazole hydrate (32 mg) and *N*-methylmorpholine (58 µl) were dissolved in DMF (3 ml). After being stirred at room temperature for 30 min *N*-(3-dimethylaminopropyl)-*N'*-ethylcarbodiimide hydrochloride (48 mg) was added and stirring was continued for another hour. An additional amount of *N*-methylmorpholine (12 µl) was added and stirring was continued overnight. The reaction mixture was diluted with EtOAc (70 ml), washed with sat. Na₂CO₃ (3 x 25 ml), H₂O and brine (each with 25 ml). The organic layer was dried (Na₂SO₄) and the solvent removed *in vacuo*. Purification of the crude product by column chromatography furnished the corresponding amine in form of a yellowish resin. This was dissolved in EtOAc (1 ml) and treated with hydrogen chloride solution, 1.0 M in diethyl ether (114 µl). The resulting suspension was diluted with hexane (5 ml) in order to obtain a complete precipitation of the corresponding dihydrochloride. The solid was filtered off, washed with hexane, and dried in a desiccator over Sicapent overnight to provide the desired compound in form of an off-white solid.

### Synthesis of Example 4:

### Intermediate 4a):

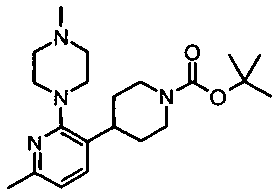

To a cooled solution (0°C) of 1-methylpiperazine (257 µl) in dry THF (2 ml) was added n-butyllithium, 2.5 M in hexane (0.50 ml) dropwise via syringe. After being stirred at room temperature for 15 min, the resulting lithium amide was then added dropwise to a solution of 2a) (387 mg) in dry THF (2 ml) at 0°C. After 2.5 h the reaction mixture was hydrolyzed with sat. NH₄Cl (2 ml) and diluted with EtOAc (70 ml). Extraction of the organic layer with NaHCO₃ (2 x 25 ml) and brine (25 ml), drying over Na₂SO₄, filtration and concentration *in vacuo* led to a yellow oil. This was purified by column chromatography to afford the desired compound in form of a yellowish resin.

### Intermediate 4b):

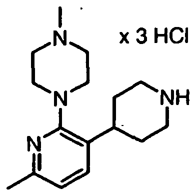

To Boc-protected intermediate 4a) (117 mg) in a mixture of DCM (1 ml) and MeOH (1 ml) was added hydrogen chloride, 4.0 M sol. in 1,4-dioxane (2 ml) and the solution was stirred at room temperature for 1 h. Evaporation of all volatiles and co-evaporation with toluene (2 x 5 ml), and acetone (5 ml) led to a beige solid, which was dried further in a desiccator over Sicapent overnight.

### Example 4:

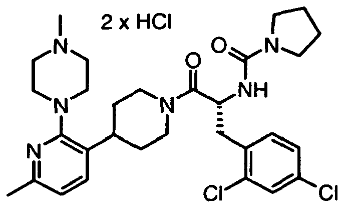

Intermediate 4b) (66 mg) and B-C Moiety 1 (70 mg), 1-hydroxybenzotriazole hydrate (37 mg) and *N*-methylmorpholine (68 µl) were dissolved in DMF (3 ml). After being stirred at room temperature for 30 min *N*-(3-dimethylaminopropyl)-*N*-ethylcarbodiimide hydrochloride (56 mg) was added and stirring was continued for another hour. An additional amount of *N*-methylmorpholine (14 µl) was added and stirring was continued overnight. The reaction mixture was diluted with EtOAc (70 ml), washed with sat. Na₂CO₃ (3 x 25 ml), H₂O and brine (each with 25 ml). The organic layer was dried (Na₂SO₄) and the solvent removed *in vacuo*. Purification of the crude product by column chromatography furnished the corresponding amine in form of a yellowish solid. This was dissolved in EtOAc (1 ml) and treated with hydrogen chloride solution, 1.0 M in diethyl ether (214 µl). The resulting suspension was diluted with hexane (5 ml) in order to obtain a complete precipitation of the corresponding dihydrochloride. The solid was filtered off, washed with hexane, and dried in a desiccator over Sicapent overnight to provide the desired compound in form of a white solid.

### Synthesis of Example 9:

### Intermediate 9a):

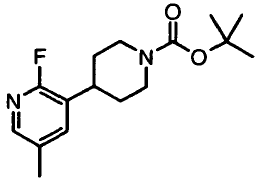

A flame dried Schlenk flask was charged with 3-bromo-2-fluoro-5-picoline (1.00 g), [1,1'-bis(diphenylphosphino)ferrocene]dichloropalladium(II) complex with dichloromethane (129 mg), copper(I) iodide (60 mg), and dry *N,N*-dimethylacetamide (7 ml). The resulting mixture was degassed with alternating vacuum/argon purges. Then 1-tert-butoxycarbonylpiperidin-4-yl)(iodo)zinc (7.37 mmol, prepared as described in above) was added. The mixture was degassed once again and then heated to 80°C overnight. The main part of *N,N*-dimethylacetamide was then evaporated and the remainder was taken up in a mixture of EtOAc and water (100 ml each). The mixture was then filtered through Celite and transferred into a separatory funnel. The phases were separated and the water layer was extracted with EtOAc (3 x 50 ml). The combined organic layer was washed with water and brine (100 ml each), dried (Na₂SO₄), filtered and concentrated *in vacuo*. The crude product was purified by column chromatography to furnish the desired compound in form of a white solid.

### Intermediate 9b):

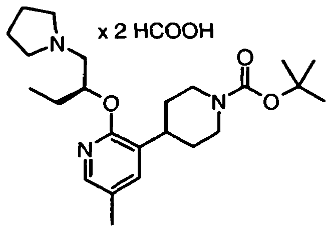

A solution of (S)-1-pyrrolidin-1-yl-butan-2-ol (191 mg) in DMF (1 ml) was added under argon at 0°C to a suspension of sodium hydride, 60% dispersion in mineral oil (36 mg) in DMF (1 ml). The cooling bath was removed and the mixture stirred at room temperature for 1 h. Then a solution of intermediate 9a) (158 mg) in DMF (1 ml) was added and the reaction mixture was heated to 60°C for 1 h. The reaction mixture was cooled to 0°C and was then hyrolyzed with sat. NH₄Cl (1 ml). EtOAc (70 ml) was added and the mixture was washed with NaHCO₃ (2 x 25 ml). The combined aqueous layer was re-extracted with EtOAc (25 ml) and the combined organic layer then washed with brine (25 ml), dried (Na₂SO₄), filtered and concentrated *in vacuo*. The crude product was purified by preparative HPLC to afford the corresponding diformate in form of a yellowish oil.

### Intermediate 9c):

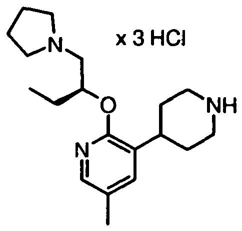

To Boc-protected intermediate 9b) (125 mg) in a mixture of DCM (2 ml) and MeOH (0.5 ml) was added hydrogen chloride, 4.0 M sol. in 1,4-dioxane (2 ml) and the solution was stirred at room temperature for 30 min. Evaporation of all volatiles and co-evaporation with toluene, acetone, and diethyl ether (5 ml each) led to a white solid, which was dried further in a desiccator over Sicapent overnight.

### Example 9:

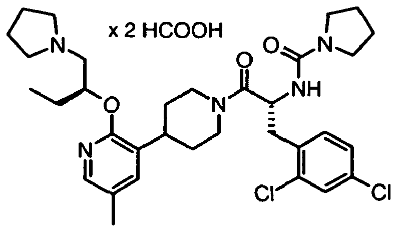

Intermediate 9c) (55 mg) and B-C Moiety 1 (55 mg), 1-hydroxybenzotriazole hydrate (29 mg) and *N*-methylmorpholine (54 µl) were dissolved in DMF (3 ml). After being stirred at room temperature for 30 min, *N*-(3-dimethylaminopropyl)-*N'*-ethylcarbodiimide hydrochloride (44 mg) was added and stirring was continued for another hour. An additional amount of *N*-methylmorpholine (11 µl) was added and stirring was continued overnight. The reaction mixture was diluted with EtOAc (70 ml), washed with sat. Na₂CO₃ (3 x 25 ml), water and brine (25 ml each). The organic layer was dried (Na₂SO₄) and the solvent removed *in vacuo*. The crude product was purified by preparative HPLC to afford the corresponding diformate in form of a brownish resin.

### Synthesis of Example 11:

### Intermediate 11a):

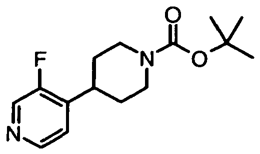

A flame dried Schlenk flask was charged with 3-fluoro-4-iodopyridine (1.00 g), [1,1'-bis(diphenylphosphino)ferrocene]dichloropalladium(II) complex with dichloromethane (110 mg), copper(l) iodide (51 mg), and dry *N,N*-dimethylacetamide (6 ml). The resulting mixture was degassed with alternating vacuum/argon purges. Then 1-tert-butoxycarbonylpiperidin-4-yl)(iodo)zinc (6.28 mmol, prepared as described above) was added. The mixture was degassed once again and then heated to 80°C overnight. The mainpart of *N,N*-dimethylacetamide was then evaporated and the remainder was taken up in a mixture of EtOAc and water (each with 100 ml). The mixture was then filtered through Celite and transferred into a separatory funnel. The phases were separated and the water layer was extracted with EtOAc (3 x 30 ml). The combined organic layer was washed with water and brine (each with 75 ml), dried (Na₂SO₄), filtered and concentrated *in vacuo*. The crude product was purified by column chromatography to furnish the desired compound in form of a brownish solid.

### Intermediate 11b):

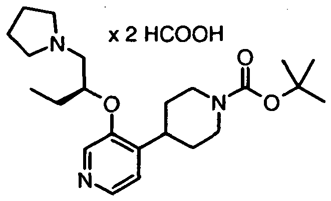

A solution of (S)-1-pyrrolidin-1-yl-butan-2-ol (156 mg) in DMF (2 ml) was added under argon at 0°C to a suspension of sodium hydride, 60% dispersion in mineral oil (29 mg) in DMF (1 ml). The cooling bath was removed and the mixture stirred for 1 h at room temperature. Then a solution of intermediate 11a) (102 mg) in DMF (2 ml) was added and the reaction mixture was heated to 120°C overnight. The reaction mixture was cooled to 0°C and was then hyrolyzed with sat. NH₄Cl (1 ml). EtOAc (50 ml) was added and the mixture was washed with NaHCO₃ (2 x 25 ml). The combined aqueous layer was re-extracted with EtOAc (25 ml) and the combined organic layer then washed with brine (25 ml), dried (Na₂SO₄), filtered and concentrated *in vacuo*. The crude product was purified by preparative HPLC to afford the corresponding diformate in form of a yellowish resin.

### Intermediate 11c):

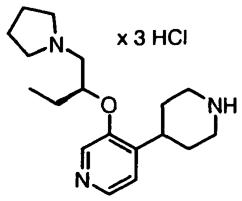

To Boc-protected intermediate 11b) (111 mg) in a mixture of DCM (1 ml) and MeOH (1 ml) was added hydrogen chloride, 4.0 M sol. in 1,4-dioxane (2 ml) and the solution was stirred at room temperature for 15 min. Evaporation of all volatiles and co-evaporation with toluene (2 x 5 ml), and acetone (5 ml) led to a beige solid, which was dried further in a desiccator over Sicapent overnight.

### Example 11:

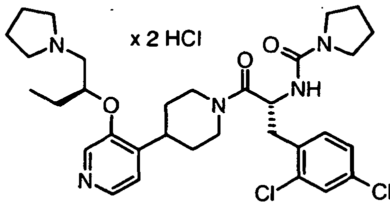

Intermediate 11c) (69 mg) and B-C Moiety 1 (58 mg), 1-hydroxybenzotriazole hydrate (31 mg) and *N*-methylmorpholine (57 µl) were dissolved in DMF (5 ml). After being stirred at room temperature for 30 min *N*-(3-dimethylaminopropyl)-*N'*-ethylcarbodiimide hydrochloride (47 mg) was added and stirring was continued for another hour. An additional amount of *N*-methylmorpholine (12 µl) was added and stirring was continued overnight. The reaction mixture was diluted with EtOAc (70 ml), washed with sat. Na₂CO₃ (3 x 25 ml), H₂O and brine (each with 25 ml). The organic layer was dried (Na₂SO₄) and the solvent removed *in vacuo*. Purification of the crude product by column chromatography furnished the corresponding amine in form of a yellowish resin. This was dissolved in EtOAc (1 ml) and treated with hydrogen chloride solution, 1.0 M in diethyl ether (143 µl). The resulting suspension was diluted with hexane (5 ml) in order to obtain a complete precipitation of the corresponding dihydrochloride. The solid was filtered off, washed with hexane, and dried in a desiccator over Sicapent overnight to provide the desired compound in form of a beige solid.

### Synthesis of Example 12:

### Intermediate 12a):

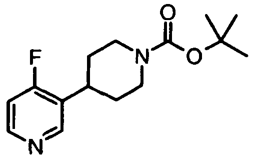

A flame dried Schlenk flask was charged with 4-fluoro-3-iodo-pyridine (1.00 g), [1,1'-bis(diphenylphosphino)ferrocene]dichloropalladium(II) complex with dichloromethane (110 mg), copper(I) iodide (51 mg), and dry *N,N*-dimethylacetamide (6 ml). The resulting mixture was degassed with alternating vacuum/argon purges. Then 1-tert-butoxycarbonylpiperidin-4-yl)(iodo)zinc (6.28 mmol, prepared as described above) was added. The mixture was degassed once again and then heated to 80°C overnight. The main part of *N,N*-dimethylacetamide was then evaporated and the remainder was taken up in a mixture of EtOAc and water (each with 100 ml). The mixture was then filtered through Celite and transferred into a separatory funnel. The phases were separated and the water layer was extracted with EtOAc (3 x 30 ml). The combined organic layer was washed with water and brine (each with 75 ml), dried (Na₂SO₄), filtered and concentrated *in vacuo*. The crude product was purified by column chromatography to furnish the desired compound in form of a brownish solid.

### Intermediate 12b):

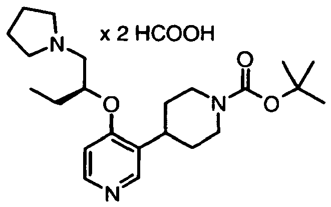

A solution of (S)-1-pyrrolidin-1-yl-butan-2-ol (187 mg) in DMF (2 ml) was added under argon at 0°C to a suspension of sodium hydride, 60% dispersion in mineral oil (35 mg) in DMF (1 ml). The cooling bath was removed and the mixture stirred for 1 h at room temperature. Then a solution of intermediate 12a) (122 mg) in DMF (2 ml) was added and the reaction mixture was heated to 60°C for 1 h. The reaction mixture was cooled to 0°C and was then hyrolyzed with sat. NH₄Cl (1 ml). EtOAc (70 ml) was added and the mixture was washed with NaHCO₃ (2 x 25 ml). The combined aqueous layer was re-extracted with EtOAc (25 ml) and the combined organic layer then washed with brine (25 ml), dried (Na₂SO₄), filtered and concentrated *in vacuo*. The crude product was purified by preparative HPLC to afford the corresponding diformate in form of a yellowish resin.

### Intermediate 12c):

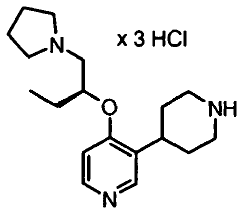

To Boc-protected intermediate 12b) (151 mg) in a mixture of DCM (1 ml) and MeOH (1 ml) was added hydrogen chloride, 4.0 M sol. in 1,4-dioxane (2 ml) and the solution was stirred at room temperature for 2 h. Evaporation of all volatiles and co-evaporation with toluene (2 x 5 ml), acetone, and ether (5 ml each) led to a beige solid, which was dried further in a desiccator over Sicapent overnight.

### Example 12:

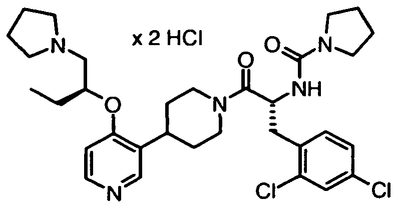

Intermediate 12c) (68 mg) and B-C Moiety 1 (61 mg), 1-hydroxybenzotriazole hydrate (33 mg) and *N*-methylmorpholine (59 µl) were dissolved in DMF (3 ml). After being stirred at room temperature for 30 min *N*-(3-dimethylaminopropyl)-*N'*-ethylcarbodiimide hydrochloride (49 mg) was added and stirring was continued for another hour. An additional amount of *N*-methylmorpholine (12 µl) was added and stirring was continued overnight. The reaction mixture was diluted with EtOAc (70 ml), washed with sat. Na₂CO₃ (3 x 25 ml), water and brine (each with 25 ml). The organic layer was dried (Na₂SO₄) and the solvent removed *in vacuo*. Purification of the crude product by column chromatography furnished the corresponding amine in form of a brownish resin. This was dissolved in EtOAc (1 ml) and treated with hydrogen chloride solution, 1.0 M in diethyl ether (178 µl). The resulting suspension was diluted with hexane (5 ml) in order to obtain a complete precipitation of the corresponding dihydrochloride. The solid was filtered off, washed with hexane, and dried in a desiccator over Sicapent overnight to provide the desired compound in form of an off-white solid.

### Synthesis of Example 14:

### Intermediate 14a):

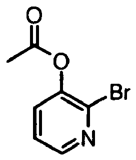

A solution of 2-bromo-3-pyridinol (1.04 g) in pyridine (2.00 ml) and dry DCM (20 ml) was cooled to 0°C. Acetic anhydride (1.76 ml) was added and the reaction mixture was stirred at room temperature overnight. All volatiles were evaporated and the remainder was suspended in water (30 ml). After being stirred at room temperature for 1 h, the suspension was diluted with 1 N NaHCO₃ (50 ml) and extracted with EtOAc (3 x 50 ml). The combined organic layer was washed with 1 N NaHCO₃, and brine (50 ml each), dried (MgSO₄), filtered and evaporated to afford the desired compound in form of an amber oil.

### Intermediate 14b):

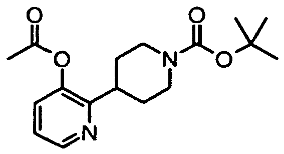

A flame dried Schlenk flask was charged with intermediate 14a) (2.00 g), [1,1'-bis(diphenylphosphino)ferrocene]dichloropalladium(II) complex with dichloromethane (227 mg), copper(I) iodide (106 mg), and dry *N,N*-dimethylacetamide (10 ml). The resulting mixture was degassed with alternating vacuum/argon purges. Then 1-tert-butoxycarbonylpiperidin-4-yl)(iodo)zinc (13.0 mmol, prepared as described above) was added. The mixture was degassed once again and then heated to 80°C overnight. The main part of *N,N*-dimethylacetamide was then evaporated and the remainder was taken up in a mixture of EtOAc and water (each with 200 ml). The mixture was then filtered through Celite and transferred into a separatory funnel. The phases were separated and the water layer was extracted with EtOAc (3 x 75 ml). The combined organic layer was washed with water and brine (150 ml each), dried (Na₂SO₄), filtered and concentrated *in vacuo*. The crude product was purified by column chromatography to furnish the desired compound in form of a brownish solid.

### Intermediate 14c):

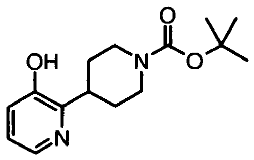

A solution of intermediate 14b) (1.97 g) in MeOH (60 ml) was cooled to 0°C. A solution of lithium hydroxide (368 mg) in water (30 ml) was added and the reaction mixture was stirred at 0°C for 10 min. The pH was adjusted to -7 by dropwise addition of 0.5 M HCI at 0°C. Water (100 ml) was added and an extraction with EtOAc (3 x 100 ml) was carried out. After washing with brine (150 ml), drying (Na₂SO₄), filtration and evaporation the desired compound was obtained as brownish foam.

### Intermediate 14d):

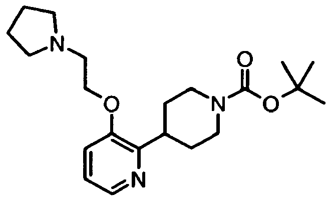

A solution of intermediate 14c) (207 mg), 1-(2-chloroethyl)pyrrolidine hydrochloride (190 mg), and cesium carbonate (969 mg) in DMF (10 ml) was stirred at room temperature overnight. All volatiles were evaporated and the residue was partitioned between EtOAc and sat. NaHCO₃ (50 ml each). The aqueous layer was extracted with EtOAc (2 x 25 ml). The combined organic layer was washed with water and brine (25 ml each), dried (Na₂SO₄) and evaporated to afford the desired compound as brownish resin.

### Intermediate 14e):

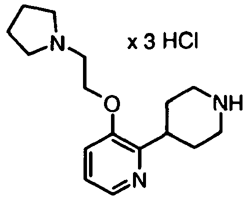

To Boc-protected intermediate 14d) (253 mg) in a mixture of DCM (2 ml) and MeOH (0.5 ml) was added hydrogen chloride, 4.0 M sol. in 1,4-dioxane (2 ml) and the solution was stirred at room temperature for 15 min. Evaporation of all volatiles and co-evaporation with toluene, acetone, and ether (5 ml each) led to a beige solid, which was dried further in a desiccator over Sicapent overnight.

### Example 14:

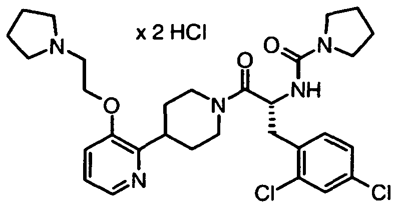

Intermediate 14e) (180 mg) and B-C Moiety 1 (201 mg), 1-hydroxybenzotriazole hydrate (107 mg) and *N*-methylmorpholine (195 µl) were dissolved in DMF (5 ml). After being stirred at room temperature for 30 min *N*-(3-dimethylaminopropyl)-*N'*-ethylcarbodiimide hydrochloride (161 mg) was added and stirring was continued for another hour. An additional amount of *N*-methylmorpholine (41 µl) was added and stirring was continued overnight. The reaction mixture was diluted with EtOAc (100 ml), washed with sat. Na₂CO₃ (3 x 50 ml), water and brine (each with 50 ml). The organic layer was dried (Na₂SO₄) and the solvent removed *in vacuo*. Purification of the crude product by column chromatography furnished the corresponding amine in form of a brownish resin. This was dissolved in EtOAc (1 ml) and treated with hydrogen chloride solution, 1.0 M in diethyl ether (564 µl). The resulting suspension was diluted with hexane (5 ml) in order to obtain a complete precipitation of the corresponding dihydrochloride. The solid was filtered off, washed with hexane, and dried in a desiccator over Sicapent overnight to provide the desired compound in form of a white solid.

### Synthesis of Example 21:

### Intermediate 21a):

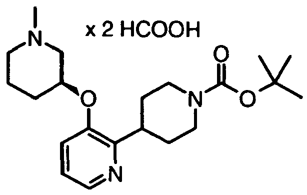

A solution of (S)-1-methyl-piperidin-3-ol (228 mg) in DMF (2 ml) was added under argon at 0°C to a suspension of sodium hydride, 60% dispersion in mineral oil (53 mg) in DMF (1 ml). The cooling bath was removed and the mixture stirred at room temperature for 1 h. Then a solution of 3-fluoro-3',4',5',6'-tetrahydro-2'H-[2,4']bipyridinyl-1'-carboxylic acid tert-butyl ester (185 mg) in DMF (2 ml) was added and the reaction mixture was heated to 120°C overnight. The reaction mixture was cooled to 0°C and then hyrolyzed with sat. NH₄Cl (1 ml). EtOAc (50 ml) was added and the mixture was washed with NaHCO₃ (2 x 25 ml). The combined aqueous layer was re-extracted with EtOAc (25 ml) and the combined organic layer then washed with brine (25 ml), dried (Na₂SO₄), filtered and concentrated *in vacuo.* The crude product was purified by preparative HPLC to afford the corresponding diformate in form of a yellowish resin.

### Intermediate 21b):

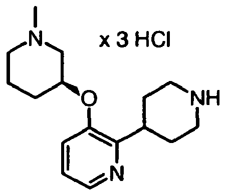

To Boc-protected intermediate 21a) (148 mg) in a mixture of DCM (1 ml) and MeOH (1 ml) was added hydrogen chloride, 4.0 M sol. in 1,4-dioxane (2 ml) and the solution was stirred at room temperature for 15 min. Evaporation of all volatiles and co-evaporation with toluene (2 x 5 ml), and acetone (5 ml) led to a beige solid, which was dried further in a desiccator over Sicapent overnight.

### Example 21:

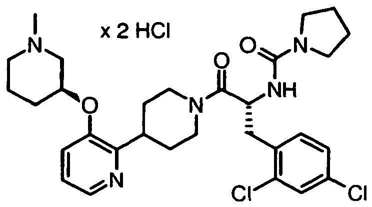

Intermediate 21b) (93 mg) and B-C Moiety 1 (83 mg), 1-hydroxybenzotriazole hydrate (44 mg) and *N*-methylmorpholine (81 µl) were dissolved in DMF (5 ml). After being stirred at room temperature for 30 min *N*-(3-dimethylaminopropyl)-*N'*-ethylcarbodiimide hydrochloride (67 mg) was added and stirring was continued for another hour. An additional amount of *N*-methylmorpholine (17 µl) was added and stirring was continued overnight. The reaction mixture was diluted with EtOAc (70 ml), washed with sat. Na₂CO₃ (3 x 25 ml), water and brine (each with 25 ml). The organic layer was dried (Na₂SO₄) and the solvent removed *in vacuo*. Purification of the crude product by column chromatography furnished the corresponding amine in form of a yellowish resin. This was dissolved in EtOAc (1 ml) and treated with hydrogen chloride solution, 1.0 M in diethyl ether (238 µl). The resulting suspension was diluted with hexane (5 ml) in order to obtain a complete precipitation of the corresponding dihydrochloride. The solid was filtered off, washed with hexane, and dried in a desiccator over Sicapent overnight to provide the desired compound in form of a white solid.

### Synthesis of Example 28:

### Intermediate 28a):

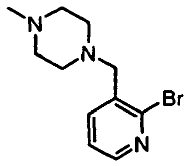

Sodium triacetoxyborohydride (1512 mg) was added to a solution of 2-bromo-3-pyridinecarboxaldehyde (948 mg) and 1-methylpiperazine (566 µl) in 1,2-dichloroethane (20 ml). After being stirred at room temperature for 1 h, the mixture was diluted with EtOAc (100 ml) and washed with sat. NaHCO₃ (2 x 50 ml), H₂O and brine (each with 50 ml). After drying over Na₂SO₄, filtration and evaporation of the solvent a brownish oil was obtained. Purification by chromatography afforded the desired compound in form of a yellowish oil.

### Intermediate 28b):

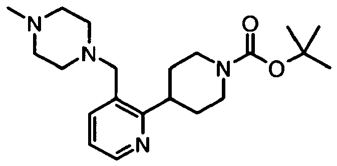

A flame dried Schlenk flask was charged with intermediate 28a) (729 mg), [1,1'-bis(diphenylphosphino)ferrocene]dichloropalladium(II) complex with dichloromethane (66 mg), copper(I) iodide (31 mg), and dry *N,N*-dimethylacetamide (4 ml). The resulting mixture was degassed with alternating vacuum/argon purges. Then 1-tert-butoxycarbonylpiperidin-4-yl)(iodo)zinc (3.78 mmol, prepared as described above) was added. The mixture was degassed once again and then heated to 80°C overnight. The main part of *N,N*-dimethylacetamide was then evaporated and the remainder was taken up in a mixture of EtOAc and water (each with 50 ml). This was then filtered through Celite and transferred into a separatory funnel. The phases were separated and the water layer was extracted with EtOAc (2 x 50 ml). The combined organic layer was washed with brine (75 ml), dried (Na₂SO₄), filtered and concentrated *in vacuo*. The crude product was purified by chromatography to furnish the desired compound in form of a brown oil.

### Intermediate 28c):

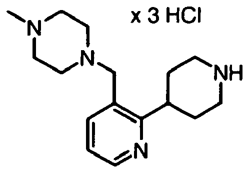

To Boc-protected intermediate 28b) (383 mg) in a mixture of DCM (3 ml) and MeOH (3 ml) was added hydrogen chloride, 4.0 M sol. in 1,4-dioxane (6 ml) and the solution was stirred at room temperature for 1 h. Evaporation of all volatiles and co-evaporation with toluene and acetone (each with 20 ml) led to a light brown solid, which was dried further in a desiccator (Sicapent) overnight.

### Example 28:

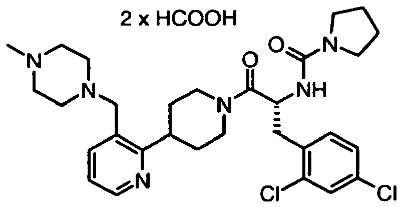

Intermediate 28c) (165 mg) and B-C moiety 1 (152 mg), 1-hydroxy-benzotriazole hydrate (80 mg) and *N*-methylmorpholine (147 µl) were dissolved in DMF (2 ml). After being stirred for 30 min at room temperature *N*-(3-dimethylaminopropyl)-*N*-ethylcarbodiimide hydrochloride (121 mg) was added and stirring was continued for another hour. An additional amount of *N*-methylmorpholine (31 µl) was added and stirring was continued overnight. The reaction mixture was diluted with EtOAc (70 ml), washed with sat. Na₂CO₃ (3 x 25 ml), water and brine (each with 25 ml). The organic layer was dried (Na₂SO₄) and the solvent removed *in vacuo*. Purification of the crude product by preparative HPLC furnished the corresponding diformate as slightly brownish resin.

### Synthesis of Example 41:

### Intermediate 41a):

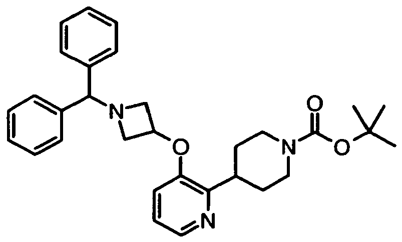

A solution of 1-benzhydrylazetan-3-ol (674 mg) in DMF (2 ml) was added under argon at 0°C to a suspension of sodium hydride, 60% dispersion in mineral oil (75 mg) in DMF (2 ml). The cooling bath was removed and the mixture stirred for 1 h at room temperature. Then a solution of 3-fluoro-3',4',5',6'-tetrahydro-2'H-[2,4']bipyridinyl-1'-carboxylic acid tert-butyl ester (263 mg) in DMF (2 ml) was added and the reaction mixture was heated to 120°C overnight. The reaction mixture was cooled to 0°C and was then hydrolyzed with sat. NH₄Cl (1 ml). EtOAc (50 ml) was added and the mixture was washed with NaHCO₃ (2 x 25 ml). The combined aqueous layer was re-extracted with EtOAc (25 ml) and the combined organic layer then washed with water and brine (each with 25 ml), dried (Na₂SO₄), filtered and concentrated *in vacuo*. The crude product was purified by chromatography to afford the desired product as yellowish foam.

### Intermediate 41b):

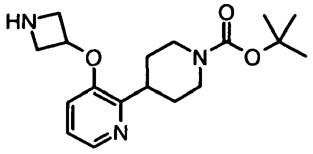

Intermediate 41a) (122 mg) was hydrogenated overnight in the presence of palladium hydroxide, 20 wt% on carbon (10 mg) in a mixture of MeOH (2 ml) and AcOH (0.2 ml) under 1 bar of hydrogen at room temperature. Filtration through a syringe filter and complete evaporation of the solvent led to the desired compound as colorless oil.

### Intermediate 41c):

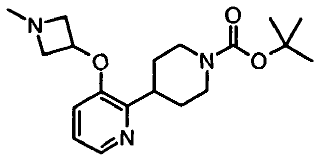

Sodium triacetoxyborohydride (72 mg) was added to a solution of intermediate 41 b) (137 mg) and formaldehyde, 36.5% in water (18.5 µl) in 1,2-dichloroethane (2 ml). The reaction mixture was stirred for 2 h at room temperature. Then the mixture was diluted with EtOAc (50 ml) and washed with sat. NaHCO₃ (2 x 25 ml) and H₂O (25 ml). The aqueous layers were merged and reextracted with EtOAc (25 ml). All organic layers were merged, washed with brine (25 ml), dried (Na₂SO₄) and evaporated. The crude product was purified by chromatography to provide the desired product as colorless oil.

### Intermediate 41d):

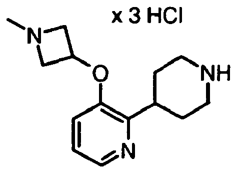

To Boc-protected intermediate 41c) (50 mg) in a mixture of DCM (1 ml) and MeOH (1 ml) was added hydrogen chloride, 4.0 M sol. in 1,4-dioxane (2 ml) and the solution was stirred at room temperature for 1 h. Evaporation of all volatiles and co-evaporation with toluene and acetone (5 ml each) led to a white solid, which was dried further in a desiccator (Sicapent) overnight.

### Example 41:

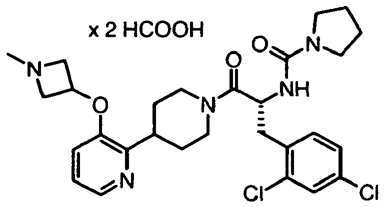

Intermediate 41d) (51 mg) and B-C moiety 1 (62 mg), 1-hydroxy-benzotriazole hydrate (33 mg) and *N*-methylmorpholine (60 µl) were dissolved in DMF (2 ml). After being stirred at room temperature for 30 min *N*-(3-dimethylaminopropyl)-*N*-ethylcarbodiimide hydrochloride (50 mg) was added and stirring was continued for another hour. An additional amount of *N*-methylmorpholine (13 µl) was added and stirring was continued overnight. The reaction mixture was diluted with EtOAc (70 ml) and washed with sat. Na₂CO₃ (3 x 25 ml), water and brine (each with 25 ml). The organic layer was dried (Na₂SO₄) and the solvent removed *in vacuo.* Purification of the crude product by preparative HPLC furnished the corresponding diformate as colorless, glassy solid.

### Synthesis of Example 57:

### Intermediate 57a):

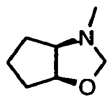

A mixture of cis-(1*S*,2*R*)-2-aminocyclopentanol hydrochloride (6.55 g), formaldehyde solution 36.5% in water (12.2 ml) and formic acid (7.18 ml) in water (30 ml) was heated to reflux overnight. The reaction mixture was concentrated *in vacuo* and the remainder was made alkaline (pH 14) by addition of 1 N NaOH. Furthermore, solid NaCl was added until saturation. The aqueous phase was extracted with DCM (3 x 50 ml) and the combined organic layer was washed with water and brine (each with 30 ml), then dried (Na₂SO₄) and concentrated *in vacuo* to provide a yellowish liquid.

### Intermediate 57b):

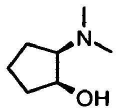

Intermediate 57a) (1.27 g) was dissolved in abs. EtOH (50 ml). sodium borohydride (1.13 g) was added in small portions at room temperature. After being stirred overnight, sat. NH₄Cl (10 ml) was added, and the main part of EtOH was removed *in vacuo.* The residue was diluted with 0.25 N NaOH (50 ml) and solid NaCl was added until saturation. The aqueous phase was extracted with DCM (3 x 25 ml) and the combined organic layer was washed with water and brine (each with 25 ml), then dried (Na₂SO₄) and concentrated *in vacuo.* The crude product was purified by chromatography to afford the desired product in form of colorless crystals.

### Intermediate 57c):

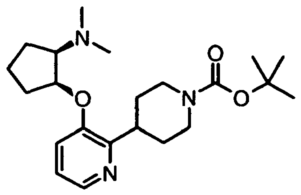

A solution of intermediate 57b) (333 mg) in DMF (1 ml) was added under argon at 0°C to a suspension of sodium hydride, 60% dispersion in mineral oil (69 mg) in DMF (3 ml). The cooling bath was removed and the mixture stirred for 1 h at room temperature. Then a solution of 3-fluoro-3',4',5',6'-tetrahydro-2'H-[2,4']bipyridinyl-1'-carboxylic acid tert-butyl ester (241 mg) in DMF (1 ml) was added and the reaction mixture was heated to 120°C overnight. The reaction mixture was cooled to 0°C and was then quenched with sat. NH₄Cl (1 ml). EtOAc (70 ml) was added and the mixture was washed with NaHCO₃ (2 x 30 ml). The combined aqueous layer was re-extracted with EtOAc (30 ml) and the combined organic layer then washed with brine (50 ml), dried (Na₂SO₄), filtered and concentrated *in vacuo.* The crude product was purified by chromatography to afford the desired product as brownish oil.

### Intermediate 57d):

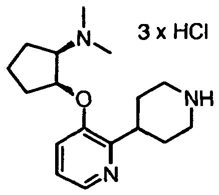

To Boc-protected intermediate 57c) (270 mg) in a mixture of DCM (1 ml) and MeOH (1 ml) was added hydrogen chloride, 4.0 M sol. in 1,4-dioxane (2 ml) and the solution was stirred at room temperature for 10 min. Evaporation of all volatiles and co-evaporation with toluene and acetone (each with 5 ml) led to a beige solid, which was dried further in a desiccator (Sicapent) overnight.

### Example 57:

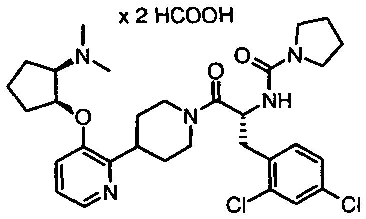

Intermediate 57d) (110 mg) and B-C moiety 1 (100 mg), 1-hydroxy-benzotriazole hydrate (53 mg) and *N*-methylmorpholine (97 µl) were dissolved in DMF (2 ml). After being stirred at room temperature for 30 min *N*-(3-dimethylaminopropyl)-*N*-ethylcarbodiimide hydrochloride (80 mg) was added and stirring was continued for another hour. An additional amount of *N*-methylmorpholine (20 µl) was added and stirring was continued overnight. The reaction mixture was diluted with EtOAc (70 ml) and washed with sat. Na₂CO₃ (3 x 25 ml), water and brine (each with 25 ml). The organic layer was dried (Na₂SO₄) and the solvent removed *in vacuo.* Purification of the crude product by preparative HPLC furnished the corresponding diformate as colorless, glassy solid.

### Synthesis of Example 59:

### Intermediate 59a):

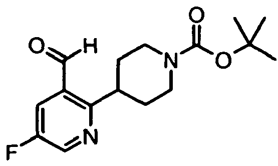

A flame dried Schlenk flask was charged with 2-chloro-5-fluoro-3-formylpyridine (1596 mg), [1,1'-bis(diphenylphosphino)ferrocene]dichloropalladium(II) complex with dichloromethane (245 mg), copper(I) iodide (114 mg), and dry *N*,*N*-dimethylacetamide (14 ml). The resulting mixture was degassed with alternating vacuum/argon purges. Then 1-tert-butoxycarbonylpiperidin-4-yl)(iodo)zinc (14.0 mmol, prepared as described above) was added. The mixture was degassed once again and then heated to 80°C overnight. The main part of *N*,*N*-dimethylacetamide was then evaporated and the remainder was taken up in a mixture of EtOAc and water (each with 75 ml). This was then filtered through Celite and transferred into a separatory funnel. The phases were separated and the water layer was extracted with EtOAc (2 x 100 ml). The combined organic layer was washed with brine (100 ml), dried (Na₂SO₄), filtered and concentrated *in vacuo.* The crude product was purified by column chromatography to furnish the desired compound in form of a yellow resin.

### Intermediate 59b):

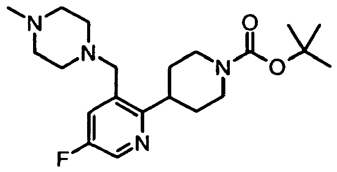

Sodium triacetoxyborohydride (585 mg) was added to a solution of intermediate 59a) (607 mg) and 1-methylpiperazine (219 µl) in 1,2-dichloroethane (10 ml). After being stirred at room temperature for 3 h, more sodium triacetoxyborohydride (209 mg) was added and stirring was continued onvernight. The mixture was diluted with EtOAc (100 ml) and washed with sat. NaHCO₃ (2 x 50 ml), H₂O (50 ml) and brine (75 ml). After drying over Na₂SO₄, filtration and evaporation of the solvent a brownish resin was obtained. Purification by chromatography afforded the desired compound in form of a brownish oil.

### Intermediate 59c):

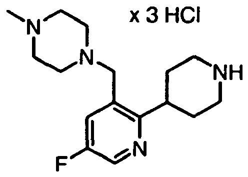

To Boc-protected intermediate 59b) (504 mg) in a mixture of DCM (1.5 ml) and MeOH (3.5 ml) was added hydrogen chloride, 4.0 M sol. in 1,4-dioxane (3 ml) and the solution was stirred at room temperature for 1.5 h. Evaporation of all volatiles and co-evaporation with toluene (2 x 10 ml) and acetone (15 ml) led to a brown solid, which was dried further in a desiccator (Sicapent) overnight.

### Example 59:

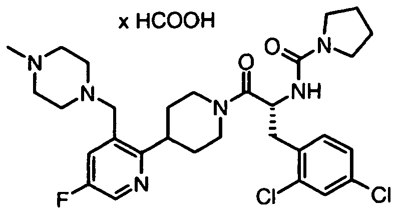

Intermediate 59c) (150 mg) and B-C moiety 1 (129 mg), 1-hydroxy-benzotriazole hydrate (69 mg) and *N*-methylmorpholine (125 µl) were dissolved in DMF (5 ml). After being stirred at room temperature for 30 min *N*-(3-dimethylaminopropyl)-*N*-ethylcarbodiimide hydrochloride (103 mg) was added and stirring was continued for another hour. An additional amount of *N*-methylmorpholine (26 µl) was added and stirring was continued overnight. The reaction mixture was diluted with EtOAc (50 ml) and washed with sat. Na₂CO₃ (3 x 25 ml). The combined aqueous layer was re-extracted with EtOAc (25 ml). Then all organic layers were merged and washed with brine (25 ml). The organic layer was dried (Na₂SO₄) and the solvent removed *in vacuo.* Purification of the crude product by preparative HPLC furnished the corresponding formate as yellowish resin.

### Synthesis of Examples 60 and 61:

### Intermediate 60 and 61a):

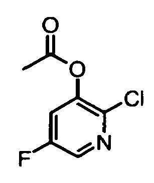

A solution of 2-chloro-5-fluoropyridin-3-ol (2.50 g) and pyridine (5.61 ml) in DCM (60 ml) was cooled to 0°C. To this acetic anhydride (4.96 ml) was added dropwise and the reaction mixture was stirred at room temperature overnight. All volatiles were evaporated and the residue was diluted with water (30 ml) and stirred for 1 h to hydrolyze unreacted acetic anhydride. This mixture was diluted with EtOAc (100 ml) and 0.5 M NaHCO₃ (150 ml). After phase separation, the aqueous layer was extracted with EtOAc (2 x 100 ml). The combined organic layer was re-extracted with 0.5 M NaHCO₃ and brine (100 ml each), dried over Na₂SO₄ and evaporated. Purification by chromatography afforded the desired compound in form of a colorless liquid.

### Intermediate 60 and 61b):

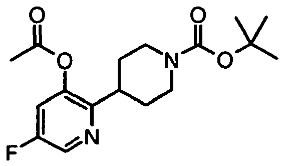

A flame dried Schlenk flask was charged with intermediate 60 and 61a) (2.38 g), [1,1'-bis(diphenylphosphino)ferrocene]dichloropalladium(II) complex with dichloromethane (307 mg), copper(I) iodide (143 mg), and dry *N*,*N*-dimethylacetamide (15 ml). The resulting mixture was degassed with alternating vacuum/argon purges. Then 1-tert-butoxycarbonylpiperidin-4-yl)(iodo)zinc (17.6 mmol, prepared as described above) was added. The mixture was degassed once again and then heated to 80°C overnight. The main part of *N*,*N*-dimethylacetamide was then evaporated and the remainder was taken up in a mixture of EtOAc and water (each with 75 ml). This was then filtered through Celite and transferred into a separatory funnel. The phases were separated and the water layer was extracted with EtOAc (2 x 50 ml). The combined organic layer was washed with water and brine (75 ml each), dried (Na₂SO₄), filtered and concentrated *in vacuo.* The crude product was purified by column chromatography to furnish the desired compound in form of a brownish oil.

### Intermediate 60 and 61c):

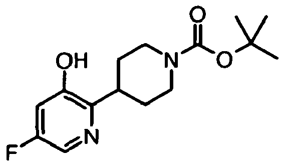

A solution of intermediate 60 and 61b) (1.82 g) in MeOH (50 ml) was cooled to 0°C. A solution of lithium hydroxide (0.32 g) in water (25 ml) was added at once and stirring was continued at 0°C. After 15 min the pH was adjusted to -7 by dropwise addition of 0.5 M HCl. Then the mixture was diluted with water (100 ml) and extracted with EtOAc (3 x 100 ml). The combined organic layer was washed with brine (100 ml), dried (Na₂SO₄) and evaporated to furnish a brownish foam.

### Intermediate 60 and 61d):

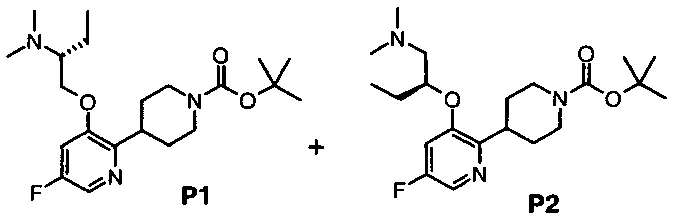

Under argon a solution of intermediate 60 and 61c) (375 mg), (R)-2-dimethylamino-butan-1-ol (297 mg), and triphenylphosphine (664 mg) in THF (15 ml) was cooled to 0°C. To this diethyl azodicarboxylate, 40% in toluene (1.16ml) was added dropwise. The cooling bath was removed and stirring was continued for 3 h at room temperature. Evaporation of all volatiles furnished a brown oil, which was first purified by chromatography (removal of Ph₃PO) and then by preparative HPLC to separate the isomers. Both isomers were obtained as brownish resins.

### Intermediate 60e):

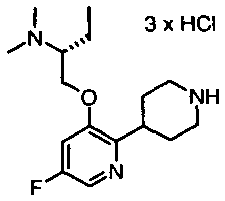

To Boc-protected intermediate 60 and 61d) P1 (82 mg) in a mixture of DCM (1 ml) and MeOH (1 ml) was added hydrogen chloride, 4.0 M sol. in 1,4-dioxane (2 ml) and the solution was stirred at room temperature for 10 min. Evaporation of all volatiles and co-evaporation with toluene and acetone (each with 5 ml) led to the desired product as white solid.

### Example 60:

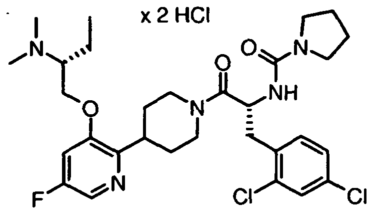

Intermediate 60e) (95 mg) and B-C moiety 1 (89 mg), 1-hydroxy-benzotriazole hydrate (47 mg) and *N*-methylmorpholine (86 µl) were dissolved in DMF (3 ml). After being stirred for 30 min at room temperature *N*-(3-dimethylaminopropyl)-*N*-ethylcarbodiimide hydrochloride (71 mg) was added and stirring was continued for another hour. An additional amount of N*-*methylmorpholine (18 µl) was added and stirring was continued overnight. The reaction mixture was diluted with EtOAc (50 ml) and washed with sat. Na₂CO₃ (3 x 25 ml) and water (25 ml). All aqueous layers were merged and re-extracted with EtOAc (25 ml). Then the combined organic layer was washed with brine (25 ml), dried (Na₂SO₄) and evaporated *in vacuo.* The residue was purified by preparative HPLC to provide the corresponding amine as yellowish resin. This was dissolved in EtOAc (1 ml) and treated with hydrogen chloride solution, 1.0 M in diethyl ether (207 µl). The resulting suspension was diluted with hexane (5 ml) in order to obtain a complete precipitation of the corresponding dihydrochloride. The solid was filtered off, washed with hexane, and dried in a desiccator (Sicapent) overnight to provide the desired compound in form of a white solid.

### Intermediate 61e):

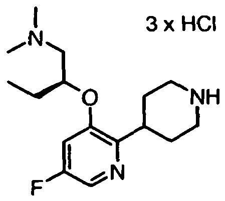

To Boc-protected intermediate 60 and 61 d) P2 (40 mg) in a mixture of DCM (1 ml) and MeOH (1 ml) was added hydrogen chloride, 4.0 M sol. in 1,4-dioxane (2 ml) and the solution was stirred at room temperature for 10 min. Evaporation of all volatiles and co-evaporation with toluene and acetone (each with 5 ml) led to the desired product as white solid.

### Example 61:

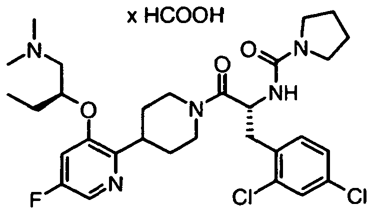

Intermediate 61e) (48 mg) and B-C moiety 1 (43 mg), 1-hydroxy-benzotriazole hydrate (23 mg) and *N*-methylmorpholine (42 µl) were dissolved in DMF (3 ml). After being stirred for 30 min at room temperature *N*-(3-dimethylaminopropyl)-*N*-ethylcarbodiimide hydrochloride (35 mg) was added and stirring was continued for another hour. An additional amount of *N*-methylmorpholine (9 µl) was added and stirring was continued overnight. The reaction mixture was diluted with EtOAc (50 ml) and washed with sat. Na₂CO₃ (3 x 25 ml) and water (25 ml). All aqueous layers were merged and re-extracted with EtOAc (25 ml). Then the combined organic layer was washed with brine (25 ml), dried (Na₂SO₄) and evaporated *in vacuo.* The crude product was purified by preparative HPLC to provide the corresponding formate as colorless resin.

### Synthesis of Example 63:

### Intermediate 63a):

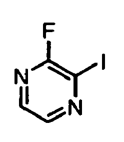

A solution of *n*-butyllithium, 2.5 M in hexane (8.97 ml) was added to cooled (-50°C), stirred, dry THF (200 ml) under an atmosphere of argon. Then 2,2,6,6-tetramethylpiperidine (4.13 ml) was added. The mixture was warmed to 0°C for 20 minutes. The mixture was then carried to -78°C. To this mixture a solution of fluoropyrazine (2.00 g) in THF (50 ml) was added. Stirring was continued at this temperature for 5 minutes. Then a solution of iodine (10.4 g) in THF (50 ml) was introduced and stirring was continued for 1 h at -78°C. Hydrolysis was then carried out at -78°C using a solution of 35% aqueous hydrochloric acid (20 ml), EtOH (20 ml) and THF (50 ml). The solution was warmed to room temperature, made slightly basic (pH 10) with sat. NaHCO₃. The solution was decolorized with sodium thiosulphate and evaporated to remove the organic solvents. The residue was diluted with water (150 ml) and extracted with DCM (3 x 200 ml). The organic extract was dried (Na₂SO₄) and evaporated. The crude product was purified by column chromatography to furnish the desired compound as yellowish crystals (mp. 41-44°C).

### Intermediate 63b):

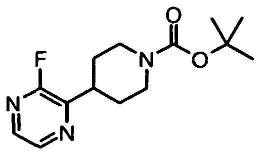

A flame dried Schlenk flask was charged with intermediate 63a) (1.00 g), [1,1'-bis(diphenylphosphino)ferrocene]dichloropalladium(II) complex with dichloromethane (109 mg), copper(I) iodide (51 mg), and dry *N*,*N*-dimethylacetamide (6 ml). The resulting mixture was degassed with alternating vacuum/argon purges. Then 1-tert-butoxycarbonylpiperidin-4-yl)(iodo)zinc (6.25 mmol, prepared as described above) was added. The mixture was degassed once again and then heated to 80°C overnight. The mainpart of *N*,*N*-dimethylacetamide was then evaporated and the remainder was taken up in a mixture of EtOAc and water (100 ml each). The mixture was then filtered through Celite and transferred into a separatory funnel. The phases were separated and the water layer was extracted with EtOAc (3 x 30 ml). The combined organic layer was washed with water and brine (75 ml each), dried (Na₂SO₄), filtered and concentrated *in vacuo.* The crude product was purified by column chromatography to furnish the desired compound in form of a brownish oil.

### Intermediate 63c):

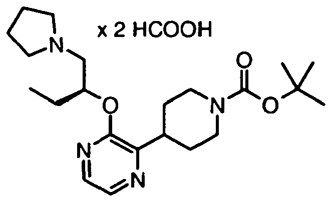

A solution of (S)-1-pyrrolidin-1-yl-butan-2-ol (211 mg) in DMF (2 ml) was added under argon at 0°C to a suspension of sodium hydride, 60% dispersion in mineral oil (39 mg) in DMF (1 ml). The cooling bath was removed and the mixture stirred for 1 h at room temperature. Then a solution of intermediate 63b) (138 mg) in DMF (2 ml) was added and the reaction mixture was heated to 60°C for 1 h. The reaction mixture was cooled to 0°C and was then hyrolyzed with sat. NH₄Cl (1 ml). EtOAc (70 ml) was added and the mixture was washed with NaHCO₃ (2 x 25 ml). The combined aqueous layer was re-extracted with EtOAc (25 ml) and the combined organic layer then washed with brine (25 ml), dried (Na₂SO₄), filtered and concentrated *in vacuo.* The crude product was purified by preparative HPLC to afford the corresponding formate in form of a yellowish resin.

### Intermediate 63d):

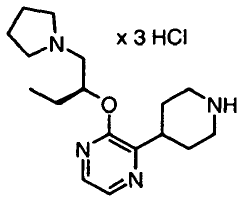

To Boc-protected intermediate 63c) (181 mg) in a mixture of DCM (1 ml) and MeOH (1 ml) was added hydrogen chloride, 4.0 M sol. in 1,4-dioxane (2 ml) and the solution was stirred at room temperature for 2 h. Evaporation of all volatiles and co-evaporation with toluene (2 x 5 ml), acetone (5 ml), and ether (5 ml) led to a beige solid, which was dried further in a desiccator over Sicapent overnight.

### Example 63:

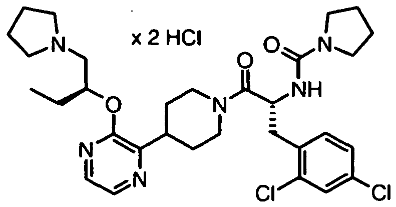

Intermediate 63d) (67 mg) and B-C Moiety 1) (68 mg), 1-hydroxybenzotriazole hydrate (36 mg) and *N*-methylmorpholine (66 µl) were dissolved in DMF (3 ml). After being stirred for 30 min at room temperature N-(3-dimethylaminopropyl)-*N*-ethylcarbodiimide hydrochloride (54 mg) was added and stirring was continued for another hour. An additional amount of *N*-methylmorpholine (14 µl) was added and stirring was continued overnight. The reaction mixture was diluted with EtOAc (70 ml), washed with sat. Na₂CO₃ (3 x 25 ml), water and brine (each with 25 ml). The organic layer was dried (Na₂SO₄) and the solvent removed *in vacuo.* Purification of the crude product by column chromatography furnished the corresponding amine in form of a brownish resin. This was dissolved in EtOAc (1 ml) and treated with hydrogen chloride solution, 1.0 M in diethyl ether (178 µl). The resulting suspension was diluted with hexane (5 ml) in order to obtain a complete precipitation of the corresponding hydrochloride. The solid was filtered off, washed with hexane, and dried in a desiccator over Sicapent overnight to provide the desired compound in form of an off-white solid.

### Synthesis of Example 64:

### Intermediate 64a):

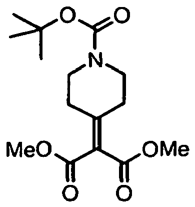

A three-necked flask equipped with an internal thermometer was charged with dry THF (80 ml) and cooled to -10°C under an argon atmosphere. While stirring with a magnetic stirrer, a 1 M solution of titanium tetrachloride (40 ml) was added dropwise within 30 min to maintain the temperature between -10°C and -5°C. After stirring an additional 30 min at -5°C, dimethyl malonate (2.286 ml) and N-Boc-piperidin-4-one (4.384 g) were added at -15°C to -10°C. The reaction mixture turned into a brown suspension. A solution of anhydrous pyridine (6.632 ml) in THF (14 ml) was added within 30 min while keeping the temperature below -10°C. The reaction became a clear brown solution. The cooling bath was removed and stirring was continued at room temperature for 46 h. The THF was evaporated and the remaining oil was suspended in 1 N NaHCO₃. The precipitating titanium salts were filtered off and washed with ethyl acetate. The filtrate was washed with a second portion of 1 N NaHCO₃ and brine. The org. phase was dried over MgSO₄, filtered and evaporated to yield an orange oil. It was purified by silica gel column chromatography.

### Intermediate 64b):

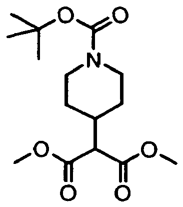

In a two-necked 250 ml flask, intermediate 64a) (3.16 g) was dissolved in a mixture of methanol (70 ml) and water (0.5 ml). The flask was evacuated and flushed with argon. 5% Palladium on activated charcoal (400 mg) was added and the flask was evacuated and flushed with hydrogen. The reaction mixture was vigorously stirred over night at room temperature under hydrogen atmosphere (1 atm). The catalyst was filtered through Celite and the filter was washed with methanol (150 ml). The filtrate was evaporated *in vacuo.* The crude product was taken up DCM (15 ml), filtered through a 0.45 µm syringe filter and evaporated *in vacuo.* A clear, colorless oil was obtained that was dried under high vacuum over night.

### Intermediate 64c):

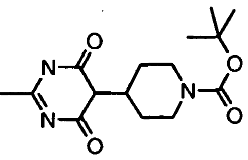

Sodium methoxide was prepared by dissolving sodium (306 mg) in cooled methanol (6.0 ml). Acetamidine hydrochloride (415 mg) was added to this mixture and the precipitating sodium chloride was removed by filtration through a 25 mm syringe filter. Intermediate 64b) (1.40 g) was added to the briskly stirred solution. A slightly cloudy solution formed that was kept at room temeprature for 2 days. The solvents were evaporated *in vacuo.* The residue was dissolved in water (10 ml) and the aqueous phase was extracted with toluene (6 ml). The organic phase was discarded. The aqueous layer was cooled to 0°C and 6 N HCl was added dropwise until pH 4 was reached. A colorless solid precipitated that was collected by suction filtration and washed with cold water (4 ml). The product was dried in vacuum.

### Intermediate 64d):

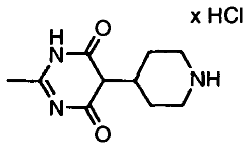

The Boc-protected intermediate 64c) was suspended in a mixture of dioxane (6 ml) and methanol (1 ml). 4 N HCl in dioxane (6 ml) was added. The reaction was stirred at room temperature for 1 h. Evaporation of all volatiles including co-evaporation with toluene (2 x 20 ml) furnished a white solid, which was dried in high vacuum overnight. The crude product was triturated with dry diethyl ether (6 ml). The solvent was decanted and the product was dried *in vacuo.*

### Intermediate 64e):

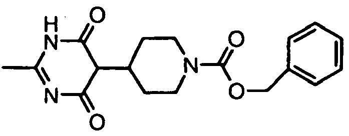

A solution of Z-OSu (546 mg) in DMF (10 ml) was treated with triethylamine (0.55 ml). A suspension of intermediate 64d) in DMF (3 ml) was added and the reaction was stirred at room temperature for 15 hours. The solvent was evaporated in vacuum and the remaining off-white solid was triturated with diethyl ether (2 x). The product was isolated by filtration and dried in vacuum.

### Intermediate 64f):

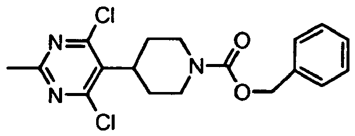

Intermediate 64e) was suspended in acetonitrile (4 ml) and treated with sym-collidine (0.231 ml). A solution of POCl₃ (0.54 ml) in acetonitrile (2 ml) was added dropwise at 0°C and the reaction was stirred at reflux temperature for 8 h. The solvent and excess reagents were removed by distillation in vacuum. The remaining brown solid was dissolved in ice cold ethyl acetate and extracted with water, 1 N NaHCO₃ and brine. The organic phase was dried over MgSO₄, filtered and evaporated in vacuum.

### Intermediate 64g):

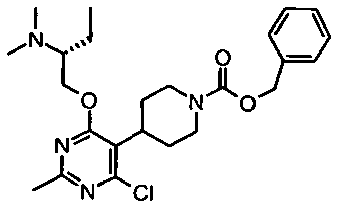

In a flame dried flask, sodium hydride (60% in mineral oil, 18.9 mg) was suspended in THF (5 ml) under an argon atmosphere. (R)-2-Dimethylamino-butan-1-ol (110 mg) was added dropwise and the mixture was stirred at room temperature for 1 h to form the corresponding sodium alcoxide. Dichloropyrimidine intermediate 64f) was dissolved in THF (3 ml) under argon atmosphere. The solution was cooled to 0°C and the above prepared sodium alcoholate was added dropwise within 30 min. The mixture was allowed to warm up to room temperature and was stirred at room temperature for 5 h. Additional 0.15 equivalents of the alcoxide described above were added at 0°C and stirring was continued at room temperature for 3 h. The reaction mixture was evaporated *in vacuo* to yield an amber, viscous oil that was dissolved in ethyl acetate. The solution was washed with 1 N NaHCO₃ and brine, dried over MgSO₄, filtered and evaporated. The crude product was purified by chromatography.

### Intermediate 64h):

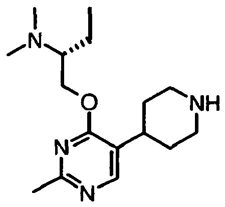

Intermediate 64g) (115 mg) was dissolved in methanol (5 ml). Calcium oxide (84 mg) was added, followed by 10% palladium on carbon (100 mg). The mixture was repeatedly degassed *in vacuum* and flushed with argon and finally with hydrogen. The reaction was stirred under a hydrogen balloon over night. The reaction was filtered through Celite and the filter was washed with 80 ml of methanol. The solvents were evaporated and the white, turbid residue was diluted with ethyl acetate and extracted with 1 N Na₂CO₃ and brine. The organic phase was dried over MgSO₄, filtered and evaporated to yield the desired product as a colorless oil.

### Example 64:

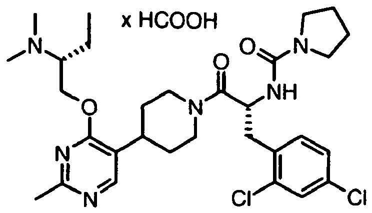

B-C moiety 1 (84.5 mg), HOBt (42.8 mg), EDCI (76 mg) and N-methylmorpholine (56.4 µl) in DMF (2.6 ml) were stirred at room temperature for one hour. Intermediate 64h) (68.0 mg) was added to the bright yellow solution and stirring was continued over night. The DMF was evaporated *in vacuum* and the remaining yellow oil was dissolved in ethyl acetate (100 ml). The organic solution was extracted with 1 N Na₂CO₃ (2 x 30 ml) and brine. Each aqueous phase was reextracted once with ethyl acetate. The combined organic layer was dried (MgSO₄), filtered and evaporated *in vacuo.* The product was purified by preparative HPLC.

### Synthesis of Example 73:

### Intermediate 73a):

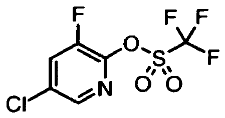

5-Chloro-3-fluoro-pyridin-2-ol (1.04 g) was dissolved in pyridine (13 ml) and cooled to 0°C under argon. Then trifluoromethanesulfonic anhydride (1.36 ml) was added dropwise via a syringe. After being stirred at room temperature overnight the reaction mixture was concentrated *in vacuo.* The remainder was partitioned between water and diethyl ether (100 ml each). The water layer was extracted with diethyl ether (2 x 50 ml) and the combined ether extract was washed with water and brine (50 ml each). Drying over Na₂SO₄ and evaporation of the solvent afforded a brown oil. Purification by column chromatography led to the desired compound in form of a yellowish liquid.

### Intermediate 73b):

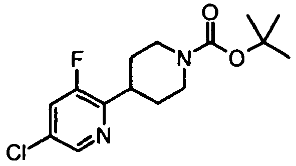

A flame dried Schlenk flask was charged with intermediate 73a) (956 mg), [1,1'-bis(diphenylphosphino)ferrocene]dichloropalladium(II) complex with dichloromethane (84 mg), copper(I) iodide (39 mg), and dry *N*,*N*-dimethylacetamide (5 ml). The resulting mixture was degassed with alternating vacuum/argon purges. Then 1-tert-butoxycarbonylpiperidin-4-yl)(iodo)zinc (4.89 mmol, prepared as described above) was added. The mixture was degassed once again and then heated to 80°C overnight. The main part of *N*,*N*-dimethylacetamide was then evaporated and the remainder was taken up in a mixture of EtOAc and water (each with 75 ml). This was then filtered through Celite and transferred into a separatory funnel. The phases were separated and the water layer was extracted with EtOAc (2 x 50 ml). The combined organic layer was washed with water and brine (each with 50 ml), dried (Na₂SO₄), filtered and concentrated *in vacuo.* The crude product was purified by column chromatography to furnish the desired compound in form of a brownish solid.

### Intermediate 73c):

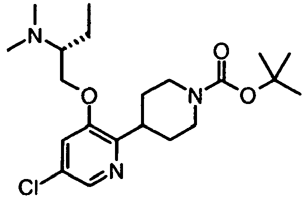

A solution of (R)-2-dimethylamino-butan-1-ol (252 mg) in DMF (1 ml) was added under argon at 0°C to a suspension of sodium hydride, 60 % dispersion in mineral oil (64 mg) in DMF (2 ml). The cooling bath was removed and the mixture stirred for 1 h at room temperature. Then a solution of intermediate 73b) (338 mg) in DMF (2 ml) was added and the reaction mixture was heated to 120°C overnight. The reaction mixture was cooled to 0°C and was then hydrolyzed with sat. NH₄Cl (1 ml). EtOAc (50 ml) was added and the mixture was washed with NaHCO₃ (2 x 25 ml). The combined aqueous layer was re-extracted with EtOAc (25 ml) and the combined organic layer then washed with brine (25 ml), dried (Na₂SO₄), filtered and concentrated *in vacuo.* The crude product was purified by chromatography to afford the desired product as yellowish oil.

### Intermediate 73d):

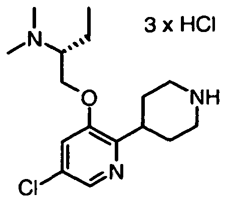

To Boc-protected intermediate 73c) (380 mg) in a mixture of DCM (1 ml) and MeOH (1 ml) was added hydrogen chloride, 4.0 M sol. in 1,4-dioxane (2 ml) and the solution was stirred at room temperature for 1 h. Evaporation of all volatiles and co-evaporation with toluene and acetone (each with 5 ml) led to a white solid, which was dried further in a desiccator (Sicapent) overnight.

### Example 73:

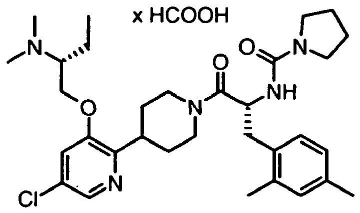

Intermediate 73d) (166 mg) and (R)-3-(2,4-dimethyl-phenyl)-2-[(pyrrolidine-1-carbonyl)-amino]-propionic acid (130 mg), 1-hydroxy-benzotriazole hydrate (79 mg) and N-methylmorpholine (143 µl) were dissolved in DMF (5 ml). After being stirred at room temperature for 30 min *N*-(3-dimethylaminopropyl)-*N*-ethylcarbodiimide hydrochloride (118 mg) was added and stirring was continued for another hour. An additional amount of *N*-methylmorpholine (30 µl) was added and stirring was continued overnight. The reaction mixture was diluted with EtOAc (50 ml) and washed with sat. Na₂CO₃ (3 x 25 ml) and water (25 ml). All aqueous layers were merged and re-extracted with EtOAc (2 x 25 ml). Then the combined organic layer was washed with brine (25 ml), dried (Na₂SO₄) and evaporated *in vacuo.* The residue was purified by preparative HPLC to provide the corresponding formate as yellowish resin.

### Synthesis of Example 85:

### Intermediate 85a):

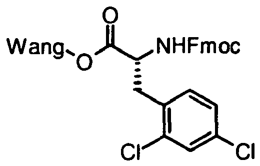

Wang resin (500 mg, Wang resin co. 100-200 mesh (Nova, 01-64-5027), 1.1 mmol/g) was treated with a solution of Fmoc-D-2,4-dichlorophenylalanine (753 mg) activated with DIC (255 µl) in the presence of DMAP (5 mg) in DMF (5 ml). The mixture was reacted overnight. Excess of the reagents was removed by filtration. The resin was washed with DMF (3 x 4 ml). DMF (3 ml) was added followed by acetic anhydride (260 µl). The mixture was reacted for 1 h. Excess of the reagent was removed by filtration. The resin-bound intermediate was successively washed with DMF (3 x 4 ml), MeOH (3 x 4 ml), THF (3 x 4 ml), DCM (3 x 4 ml) and diethyl ether (3 x 4 ml). The resin was dried under reduced pressure.

### Intermediate 85b):

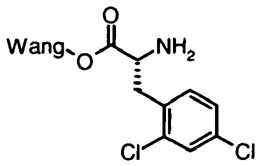

Intermediate 85a) (200 mg) was treated with a solution of 20% piperidine in DCM (4 ml). The mixture was reacted for 30 min. Excess of the reagents was removed by filtration. The resin-bound intermediate was successively washed with DMF (3 x 2 ml), MeOH (3 x 2 ml), THF (3 x 2 ml), DCM (3 x 2 ml) and diethyl ether (3 x 2 ml). The resin was dried under reduced pressure.

### Intermediate 85c):

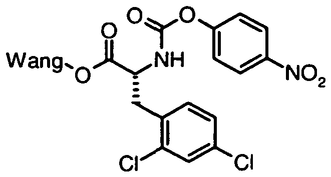

Intermediate 85b) (200 mg) was treated with a cold solution of 4-nitrophenyl chloroformate (222 mg) and triethylamine (216 µl) in DCM (2.5 ml). The mixture was reacted at room temperature for 2 h. Excess of the reagents was removed by filtration. The resin-bound intermediate was successively washed with DCM (3 x 2 ml), THF (3 x 2 ml), DCM (3 x 2 ml) and diethyl ether (3 x 2 ml). The resin was dried under reduced pressure.

### Intermediate 85d):

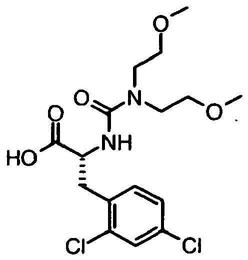

Intermediate 85c) (200 mg) was treated with a solution of bis(2-methoxyethyl)-amine (322 µl) in DCM (2 ml). The mixture was reacted at room temperature for 2 h. Excess of the reagent was removed by filtration. The resin-bound intermediate was successively washed with DMF (3 x 3 ml), MeOH, (3 x 3 ml), THF (3 x 3 ml), DCM (3 x 3 ml) and diethyl ether (3 x 3 ml). The resin was dried under reduced pressure. 20% TFA in DCM (3 ml) was added to the resin and the mixture was reacted for 30 min. The product was filtered off and the solvent removed.

### Example 85:

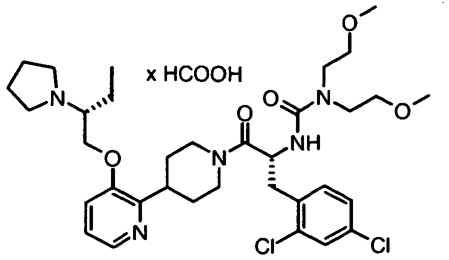

To intermediate 85d) (95 mg) in DMF (2 ml) was added intermediate 3-((R)-2-pyrrolidin-1-yl-butoxy)-1',2',3',4',5',6'-hexahydro-[2,4']bipyridinyl trihydrochloride (91 mg), N-methylmorpholine (109 µl), and HOBt (37 mg) and the mixture was stirred for 20 min. EDCI (46 mg) was added and stirring was continued overnight. The reaction mixture was poured into brine (20 ml), diluted with ethyl acetate and the organic phase was separated. The aqueous phase was extracted two times with ethyl acetate. The combined organic phases were washed twice with saturated sodium bicarbonate solution, twice with water and brine, dried over Na₂SO₄ and concentrated. The crude product was purified with preparative LC-MS and subsequently lyophilized from 80% tBuOH in water to yield a pale yellow solid.

### Synthesis of Example 88:

### Intermediate 88a):

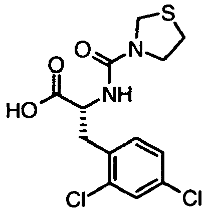

Intermediate 85c) (200 mg) was treated with a solution of thiazolidine (172 µl) in DCM (2 ml). The mixture was reacted at room temperature for 3 h. DBU (164 µl) was added and the mixture was reacted at room temperature for 1 h. Excess of the reagents was removed by filtration. The resin-bound intermediate was successively washed with DMF (3 x 3 ml), MeOH, (3 x 3 ml), THF (3 x 3 ml), DCM (3 x 3 ml) and diethyl ether (3 x 3 ml). The resin was dried under reduced pressure. 20% TFA in DCM (3 ml) was added to the resin and the mixture was reacted for 30 min. The product was filtered off and the solvent removed.

### Example 88:

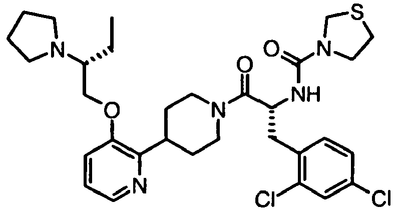

To intermediate 88a) (60 mg) in DMF (2 ml) was added 3-((R)-2-pyrrolidin-1-yl-butoxy)-1',2',3',4',5',6'-hexahydro-[2,4']bipyridinyl trihydrochloride (66 mg), N-methylmorpholine (79 µl), and HOBt (28 mg) and the mixture was stirred for 20 min. EDCI (35 mg) was added and stirring was continued overnight. The reaction mixture was poured into brine (20 ml), diluted with ethyl acetate and the organic phase was separated. The aqueous phase was extracted two times with ethyl acetate. The combined organic phases were washed twice with saturated sodium bicarbonate solution, twice with water and brine, dried over Na₂SO₄ and concentrated. The crude product was purified with preparative LC-MS and subsequently lyophilized from 80% tBuOH in water to yield a white solid.

### Synthesis of Example 99:

### Intermediate 99a):

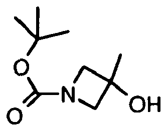

In a flame dried flask 3-oxo-azetidine-1-carboxylic acid *tert*-butyl ester (500 mg) was dissolved in THF (6 ml) under argon and the solution was cooled to 0°C. Methylmagnesium bromide (3 M solution in diethylether, 1.95 ml) was added dropwise and the milky suspension was stirred for 3 h. The reaction was carefully hydrolyzed with saturated aqueous NH₄Cl and extracted with EtOAc (80 ml). The organic phase was washed with brine, dried over Na₂SO₄, filtered and evaporated to yield the product as a colorless solid.

### Intermediate 99b):

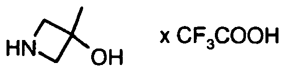

Trifluoroacetic acid (4 ml) was added dropwise to a solution of the intermediate 99a) in DCM (40 ml) at room temperature. The mixture was stirred for 90 min. Half of the solvent was evaporated, then toluene was added and evaporation was continued. This co-evaporation procedure was repeated twice before evaporating all the solvent. The remaining product was dried under high vacuum overnight.

### Intermediate 99c):

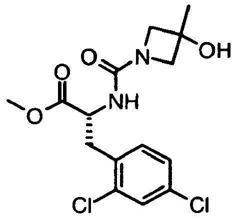

To an ice cold solution of intermediate 99b) (4.451 g) and triethylamine (2.34 ml) in DMF (30 ml) was added a solution of (R)-3-(2,4-dichloro-phenyl)-2-isocyanato-propionic acid methyl ester (3.048 g) in anhydrous DMF (50 ml) under Ar. The mixture was left stirring at 0°C for 4 h. The reaction mixture was evaporated *in vacuo.* The residue was diluted in EtOAc (300 ml), the organic phase was washed with 0.1 N HCl (2 x 90 ml), 0.1 N NaHCO₃ and brine. The aqueous phase was re-extracted with EtOAc, the combined organic phases were dried under Na₂SO₄, filtered and evaporated. The crude compound was purified with column chromatography to obtain a colorless, stable foam.

### Intermediate 99d):

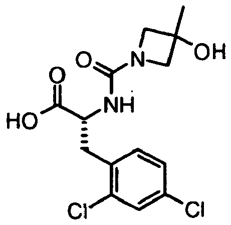

Intermediate 99c) was dissolved in a mixture of MeOH (2 ml) and THF (6 ml) at 0°C. A solution of lithium hydroxide monohydrate (73 mg) in H₂O (2 ml) was added. The mixture was stirred at 0°C for 3 h. The reaction mixture was neutralized by addition of 1 N HCl and the MeOH and THF were evaporated *in vacuo.* The aqueous phase was acidified with 1 N HCl (pH - 1-2). The aqueous phase was extracted with EtOAc (30 ml). The organic layer was washed with water and brine. The aqueous phases were re-extracted with EtOAc, the combined organic layer was dried over Na₂SO₄, filtered and evaporated. The crude product was dried under high vacuum to yield a white foam.

### Example 99:

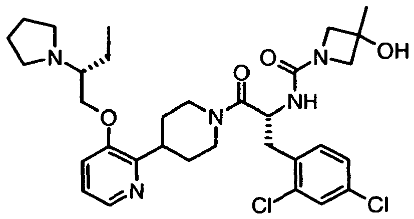

Intermediate 99d) (2.523 g), HOBt (1.113 g), EDCI (1.974 g) and NMM (1.47 ml) in DMF (50 ml) were stirred for one hour at room temperature. 3-((R)-2-Pyrrolidin-1-yl-butoxy)-1',2',3',4',5',6'-hexahydro-[2,4']bipyridinyl trihydrochloride (2.050 g) and a second portion of NMM (1.33 ml) were added and stirring was continued over night. The DMF was evaporated *in vacuo* and the remaining orange oil was dissolved in ethyl acetate (300 ml). The organic solution was washed with 1 N Na₂CO₃ (2 x 150 ml) and brine. Each aqueous phase was re-extracted once with ethyl acetate. The organic layer was dried (Na₂SO₄), filtered and evaporated *in vacuo.* The crude product was purified by preparative HPLC.

### Synthesis of Example 125:

### Intermediate 125a):

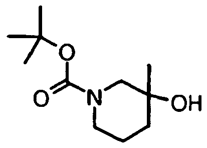

Mercury acetate (1.625 g) was dissolved in water (6 ml). A solution of 3-methylene-N-Boc-piperidine (0.986 g) in THF (6 ml) was added dropwise at room temperature. After 20 min the mercury acetate had dissolved and the initially yellow solution got clear and colorless. The mixture was stirred another 40 min at room temperature. The reaction was cooled in ice water and 3 N sodium hydroxide solution (5 ml) was added. A brown color appeared. A solution of sodium borohydride (0.19 g) in 3 N NaOH (5 ml) was then added and the mixture was stirred for 15 min. Acidic acid was added until pH 6 was reached and hydrogen evolution had ceased. The mixture was decanted from the precipitated mercury and evaporated. The residue was partitioned between water and ethyl acetate. The organic phase was washed with water and brine, dried over MgSO₄, filtered and evaporated. A pale yellow oil remained that was purified by column chromatography. The product was obtained in form of a clear, colorless, viscous oil that was dried in high vacuum.

### Intermediate 125b):

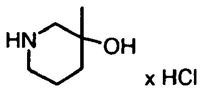

Intermediate 125a) (846 mg) was dissolved in dioxane (30 ml). 4 N HCl in dioxane (30 ml) was added and the reaction was stirred at room temperature for 3.5 hours. The solvents were evaporated and the residue was co-evaporated several times with toluene. The remaining pale yellow solid was washed and dried under high vacuum overnight.

### Intermediate 125c):

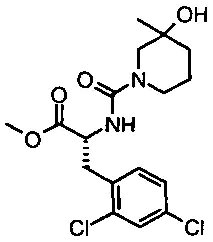

To an ice cold solution of (R)-3-(2,4-dichloro-phenyl)-2-isocyanato-propionic acid methyl ester (271 mg) in DMF (2.5 ml) was added a solution of intermediate 125b) (300 mg) and Et₃N (279 µl) in DMF (3 ml). The mixture was left stirring at 0°C for 4 h. The reaction mixture was evaporated *in vacuo.* The residue was diluted with EtOAc (50 ml), the organic phase was washed with 0.1 N HCl (2 x 20 ml) and brine. The aqueous phase was re-extracted with EtOAc and the combined organic phase was dried over Na₂SO₄, filtered and evaporated.

### Intermediate 125d):

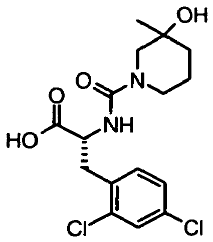

The intermediate 125c) (404 mg) was dissolved in MeOH (1.5 ml) and THF (5.0 ml) at 0°C. A solution of lithium hydroxide monohydrate (83 mg) in H₂O (1.5 ml) was added. The mixture was stirred at 0°C for 1.5 h. The reaction mixture was neutralized by addition of 1 N HCl and the MeOH and THF were evaporated *in vacuo.* The aqueous phase was acidified with 1 N HCl (pH - 1-2). The aqueous phase was extracted with ethyl acetate (50 ml). The organic layer was washed with water and brine. The aqueous phases were re-extracted with EtOAc, the combined organic layer was dried over Na₂SO₄ and evaporated *in vacuo* to yield a colorless foam.

### Example 125:

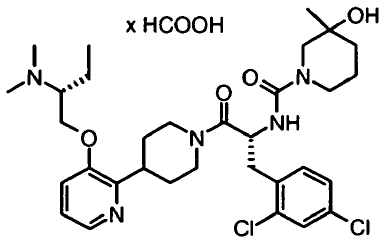

Intermediate 125d) (190 mg), HOBt (64.7 mg), EDCI (138 mg) and NMM (102 µl) in DMF (3 ml) were stirred at room temperature for one hour. [(R)-1-(1',2',3',4',5',6'-Hexahydro-[2,4']bipyridinyl-3-yloxymethyl)-propyl]-dimethyl-amine trihydrochloride (163 mg) and a second portion of NMM (93 µl) were added and stirring was continued over night. The DMF was evaporated *in vacuum* and the remaining yellow oil was dissolved in ethyl acetate (100 ml). The organic solution was washed with 1 N Na₂CO₃ (2 x 30 ml) and brine. Each aqueous phase was re-extracted once with ethyl acetate. The organic layer was dried (Na₂SO₄), filtered and evaporated *in vacuo.* The residue was purified by preparative HPLC.

### Synthesis of Example 127:

### Intermediate 127a):

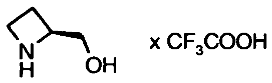

To Boc-(S)-1-azetidin-2-yl-methanol (J.Med.Chem. 2005, 48, 7637-7647) (1.47 g) in DCM (20 ml) was added TFA (10 ml) at 0°C and the solution was stirred at said temperature for 1.5 h. Evaporation of all volatiles and coevaporation with toluene (2 x 20 ml) led to the desired compound as cloudy yellow oil.

### Intermediate 127b):

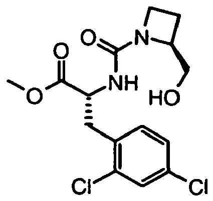

To an ice cold solution of (R)-3-(2,4-dichloro-phenyl)-2-isocyanato-propionic acid methyl ester (synthesis described above) (587 mg) in abs. DCM (6 ml) was added a solution of intermediate 127a) (1.51 g) and triethylamine (0.90 ml) in abs. DMF (4 ml) under argon. The mixture was left stirring at 0°C for 5 h, before all volatiles were evaporated. The remainder was diluted with EtOAc (100 ml) and the organic phase was washed with 0.1 M HCl (2 x 50 ml) and water (50 ml). All aqueous phases were merged and re-extracted with EtOAc (2 x 50 ml). The combined organic layer was washed with brine (50 ml), dried (Na₂SO₄), filtered and concentrated *in vacuo.* The crude product was purified by chromatography to afford the desired ester as white solid.

### Intermediate 127c):

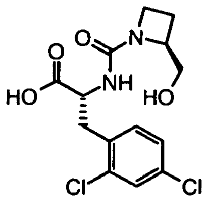

A solution of lithium hydroxide (36 mg) in water (0.9 ml) was added under argon at 0°C to a solution of intermediate 127b) (274 mg) in MeOH (2.0 ml) and THF (4.3 ml). The reaction mixture was stirred at 0°C for 2 h. The mixture was neutralized by addition of 1 M HCl and the main part of MeOH and THF were removed *in vacuo.* Then the remainder was acidified with 1 M HCl to pH 3-4 and the mixture was extracted with EtOAc (2 x 25 ml). The organic layer was washed with water and brine (each with 25 ml). The aqueous phases were re-extracted with EtOAc (25 ml), the combined organic layer was dried (Na₂SO₄) and evaporated to afford the corresponding acid as colorless foam.

### Example 127:

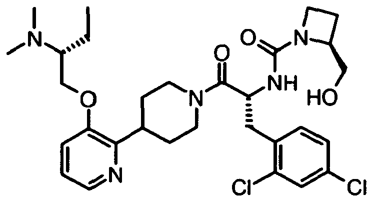

((R)-1-(1',2',3',4',5',6'-Hexahydro-[2,4']bipyridinyl-3-yloxymethyl)-propyl]-dimethyl-amine trihydrochloride (125 mg) and intermediate 127c) (123 mg), 1-hydroxy-benzotriazole hydrate (62 mg) and *N*-methylmorpholine (114 µl) were dissolved in DMF (5 ml). After being stirred at room temperature for 30 min *N*-(3-dimethylaminopropyl)-*N*-ethylcarbodiimide hydrochloride (94 mg) was added and stirring was continued for another hour. An additional amount of *N*-methylmorpholine (24 µl) was added and stirring was continued overnight. The reaction mixture was diluted with EtOAc (50 ml) and washed with sat. NaHCO₃ (3 x 25 ml). All aqueous layers were merged and re-extracted with EtOAc (25 ml). Then the combined organic layer was washed with brine (25 ml), dried (Na₂SO₄) and evaporated *in vacuo.* The residue was purified by preparative HPLC to provide the desired amine as yellowish resin.

### Synthesis of Example 129:

### Intermediate 129a):

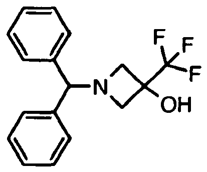

Trifluoromethyltrimethylsilane (0.93 ml) and cesium fluoride (0.96 g) were added to a solution of benzhydryl-azetidine-3-one (1.00 g) in THF (12.5 ml). After being stirred at room temperature for 1 h, sat. NH₄Cl (12.5 ml) and tetrabutylammonium fluoride (498 mg) were added and stirring was continued for another 6 h. The mixture was extracted with diethyl ether (3 x 50 ml) and the organic layer was dried over MgSO₄. Concentration *in vacuo* provided an orange oil, which was purified by chromatography to afford the desired alcohol in form of a yellow oil.

### Intermediate 129b):

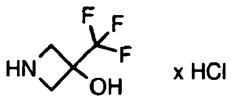

Palladium hydroxide, 20 wt% on carbon (202 mg) and 1 M HCl (1.52 ml) were added to a solution of intermediate 129 a) (466 mg) in MeOH. The reaction mixture was hydrogenated under 1 bar of hydrogen at room temperature for 3 h. The catalyst was filtered through Celite and washed with methanol. The filtrate was concentrated and dried under vacuum. The residue was then washed with hexanes. The organic layer was decanted and a beige solid was obtained.

### Intermediate 129c):

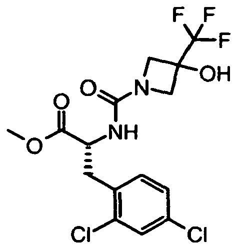

To an ice cold solution of (R)-3-(2,4-dichloro-phenyl)-2-isocyanato-propionic acid methyl ester (synthesis described above) (178 mg) in abs. DCM (3 ml) was added a solution of intermediate 129b) (230 mg) and triethylamine (0.27 ml) in abs. DMF (2 ml) under argon. The mixture was left stirring at 0°C for 3 h. Then the mixture was diluted with EtOAc (50 ml) and the organic phase was washed with 0.1 M HCl (2 x 25 ml) and water (25 ml). All aqueous phases were merged and re-extracted with EtOAc (2 x 50 ml). Then the combined organic layer was washed with brine (50 ml), dried (Na₂SO₄), filtered and concentrated *in vacuo.* The crude product was purified by chromatography to afford the desired ester as colorless foam.

### Intermediate 129d):

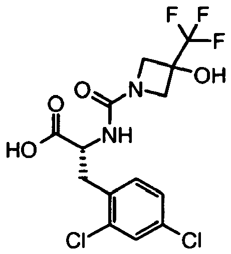

A solution of lithium hydroxide (17 mg) in water (0.5 ml) was added under argon at 0°C to a solution of intermediate 129c) (151 mg) in MeOH (0.9 ml) and THF (2.0 ml). The reaction mixture was stirred at 0°C for 3 h. The mixture was neutralized by addition of 1 M HCl and the main part of MeOH and THF were removed *in vacuo.* Then the remainder was acidified with 1 M HCl to pH 3-4 and the mixture was extracted with EtOAc (2 x 25 ml). The organic layer was washed with water and brine (each with 25 ml), dried (Na₂SO₄) and evaporated to afford the corresponding acid as colorless resin.

### Example 129:

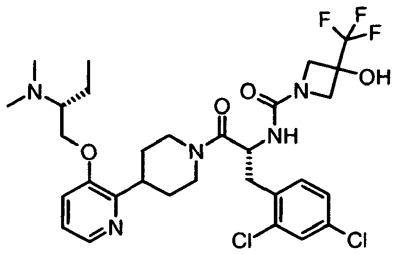

[(R)-1-(1',2',3',4',5',6'-Hexahydro-[2,4']bipyridinyl-3-yloxymethyl)-propyl]-dimethyl-amine trihydrochloride (111 mg) and intermediate 129d) (125 mg), 1-hydroxy-benzotriazole hydrate (55 mg) and *N*-methylmorpholine (100 µl) were dissolved in DMF (5 ml). After being stirred at room temperature for 30 min *N*-(3-dimethylaminopropyl)-*N*-ethylcarbodiimide hydrochloride (82 mg) was added and stirring was continued for another hour. An additional amount of N-methylmorpholine (100 µl) was added and stirring was continued overnight. The reaction mixture was diluted with EtOAc (50 ml) and washed with sat. NaHCO₃ (3 x 25 ml). All aqueous layers were merged and re-extracted with EtOAc (25 ml).

Then the combined organic layer was washed with brine (25 ml), dried (Na₂SO₄) and evaporated *in vacuo.* The residue was purified by preparative HPLC to provide the desired amine as yellowish resin.

### Synthesis of Example 130:

### Intermediate 130a):

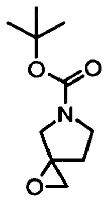

In a flame dried flask trimethylsulfoxonium iodide (2.57 g) and sodium hydride (60% in mineral oil, 476 mg) were suspended in DMSO (8.8 ml) at 10°C. The mixture was stirred at room temperature for 1.5 hours. A solution of tert-butyl-3-oxopyrrolidine-1-carboxylate (2.00 g) in DMSO (2.2 ml) was added dropwise within 15 min. Stirring was continued for 1 hour. The reaction was hydrolyzed by addition of ice water (50 ml) and brine (50 ml). The aqueous suspension was extracted with diethyl ether (2x 50 ml). The combined etheral solution was washed with brine, dried over MgSO₄ and filtered. The filtrate was evaporated *in vacuo* to yield a viscous brown oil that was purified by Kugelrohr distillation. The product was obtained as colorless oil.

### Intermediate 130b):

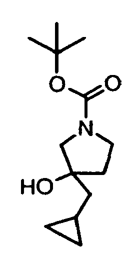

In a flame dried flask, copper(I)cyanide (105 mg) was suspended in anhydrous THF (2 ml). The suspension was cooled to -76°C and cyclopropylmagnesium bromide (0.5 M in THF, 9.34 ml) was added. The mixture was stirred for 1 hour, followed by slow addition of a solution of intermediate 130a) (155 mg) in dry THF (1 ml). The reaction was slowly warmed to room temperature and stirred over night. The mixture was partitioned between saturated aqueous NH₄Cl and diethyl ether. The ether phase was washed with water and brine. Each aqueous phase was re-extracted once with diethyl ether. The combined organic phase was dried over MgSO₄, filtered and evaporated. The crude product was purified by column chromatography.

### Intermediate 130c):

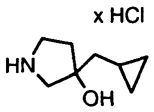

Intermediate 130b) (116 mg) was dissolved in dioxane (6 ml). 4 N HCl in dioxane (6 ml) was added and the reaction was stirred at room temperature for 1.5 hours. The solvents were evaporated and the residue was coevaporated several times with toluene. The remaining pale red solid was dried under high vacuum.

### Intermediate 130d):

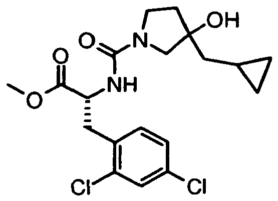

To an ice cold solution of (R)-3-(2,4-dichloro-phenyl)-2-isocyanato-propionic acid methyl ester (125 mg) in DMF (4 ml) was added a solution of intermediate 130c) (89 mg) and triethylamine (134 µl) in anhydrous DMF (2 ml) under argon. The mixture was left stirring at 0°C for 2 hours and an additional 2 hours at room temperature. The reaction mixture was evaporated *in vacuo.* The residue was diluted with DCM (80 ml), the organic phase was washed with 0.1 N HCl (20 ml), 1 N NaHCO₃ (20 ml) and brine. Each aqueous phase was re-extracted with DCM, then the combined organic phase was dried over Na₂SO₄, filtered and evaporated. A pale brown oil was obtained and dried under high vacuum. The crude product was purified by column chromatography.

### Intermediate 130e):

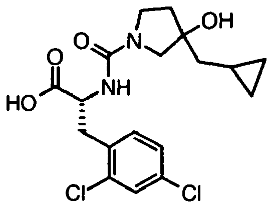

Intermediate 130d) (171 mg) was dissolved in MeOH (1 ml) and THF (5 ml) at 0°C. A solution of lithium hydroxide monohydrate (34.6 mg) in H₂O (1 ml) was added. The mixture was stirred at 0°C for 4 h. The reaction mixture was neutralized by addition of 1 N HCl and the MeOH and THF were evaporated *in vacuo.* The aqueous phase was acidified with 1 N HCl (pH - 1-2) and was extracted with EtOAc (1 x 20 ml). The organic layer was washed with water and brine. The aqueous phases were re-extracted with EtOAc, the combined organic layer was dried over Na₂SO₄ and evaporated *in vacuo* to yield a white foam. The crude product was purified by column chromatography.

### Example 130:

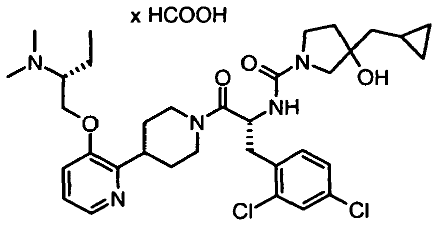

[(R)-1-(1',2',3',4',5',6'-Hexahydro-[2,4']bipyridinyl-3-yloxymethyl)-propyl]-dimethyl-amine trihydrochloride (123 mg), the intermediate 130e) (165 mg) and HOBt (73 mg) were mixed in DCM (6 ml). EDCI (73 mg) and NMM (132 µl) were added. The reaction was stirred at room temperature for one hour. A second portion of NMM (28 µl) was added and stirring was continued over night. The mixture was diluted with EtOAc (50 ml) and washed with 1 N Na₂CO₃ (3x 25 ml) and brine. Every aqueous phase was re-extracted once with EtOAc. The combined organic layer was dried (Na₂SO₄), filtered and evaporated *in vacuo.* The crude product was purified by preparative HPLC.

### Synthesis of Example 133:

### Intermediate 133a):

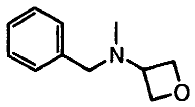

Sodium triacetoxyborohydride (5.88 g) was added to a solution of 3-oxetanone (1.00 g) and N-benzyl-methylamine (2.52 g, 2.7 ml) in 1,2-dichloroethane (300 ml), shortly followed by addition of acetic acid (1.6 ml). After being vigorously stirred for 18 h at room temperature, the resulting fine suspension was cooled down to 0°C and the reaction was quenched by careful addition of 200 ml of water. The system was left stirring for 1 h to reach a complete hydrolysis of the reagent left. The organic phase was then collected and the aqueous phase was further extracted with DCM (2 x 50 ml). The merged organic phase was then washed with sat. NaHCO₃ (2 x 50 ml), H₂O (50 ml) and brine (75 ml). After drying over Na₂SO₄, filtration and evaporation of the solvent, the compound was obtained as a crude orange syrup. Purification by chromatography afforded the desired compound in form of a yellowish oil.

### Intermediate 133b):

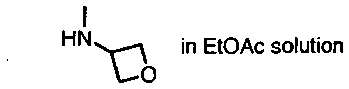

Intermediate 133a) (1.94 g) in solution in EtOAc (25 ml) was hydrogenated overnight in the presence of palladium hydroxide, 20 wt% on carbon (195 mg) under 1 bar of hydrogen at room temperature. Filtration through a syringe filter of the supernatant and further washing of the catalyst with EtOAc (5 ml) yielded a neat solution of the desired compound in 30 ml of EtOAc.

### Intermediate 133c):

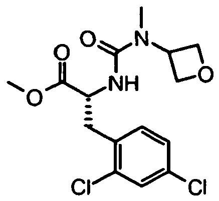

To an ice cold solution of (R)-3-(2,4-dichloro-phenyl)-2-isocyanato-propionic acid methyl ester (synthesis as described above) (450 mg) in abs. DCM (10 ml) was added a solution of intermediate 133b) in EtOAc (6 ml; 2.62 mmol). The mixture was left stirring at 0°C for 1 h, then left returning to room temperature for 17 h. At this time all volatiles were evaporated and the resulting colorless wax was extensively dried *in vacuo* to remove the excess of intermediate 133b) and yield the desired ester in a pure form.

### Intermediate 133d):

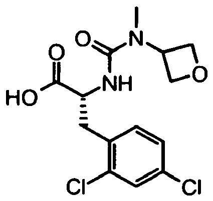

A solution of lithium hydroxide (80 mg) in water (1.5 ml) was added under argon at 0°C to a solution of intermediate 133c) (698 mg) in MeOH (9.0 ml) and THF (1.5 ml). The reaction mixture was stirred at 0°C for 2 h. The mixture was neutralized by addition of trifluoroacetic acid (380 mg; 260 µl) and the main part of MeOH and THF were removed *in vacuo.* Then the remainder was diluted with water (10 ml) and extracted with EtOAc (3 x 25 ml). The organic layer was washed with brine (25 ml), dried (Na₂SO₄) and evaporated to afford the corresponding acid as colorless foam after an extensive drying under *high vacuo.*

### Example 133:

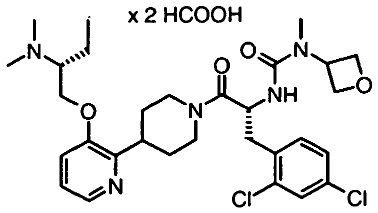

[(R)-1-(1',2',3',4',5',6'-Hexahydro-[2,4']bipyridinyl-3-yloxymethyl)-propyl]-dimethyl-amine trihydrochloride (120 mg) and intermediate 133d) (153 mg), 1-hydroxy-benzotriazole hydrate (61 mg) and *N*-methylmorpholine (119 µl) were dissolved in DCM (15 ml). After being stirred at room temperature for 30 min *N*-(3-dimethylaminopropyl)-*N*-ethylcarbodiimide hydrochloride (88 mg) was added and stirring was continued for another hour. An additional amount of *N*-methylmorpholine (26 µl) was added and stirring was continued overnight. The volatiles were evaporated and the residue was partitioned between EtOAc (50 ml) and sat. NaHCO₃ (25 ml). The organic phase was collected and further washed with sat. NaHCO₃ (2 x 25 ml). The aqueous layers were merged and extracted back with DCM (10 ml). Then the combined organic layer was washed with brine (25 ml), dried (Na₂SO₄) and evaporated *in vacuo.* The residue was purified by preparative HPLC to provide the bis-formiate salt of the desired derivative as yellowish resin.

### Synthesis of Example 134:

### Intermediate 134a):

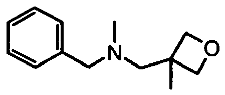

N-benzyl-methylamine (1.05 g, 1.12 ml) was added to a solution of 3-hydroxymethyl-3-methyloxetane p-tosylate (2.23 g) in acetonitrile (35 ml), in presence of solid anhydrous Na₂CO₃ (2.00 g). The suspension was then vigorously stirred at room temperature for 6 days, at which time no more progress in the conversion was noticeable. All the volatiles were evaporated and the residue was partitioned between EtOAc (50 ml) and water (50 ml). The organic phase was collected and further washed with water and brine (25 ml each). The merged aqueous phase was extracted back with DCM (25 ml). The merged organic phase was then dried over Na₂SO₄, filtered and evaporated to yield a partially crystallized crude syrup. Purification by chromatography afforded the desired compound in form of a yellowish oil.

### Intermediate 134b):

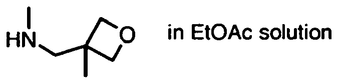

Intermediate 134a) (360 mg) in solution in EtOAc (10 ml) was hydrogenated overnight in the presence of palladium hydroxide, 20 wt% on carbon (60 mg) under 1 bar of hydrogen at room temperature. Filtration through a syringe filter and further washing of the catalyst with EtOAc (5 ml) yielded a neat solution of the desired compound in 15 ml of EtOAc.

### Intermediate 134c):

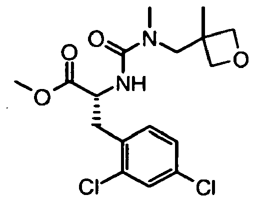

To an ice cold solution of (R)-3-(2,4-dichloro-phenyl)-2-isocyanato-propionic acid methyl ester (synthesis as described above) (300 mg) in abs. DCM (9 ml) was added a solution of intermediate 134b) in EtOAc (15 ml; 1.75 mmol). The mixture was left stirring at 0°C for 1 h, then left returning to room temperature for 17 h. At this time all volatiles were evaporated to yield a pale yellow syrup/wax. The crude product was purified by chromatography to afford the desired ester as a clear syrup crystallizing upon storage at room temperature.

### Intermediate 134d):

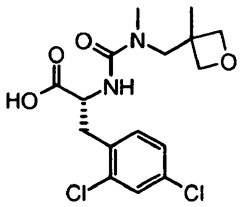

A solution of lithium hydroxide (42 mg) in water (0.8 ml) was added under argon at 0°C to a solution of intermediate 134c) (305 mg) in MeOH (5.0 ml) and THF (0.8 ml). The reaction mixture was stirred at 0°C for 2 h. The mixture was neutralized by addition of trifluoroacetic acid (200 mg; 135 µl) and the main part of MeOH and THF were removed *in vacuo.* Then the remainder was diluted with water (10 ml) and extracted with EtOAc (3 x 25 ml). The organic layer was washed with brine (25 ml), dried (Na₂SO₄) and evaporated to afford the corresponding acid as a colorless viscous syrup forming an unstable foam under *high vacuo.*

### Example 134:

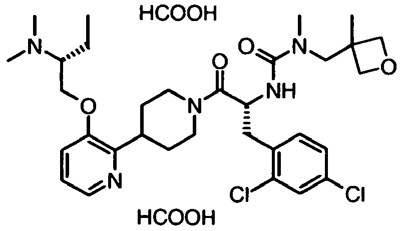

[(R)-1-(1',2',3',4',5',6'-Hexahydro-[2,4']bipyridinyl-3-yloxymethyl)-propyl]-dimethyl-amine trihydrochloride (100 mg) and intermediate 134d) (115 mg), 1-hydroxy-benzotriazole hydrate (52 mg) and N-methylmorpholine (100 µl) were dissolved in DCM (15 ml). After being stirred at room temperature for 30 min *N*-(3-dimethylaminopropyl)-*N*'-ethylcarbodiimide hydrochloride (75 mg) was added and stirring was continued for another hour. An additional amount of *N*-methylmorpholine (22 µl) was added and stirring was continued overnight. The volatiles were evaporated and the residue was partitioned between EtOAc (50 ml) and sat. NaHCO₃ (25 ml). The organic phase was collected and further washed with sat. NaHCO₃ (2 x 25 ml). The aqueous layers were merged and extracted back with DCM (10 ml). Then the combined organic layer was washed with brine (25 ml), dried (Na₂SO₄) and evaporated *in vacuo.* The residue was purified by preparative HPLC to provide the bis-formiate salt of the desired derivative as yellowish resin.

Further examples are exemplified below.

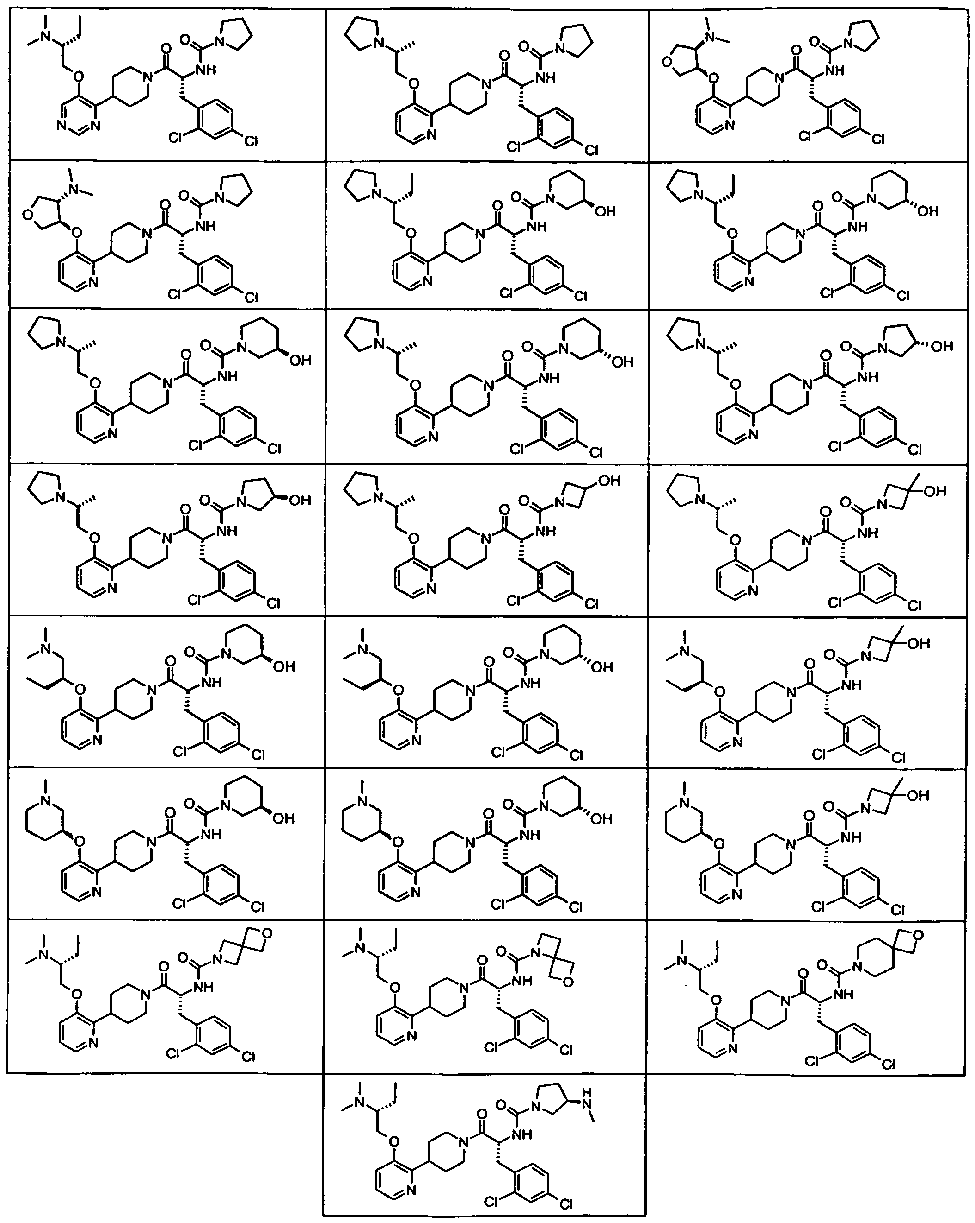

### Biological Assays

### A. Binding Assay

A membrane binding assay is used to identify competitive inhibitors of fluorescence labeled NDP-alpha-MSH binding to HEK293 cell membrane preparations expressing human melanocortin receptors.

The test compound or unlabeled NDP-alpha-MSH is dispensed at varying concentrations to a 384 well microtiter plate. Fluorescence labeled NDP-alpha-MSH is dispensed at a single concentration, followed by addition of membrane preparations. The plate is incubated for 5 h at room temperature.

The degree of fluorescence polarization is determined with a fluorescence polarization microplate reader.

### B. Functional Assay

Agonistic activity of human melanocortin receptors is determined in a homogeneous membrane based assay. Competition between unlabeled cAMP and a fixed quantity of fluorescence labeled cAMP for a limited number of binding sites on a cAMP specific antibody is revealed by fluorescence polarization.

The test compound or unlabeled NDP-alpha-MSH is dispensed at varying concentrations to a 384 well microtiter plate. Membrane preparations from HEK293 cells expressing the human melanocortin receptors are added. After a short preincubation period, an appropriate amount of ATP, GTP and the cAMP antibody is added and the plate is further incubated before the fluorescence labeled cAMP conjugate is dispensed. The plate is incubated for 2 h at 4°C before it is read on a fluorescence polarization microplate reader. The amount of cAMP produced as a response to a test compound is compared to the production of cAMP resulting from stimulation with NDP-alpha-MSH.

Representative compounds of the present invention were tested and found to bind to the melanocortin-4 receptor. These compounds were generally found to have IC₅₀ values less than 2 µM.

### Table 16: Biological data for selected examples of the invention

In the table are listed the IC₅₀ values of the hMC-4R binding assay and the EC₅₀ values of the functional assay. The IC₅₀ and EC₅₀ values were grouped in 3 classes: a ≤ 0.1 µM; b > 0.1 µM and ≤ 1.0 µM; c > 1.0 µM

| Example | hMC-4R binding assay IC₅₀/µM | hMC-4R functional assay EC₅₀/µM | % activation functional assay |
|---|---|---|---|
| SHU-9119 | a | - | 7 |
| NDP-α-MSH | a | a | 100 |
| 1 | a | - | 0 |
| 2 | a | - | 0 |
| 3 | b | - | 0 |
| 4 | a | - | 0 |
| 5 | a | - | 0 |
| 6 | a | - | 0 |
| 7 | a | - | 0 |
| 8 | a | - | 0 |
| 9 | a | - | 0 |
| 10 | b | - | 0 |
| 11 | a | - | 0 |
| 12 | a | - | 0 |
| 13 | b | - | 0 |
| 14 | b | - | 0 |
| 15 | a | - | 0 |
| 16 | a | - | 0 |
| 17 | a | - | 0 |
| 18 | a | - | 0 |
| 19 | a | - | 0 |
| 20 | a | - | 0 |
| 21 | a | - | 0 |
| 22 | a | - | 0 |
| 23 | b | - | 0 |
| 24 | a | - | 0 |
| 25 | a | - | 0 |
| 26 | b | - | 0 |
| 27 | a | - | 0 |
| 28 | b | - | 0 |
| 29 | b | - | 0 |
| 30 | a | - | -8 |
| 31 | a | - | -3 |
| 32 | a | c | 36 |
| 33 | b | - | 0 |
| 34 | c | - | -3 |
| 35 | b | - | 7 |
| 36 | a | - | 8 |
| 37 | b | - | -2 |
| 38 | b | - | -5 |
| 39 | a | - | -13 |
| 40 | a | - | -11 |
| 41 | b | - | -1 |
| 42 | b | - | -1 |
| 43 | b | - | 0 |
| 44 | b | - | -4 |
| 45 | a | b | -27 |
| 46 | c | - | -13 |
| 47 | a | b | -36 |
| 48 | a | - | 0 |
| 49 | b | - | -3 |
| 50 | a | - | 0 |
| 51 | b | - | 0 |
| 52 | b | - | 4 |
| 53 | c | - | 1 |
| 54 | b | - | 10 |
| 55 | b | - | 2 |
| 56 | a | - | -5 |
| 57 | a | - | 5 |
| 58 | b | - | 4 |
| 59 | a | - | -1 |
| 60 | a | - | -12 |
| 61 | a | - | 1 |
| 62 | a | - | -10 |
| 63 | a | - | 0 |
| 64 | a | - | 13 |
| 65 | a | - | 0 |
| 66 | b | - | 0 |
| 67 | c | c | 33 |
| 68 | a | - | -5 |
| 69 | a | - | -7 |
| 70 | a | - | -2 |
| 71 | a | - | -4 |
| 72 | a | - | 0 |
| 73 | a | - | -15 |
| 74 | a | - | -5 |
| 75 | a | - | -1 |
| 76 | a | - | 0 |
| 77 | a | - | 0 |
| 78 | a | - | 0 |
| 79 | a | - | 0 |
| 80 | a | - | 0 |
| 81 | a | - | 0 |
| 82 | a | - | 0 |
| 83 | a | - | 0 |
| 84 | a | - | 0 |
| 85 | a | - | 0 |
| 86 | a | - | 0 |
| 87 | a | - | 0 |
| 88 | a | - | 0 |
| 89 | a | - | 0 |
| 90 | a | - | 0 |
| 91 | a | - | 0 |
| 92 | a | - | 0 |
| 93 | a | - | 0 |
| 94 | a | - | 0 |
| 95 | a | - | 0 |
| 96 | a | - | 0 |
| 97 | a | - | 8 |
| 98 | a | - | 13 |
| 99 | a | - | 0 |
| 100 | a | - | -7 |
| 101 | a | - | 5 |
| 102 | a | - | -1 |
| 103 | a | a | -9 |
| 104 | a | - | 3 |
| 105 | a | - | -3 |
| 106 | a | - | -1 |
| 107 | a | - | -2 |
| 108 | a | - | 0 |
| 109 | a | - | -11 |
| 110 | a | - | 0 |
| 111 | a | - | -23 |
| 112 | a | - | -8 |
| 113 | a | - | -14 |
| 114 | a | - | -3 |
| 115 | a | - | -8 |
| 116 | a | - | -1 |
| 117 | a | - | 0 |
| 118 | a | - | 11 |
| 119 | a | - | 11 |
| 120 | a | - | 5 |
| 121 | a | - | -6 |
| 122 | a | - | 0 |
| 123 | a | - | 3 |
| 124 | a | - | -2 |
| 125 | a | - | -8 |
| 126 | a | - | -8 |
| 127 | a | - | -3 |
| 128 | a | - | -8 |
| 129 | a | - | -5 |
| 130 | a | - | -2 |
| 131 | a | - | -2 |
| 132 | a | - | -4 |
| 133 | a | - | -6 |
| 134 | a | - | -2 |
| 135 | a | - | 3 |
| 136 | a | - | 10 |
| 137 | a | - | 8 |
| 138 | a | - | 9 |
| 139 | a | - | -10 |
| 140 | a | - | 6 |
| 141 | a | - | -3 |
| 142 | a | a | -32 |
| 143 | a | - | -1 |
| 144 | a | - | 2 |

### C. In Vivo Food Intake Models

### 1. Spontaneous Feeding Paradigm

Food intake in rats is measured after i.p., s.c. or p.o. administration of the test compound (see e.g. A.S. Chen et al. Transgenic Res 2000 Apr ;9(2):145-154).

### 2. Model of LPS-Induced Anorexia and Tumor-Induced Cachexia

Prevention or amelioration of anorexia induced by either lipopolysaccharide (LPS) administration or cachexia induced by tumor growth is determined upon i.p. or p.o. administration of test compounds to rats (see e.g. D.L. Marks, N. Ling, and R.D. Cone, Cancer Res 2001 Feb 15;61(4):1432-1438).

### D. Rat Ex Copula Assay

Sexually mature male Caesarian Derived Sprague Dawley (CD) rats (over 60 days old) are used with the suspensory ligament surgically removed to prevent retraction of the penis back into the penile sheath during the ex copula evaluations. Animals receive food and water ad lib and are kept on a normal light/dark cycle. Studies are conducted during the light cycle.

### 1. Conditioning to Supine Restraint for Ex Copula Reflex Tests

This conditioning takes about 4 days. Day 1, the animals are placed in a darkened restrainer and left for 15 - 30 minutes. Day 2, the animals are restrained in a supine position in the restrainer for 15 - 30 minutes. Day 3, the animals are restrained in the supine position with the penile sheath retracted for 15 - 30 minutes. Day 4, the animals are restrained in the supine position with the penile sheath retracted until penile responses are observed. Some animals require additional days of conditioning before they are completely acclimated to the procedures; non-responders are removed from further evaluation. After any handling or evaluation, animals are given a treat to ensure positive reinforcement.

### 2. Ex Copula Reflex Tests

Rats are gently restrained in a supine position with their anterior torso placed inside a cylinder of adequate size to allow for normal head and paw grooming. For a 400 - 500 gram rat, the diameter of the cylinder is approximately 8 cm. The lower torso and hind limbs are restrained with a nonadhesive material (vetrap). An additional piece of vetrap with a hole in it, through which the glans penis will be passed, is fastened over the animal to maintain the preputial sheath in a retracted position. Penile responses will be observed, typically termed ex copula genital reflex tests. Typically, a series of penile erections will occur spontaneously within a few minutes after sheath retraction. The types of normal reflexogenic erectile responses include elongation, engorgement, cup and flip. An elongation is classified as an extension of the penile body. Engorgement is a dilation of the glans penis. A cup is defined as an intense erection where the distal margin of the glans penis momentarily flares open to form a cup. A flip is a dorsiflexion of the penile body.

Baseline and or vehicle evaluations are conducted to determine how, and if, an animal will respond. Some animals have a long duration until the first response while others are non-responders altogether. During this baseline evaluation latency to first response, number and type of responses are recorded. The testing time frame is 15 minutes after the first response.

After a minimum of 1 day between evaluations, these same animals are administered the test compound at 20 mg/kg and evaluated for penile reflexes. All evaluations are videotaped and scored later. Data are collected and analyzed using paired 2 tailed t-tests to compared baseline and/or vehicle evaluations to drug treated evaluations for individual animals. Groups of a minimum of 4 animals are utilized to reduce variability.

Positive reference controls are included in each study to assure the validity of the study. Animals can be dosed by a number of routes of administration depending on the nature of the study to be performed. The routes of administration includes intravenous (IV), intraperitoneal (IP), subcutaneous (SC) and intracerebral ventricular (ICV).

### E. Models of Female Sexual Dysfunction

Rodent assays relevant to female sexual receptivity include the behavioral model of lordosis and direct observations of copulatory activity. There is also a urethrogenital reflex model in anesthetized spinally transected rats for measuring orgasm in both male and female rats. These and other established animal models of female sexual dysfunction are described in K.E. McKenna et al, A Model For The Study of Sexual Function In Anesthetized Male And Female Rats, Am. J. Physiol. (Regulatory Integrative Comp. Physiol 30): R1276-R1285, 1991; K.E. McKenna et al, Modulation By Peripheral Serotonin of The Threshold For Sexual Reflexes In Female Rats, Pharm. Bioch. Behav., 40:151-156, 1991; and L.K. Takahashi et al, Dual Estradiol Action In The Diencephalon And The Regulation of Sociosexual Behavior In Female Golden Hamsters, Brain Res., 359: 194-207, 1985.

### Examples of a Pharmaceutical Composition

As a specific embodiment of an oral composition of a compound of the present invention, 23 mg of Example 6 is formulated with sufficient finely divided lactose to provide a total amount of 580 to 590 mg to fill a size 0 hard gelatin capsule.

As another specific embodiment of an oral composition of a compound of the present invention, 28 mg of Example 14 is formulated with sufficient finely divided lactose to provide a total amount of 580 to 590 mg to fill a size 0 hard gelatin capsule.

While the invention has been described and illustrated in reference to certain preferred embodiments thereof, those skilled in the art will appreciate that various changes, modifications and substitutions can be made therein without departing from the spirit and scope of the invention. For example, effective dosages, other than the preferred doses as set forth above, may be applicable as a consequence of the specific pharmacological responses observed and may vary depending upon the particular active compound selected, as well as from the type of formulation and mode of administration employed, and such expected variations or differences in the results are contemplated in accordance with the objects and practices of the present invention. It is intended, therefore, that the invention be limited only by the scope of the claims which follow.

## Claims

1. A compound according to formula (I) 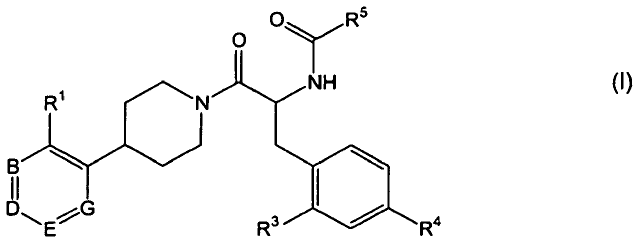 and the enantiomers, diastereomers, tautomers, solvates and pharmaceutically acceptable salts thereof,
wherein
R¹ is -N(R¹⁰)-(C(R⁶)₂)ₘ-T
-(C(R⁶)₂)ₗ-T, or
-O-(C(R⁶)₂)ₘ-T;
R⁶ is independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
T is NR⁷R⁸, 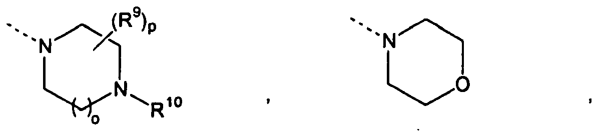 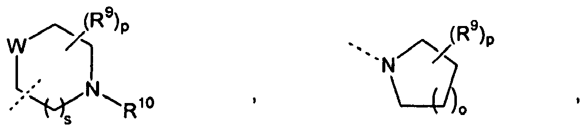 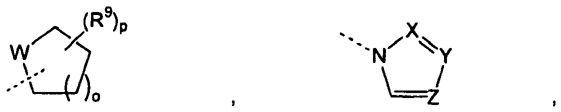 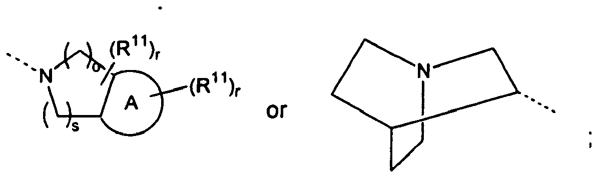
R⁷ and R⁸ are independently from each other selected from H,
C₁₋₆-alkyl,
C₂₋₆-alkenyl,
C₂₋₆-alkinyl, and
C₂₋₆-alkylene-O-C₁₋₆-alkyl,
wherein each alkyl, alkenyl and alkinyl is optionally substituted by one or more halogen atoms, CN or OH;
R⁹ is independently selected from
halogen,
CN,
OH,
C₁₋₆-alkyl, optionally substituted with 1 to 3 substituents selected from halogen, CN and OH, and O-C₁₋₆-alkyl, optionally substituted with 1 to 3 substituents selected from halogen, CN and OH,
C₁₋₆-alkylene-O-C₁₋₆-alkyl, optionally substituted with 1 to 3 substituents selected from halogen, CN and OH, or
NR¹²R¹³;
R¹⁰ is H, or C₁₋₆-alkyl;
R¹¹ is independently selected from
halogen,
CN,
OH,
C₁₋₆-alkyl, optionally substituted with 1 to 3 substituents selected from halogen, CN and OH,
C₂₋₆-alkenyl,
C₂₋₆-alkinyl,
O-C₁₋₆-alkyl, optionally substituted with 1 to 3 substituents selected from halogen, CN and OH,
C₁₋₆-alkylene-O-C₁₋₆-alkyl, optionally substituted with 1 to 3 substituents selected from halogen, CN and OH,
C₀₋₆-alkyl-C₃₋₆-cycloalkyl,
-OC(O)C₁₋₆-alkyl,
-NH₂,
-NH(C₁₋₆-alkyl), and
-N(C₁₋₆-alkyl)₂;
R¹² and R¹³ are independently from each other selected from
C₁₋₆-alkyl, optionally substituted with OH,
C₂₋₆-alkenyl,
C₂₋₆-alkinyl,
C₂₋₆-alkylene-O-C₁₋₆alkyl, and
C₂₋₆-alkylene-N-(C₁₋₆-alkyl)₂;
W is CH, O or NR¹⁰;
X is CH or N;
Y is CH or N;
Z is CH or N;
A is a 3-7-membered saturated, unsaturated or aromatic ring containing 0-2 nitrogen atoms;
B is CR² or N;
G is CR² or N;
D is CR² or N;
E is CR² or N;
with the proviso that one or two of the variables B, G, D and E must be N;
R² is independently selected from
H,
F,
Cl,
CH₃,
OCH₃, and
CF₃;
R³ is H, Cl, F, or CH₃;
R⁴ is Cl, F or CH₃;
R⁵ is 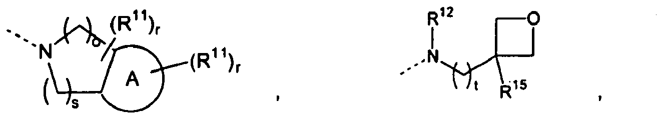 morpholine, optionally substituted by 1 to 3 same or different substituents R¹⁴, 4 to 7 membered, saturated or partially unsaturated heterocycle containing in the ring one nitrogen atom and optionally a further heteroatom selected from O, N and S, wherein heterocycle is optionally substituted by 1 to 4 same or different substituents R¹¹, or
NR¹²R¹³;
R¹⁴ is C₁₋₆-alkyl,
C₁₋₆-alkylene-O-C₁₋₆-alkyl,
C₁₋₆-alkylene-OH,
C₁₋₆-alkylene-NH₂,
C₁₋₆-alkylene-NH-C₁₋₆-alkyl, or
C₁₋₆-alkylene-N(C₁₋₆-alkyl)₂;
R¹⁵ is H or C₁₋₆-alkyl;
I is 0, 1, 2, 3, or 4;
m is 0, 1, 2, 3, or 4;
o is 0, 1, or 2;
p is 0, 1, 2, 3, or 4;
r is 0, 1, 2, 3, or 4;
s is 1, or 2 and
t is 0 or 1.

2. A compound according to claim 1, wherein
R¹ is -N(R¹⁰)-(C(R⁶)₂)ₘ-T
-(C(R⁶)₂)ₗ-T, or
-O-(C(R⁶)₂)ₘ-T;
R⁶ is independently selected from
H,
F,
OH, OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
T is NR⁷R⁸, morpholine, 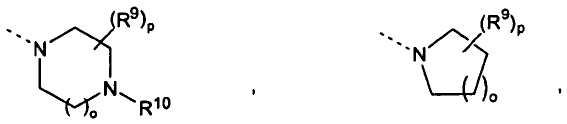 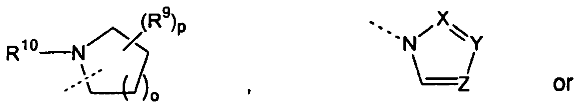 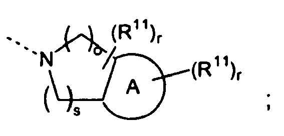
R⁷ and R⁸ are independently from each other selected from
H,
C₁₋₆-alkyl,
C₂₋₆-alkenyl,
C₂₋₆-alkinyl, and
C₂₋₆-alkylene-O-C₁₋₆-alkyl,
wherein each alkyl, alkenyl and alkinyl is optionally substituted by one or more halogen atoms, CN or OH;
R⁹ is independently selected from
halogen,
CN,
OH, C₁₋₆-alkyl optionally substituted with 1 to 3 substituents selected from halogen, CN and OH, and
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH;
R¹⁰ is H, or
C₁-C₆-alkyl;
R¹¹ is independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
-NH₂,
-NH(C₁₋₆-alkyl), and
-N(C₁₋₆-alkyl)₂;
X is CH or N;
Y is CH or N;
Z is CH or N;
A is a 3-7-membered saturated, unsaturated or aromatic ring containing 0-2 nitrogen atoms;
B is CR² or N;
G is CR² or N;
D is CR² or N;
E is CR² or N;
with the proviso that one or two of the variables B, G, D and E must be N;
R² is independently selected from
H,
F,
Cl,
CH₃,
OCH₃, and
CF₃;
R³ is H,
Cl,
F, or CH₃;
R⁴ is Cl or F;
R⁵ is 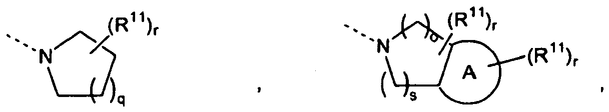 morpholine, optionally substituted by 1 to 3, same or different substituents R¹⁴, or NR¹²R¹³;
R¹² and R¹³ are independently from each other selected from
C₁₋₆-alkyl,
C₂₋₆-alkenyl,
C₂₋₆-alkinyl,
C₂₋₆-alkylene-O-C₁₋₆-alkyl, and
C₂₋₆-alkylene-N-(C₁₋₆-alkyl)₂;
R¹⁴ is C₁₋₆-alkyl,
C₁₋₆-alkylene-O-C₁₋₆-alkyl,
C₁₋₆-alkylene-OH,
C₁₋₆-alkylene-NH₂, C₁₋₆-alkylene-NH-C₁₋₆-alkyl, or
C₁₋₆-alkylene-N(C₁₋₆-alkyl)₂;
I is 0, 1, 2, 3, or 4;
m is 0, 1, 2, 3, or 4;
o is 0, 1, or 2;
p is 0, 1, 2, 3, or 4;
q is 0, 1, 2, or 3;
r is 0, 1, 2, 3, or 4 and
s is 1, or 2.

3. The compound of claim 1 or 2 according to formula (I') 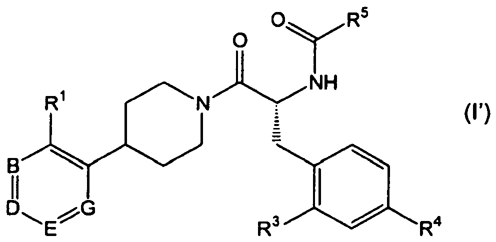 wherein B, G, D, E, R¹, R³, R⁴ and R⁵ are as defined in claim 1 or 2.

4. The compound of any of claims 1 to 3, wherein at least one of R⁷ and R⁸ is selected from C₂₋₆-alkenyl,
C₂₋₆-alkinyl, and
C₂₋₆-alkylene-O-C₁₋₆-alkyl.

5. The compound of any of claims 1 to 4, wherein
R² is selected from H, F, Cl and CH₃.

6. The compound of any of claims 1 to 5 wherein
I is 2 or 3, or
m is 2 or 3.

7. The compound of any of claims 1 to 6 as medicament.

8. The compound any of claims 1 to 6 for the treatment or prophylaxis of cancer cachexia, muscle wasting, anorexia, amyotrophic lateral sclerosis (ALS), anxiety and/or depression.

9. The compound any of claims 1 to 6 for the treatment or prophylaxis of obesity, diabetes mellitus, male or female sexual dysfunction and/or erectile dysfunction.

10. Use of the compound any of claims 1 to 6 for the preparation of a medicament for the treatment or prophylaxis of cancer cachexia, muscle wasting, anorexia, amyotrophic lateral sclerosis (ALS), anxiety and/or depression.

11. Use of a compound any of claims 1 to 6 for the preparation of a medicament for the treatment or prophylaxis of obesity, diabetes mellitus, male or female sexual dysfunction and/or erectile dysfunction.

12. A pharmaceutical composition comprising a compound of any of claims 1 to 6 and a pharmaceutically acceptable carrier.

## Patentansprüche

1. Verbindung gemäß Formel (I) 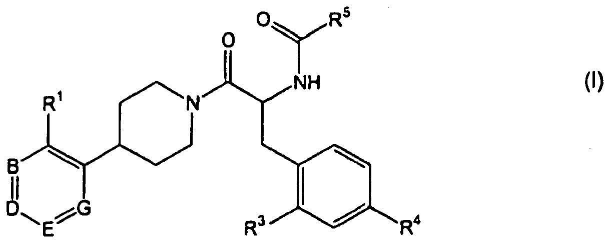 und die Enantiomere, Diastereomere, Tautomere, Solvate und pharmazeutisch verträglichen Salze davon,
wobei
R¹ ist -N(R¹⁰)-(C(R⁶)₂)ₘ-T
-(C(R⁶)₂)ₗ-T oder
-O-(C(R⁶)₂)ₘ-T;
R⁶ ist unabhängig ausgewählt aus
H,
F,
OH,
OCH₃,
C₁₋₆-Alkyl, gegebenenfalls substituiert mit 1 bis 3 Substituenten, ausgewählt aus Halogen, CN, OH und OCH₃, und
C₃₋₆-Cycloalkyl, gegebenenfalls substituiert mit 1 bis 3 Substituenten, ausgewählt aus Halogen, CN, OH und OCH₃;
T ist NR⁷R⁸, 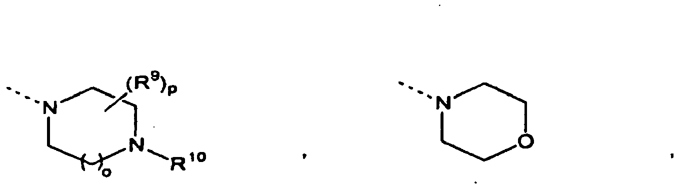 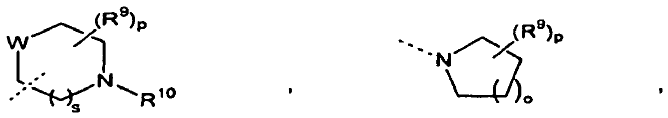 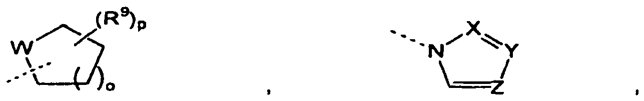 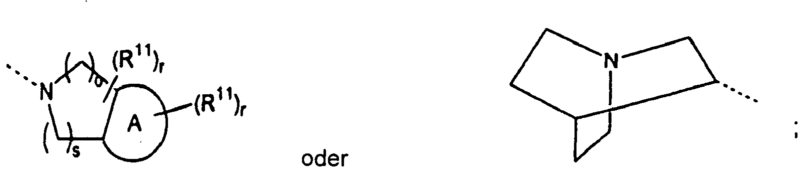
R⁷ und R⁸ sind unabhängig voneinander ausgewählt aus
H,
C₁₋₆-Alkyl,
C₂₋₆-Alkenyl,
C₂₋₆-Alkinyl und
C₂₋₆-Alkylen-O-C₁₋₆-alkyl,
wobei jedes Alkyl, Alkenyl und Alkinyl gegebenenfalls substituiert ist durch ein oder mehrere Halogenatome, CN oder OH;
R⁹ ist unabhängig ausgewählt aus
Halogen,
CN,
OH,
C₁₋₆-Alkyl, gegebenenfalls substituiert mit 1 bis 3 Substituenten, ausgewählt aus Halogen, CN und OH, und O-C₁₋₆-Alkyl, gegebenenfalls substituiert mit 1 bis 3 Substituenten, ausgewählt aus Halogen, CN und OH,
C₁₋₆-Alkylen-O-C₁₋₆-alkyl, gegebenenfalls substituiert mit 1 bis 3 Substituenten, ausgewählt aus Halogen, CN und OH, oder NR¹²R¹³;
R¹⁰ ist H oder C₁₋₆-Alkyl;
R¹¹ ist unabhängig ausgewählt aus
Halogen,
CN,
OH,
C₁₋₆-Alkyl, gegebenenfalls substituiert mit 1 bis 3 Substituenten, ausgewählt aus Halogen, CN und OH,
C₂₋₆-Alkenyl,
C₂₋₆-Alkinyl,
O-C₁₋₆-Alkyl, gegebenenfalls substituiert mit 1 bis 3 Substituenten, ausgewählt aus Halogen, CN und OH,
C₁₋₆-Alkylen-O-C₁₋₆-alkyl, gegebenenfalls substituiert mit 1 bis 3 Substituenten, ausgewählt aus Halogen, CN und OH,
C₀₋₆-Alkyl-C₃₋₆-cycloalkyl,
-OC(O)C₁₋₆-Alkyl,
-NH₂,
-NH(C₁₋₆-Alkyl) und
-N(C₁₋₆-Alkyl)₂;
R¹² und R¹³ sind unabhängig voneinander ausgewählt aus
C₁₋₆-Alkyl, gegebenenfalls substituiert mit OH,
C₂₋₆-Alkenyl,
C₂₋₆-Alkinyl, C₂₋₆-Alkylen-O-C₁₋₆-alkyl und
C₂₋₆-Alkylen-N-(C₁₋₆-alkyl)₂;
W ist CH, O oder NR¹⁰;
X ist CH oder N;
Y ist CH oder N;
Z ist CH oder N;
A ist ein 3-7-gliedriger gesättigter, ungesättigter oder aromatischer Ring, enthaltend 0-2 Stickstoffatome;
B ist CR² oder N;
G ist CR² oder N;
D ist CR² oder N;
E ist CR² oder N;
mit der Maßgabe, dass eine oder zwei der Variablen B, G, D und E N sein muss;
R² ist unabhängig ausgewählt aus
H,
F,
Cl,
CH₃,
OCH₃ und
CF₃;
R³ ist H,
Cl,
F oder
CH₃;
R⁴ ist Cl,
F oder
CH₃;
R⁵ ist 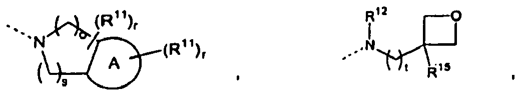 Morpholin, gegebenenfalls substituiert mit 1 bis 3 gleichen oder unterschiedlichen Substituenten R¹⁴,
ein 4 bis 7 gliedriger, gesättigter oder teilweise ungesättigter Heterocyclus, der in dem Ring ein Stickstoffatom und gegebenenfalls ein weiteres Heteroatom, ausgewählt aus O, N und S, enthält, wobei der Heterocyclus gegebenenfalls mit 1 bis 4 gleichen oder unterschiedlichen Substituenten R¹¹ substituiert ist, oder
NR¹²R¹³;
R¹⁴ ist C₁₋₆-Alkyl,
C₁₋₆-Alkylen-O-C₁₋₆-alkyl,
C₁₋₆-Alkylen-OH,
C₁₋₆-Alkylen-NH₂,
C₁₋₆-Alkylen-NH-C₁₋₆-alkyl oder
C₁₋₆-Alkylen-N(C₁₋₆-alkyl)₂;
R¹⁵ ist H oder
C₁₋₆-Alkyl;
I ist 0, 1, 2, 3 oder 4;
m ist 0, 1, 2, 3 oder 4;
o ist 0, 1 oder 2;
p ist 0, 1, 2, 3 oder 4;
r ist 0, 1, 2, 3 oder 4;
s ist 1 oder 2 und
t ist 0 oder 1.

2. Verbindung nach Anspruch 1, wobei R¹ ist -N(R¹⁰)-(C(R⁶)₂)ₘ-T
-(C(R⁶)₂)ₗ-T oder
-O-(C(R⁶)₂)ₘ-T;
R⁶ ist unabhängig ausgewählt aus
H,
F,
OH,
OCH₃,
C₁₋₆-Alkyl, gegebenenfalls substituiert mit 1 bis 3 Substituenten, ausgewählt aus Halogen, CN, OH und OCH₃, und
C₃₋₆-Cycloalkyl, gegebenenfalls substituiert mit 1 bis 3 Substituenten, ausgewählt aus Halogen, CN, OH und OCH₃;
T ist NR⁷R⁸,
Morpholin, 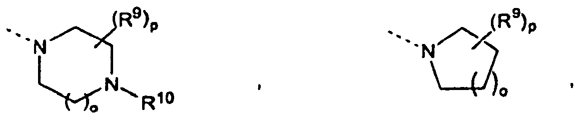 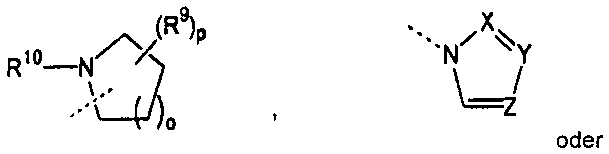 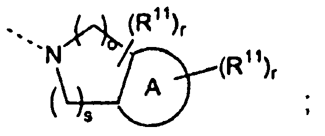
R⁷ und R⁸ sind unabhängig voneinander ausgewählt aus
H,
C₁₋₆-Alkyl,
C₂₋₆-Alkenyl,
C₂₋₆-Alkinyl und C₂₋₆-Alkylen-O-C₁₋₆-alkyl,
wobei jedes Alkyl, Alkenyl und Alkinyl gegebenenfalls substituiert ist durch ein oder mehrere Halogenatome, CN oder OH;
R⁹ ist unabhängig ausgewählt aus
Halogen,
CN,
OH,
C₁₋₆-Alkyl, gegebenenfalls substituiert mit 1 bis 3 Substituenten, ausgewählt aus Halogen, CN und OH, und
O-C₁₋₆-Alkyl, gegebenenfalls substituiert mit 1 bis 3 Substituenten, ausgewählt aus Halogen, CN und OH,
C₁₋₆-Alkylen-O-C₁₋₆-alkyl, gegebenenfalls substituiert mit 1 bis 3 Substituenten, ausgewählt aus Halogen, CN und OH;
R¹⁰ ist H oder C₁-C₆-Alkyl;
R¹¹ ist unabhängig ausgewählt aus
Halogen,
CN,
OH,
C₁₋₆-Alkyl, gegebenenfalls substituiert mit 1 bis 3 Substituenten, ausgewählt aus Halogen, CN und OH,
O-C₁₋₆-Alkyl, gegebenenfalls substituiert mit 1 bis 3 Substituenten, ausgewählt aus Halogen, CN und OH,
C₁₋₆-Alkylen-O-C₁₋₆-alkyl, gegebenenfalls substituiert mit 1 bis 3 Substituenten, ausgewählt aus Halogen, CN und OH,
-NH₂,
-NH(C₁₋₆-Alkyl) und
-N(C₁₋₆-Alkyl)₂;
X ist CH oder N;
Y ist CH oder N;
Z ist CH oder N;
A ist ein 3-7-gliedriger gesättigter, ungesättigter oder aromatischer Ring, enthaltend 0-2 Stickstoffatome;
B ist CR² oder N;
G ist CR² oder N;
D ist CR² oder N;
E ist CR² oder N;
mit der Maßgabe, dass eine oder zwei der Variablen B, G, D und E N sein muss;
R² ist unabhängig ausgewählt aus
H,
F,
Cl,
CH₃,
OCH₃ und
CF₃;
R³ ist H,
Cl,
F oder
CH₃;
R⁴ ist Cl oder F;
R⁵ ist 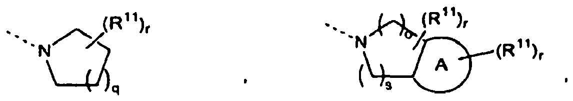 Morpholin, gegebenenfalls substituiert mit 1 bis 3 gleichen oder unterschiedlichen Substituenten R¹⁴, oder NR¹²R¹³;
R¹² und R¹³ sind unabhängig voneinander ausgewählt aus
C₁₋₆-Alkyl,
C₂₋₆-Alkenyl,
C₂₋₆-Alkinyl,
C₂₋₆-Alkylen-O-C₁₋₆-alkyl und
C₂₋₆-Alkylen-N-(C₁₋₆-alkyl)₂;
R¹⁴ ist C₁₋₆-Alkyl,
C₁₋₆-Alkylen-O-C₁₋₆-alkyl,
C₁₋₆-Alkylen-OH,
C₁₋₆-Alkylen-NH₂,
C₁₋₆-Alkylen-NH-C₁₋₆-alkyl oder
C₁₋₆-Alkylen-N(C₁₋₆-alkyl)₂;
l ist 0, 1, 2, 3 oder 4;
m ist 0, 1, 2, 3 oder 4;
o ist 0, 1 oder 2;
p ist 0, 1, 2, 3 oder 4;
q ist 0, 1, 2 oder 3;
r ist 0, 1, 2, 3 oder 4 und
s ist 1 oder 2.

3. Verbindung nach Anspruch 1 oder 2 gemäß Formel (I') 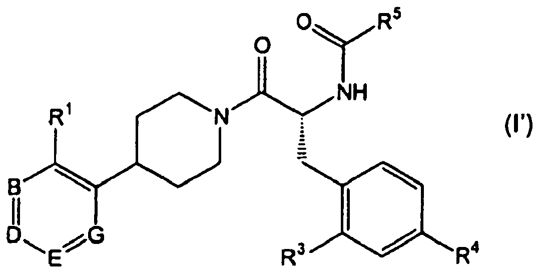 wobei B, G, D, E, R¹, R³, R⁴ und R⁵ wie in Anspruch 1 oder 2 definiert sind.

4. Verbindung nach einem der Ansprüche 1 bis 3, wobei wenigstens eines von R⁷ und R⁸ ausgewählt ist aus
C₂₋₆-Alkenyl,
C₂₋₆-Alkinyl und
C₂₋₆-Alkylen-O-C₁₋₆-alkyl.

5. Verbindung nach einem der Ansprüche 1 bis 4, wobei
R₂ ausgewählt ist aus H, F, Cl und CH₃.

6. Verbindung nach einem der Ansprüche 1 bis 5, wobei
I ist 2 oder 3, oder
m ist 2 oder 3.

7. Verbindung nach einem der Ansprüche 1 bis 6 als Medikament.

8. Verbindung nach einem der Ansprüche 1 bis 6 für die Behandlung oder Prophylaxe von Krebskachexie, Muskelschwund, Anorexie, amyotropher Lateralsklerose (ALS), Angst und/oder Depression.

9. Verbindung nach einem der Ansprüche 1 bis 6 für die Behandlung oder Prophylaxe von Fettleibigkeit, Diabetes mellitus, männlicher oder weiblicher sexueller Dysfunktion und/oder erektiler Dysfunktion.

10. Verwendung der Verbindung nach einem der Ansprüche 1 bis 6 für die Herstellung eines Medikaments für die Behandlung oder Prophylaxe von Krebskachexie, Muskelschwund, Anorexie, amyotropher Lateralsklerose (ALS), Angst und/oder Depression.

11. Verwendung einer Verbindung nach einem der Ansprüche 1 bis 6 für die Herstellung eines Medikaments für die Behandlung oder Prophylaxe von Fettleibigkeit, Diabetes mellitus, männlicher oder weiblicher sexueller Dysfunktion und/oder erektiler Dysfunktion.

12. Pharmazeutische Zusammensetzung, umfassend eine Verbindung nach einem der Ansprüche 1 bis 6 und einen pharmazeutisch verträglichen Träger.

## Revendications

1. Composé de formule (I) : 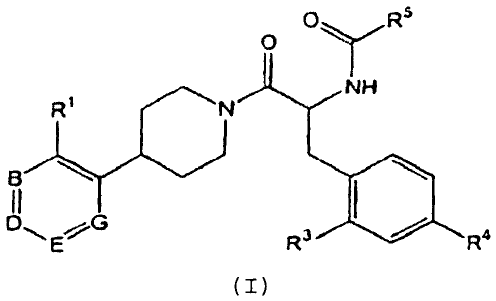 et ses énantiomères, diastéréomères, tautomères, solvates et sels pharmaceutiquement acceptables,
formule dans laquelle
R¹ est
-N(R¹⁰)-(C(R⁶)₂)ₘ-T
-(C(R⁶)₂)ₗ-T, ou
-O-(C(R⁶)₂)ₘ-T;
R⁶ est indépendamment choisi parmi
H,
F,
OH,
OCH₃,
alkyle en C₁ à C₆, éventuellement substitué par 1 à 3 substituants choisis parmi les halogènes, CN, OH et OCH₃, et
cycloalkyle en C₃ à C₆, éventuellement substitué par 1 à 3 substituants choisis parmi les halogènes, CN, OH et OCH₃ ;
T est NR⁷R⁸, 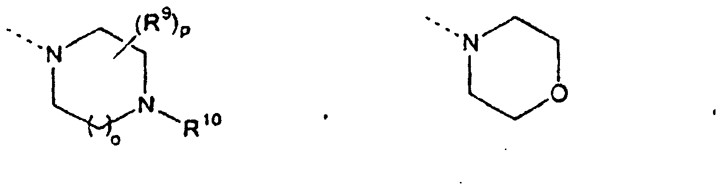 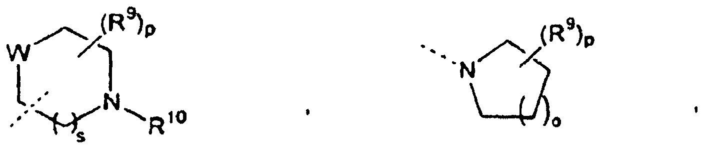 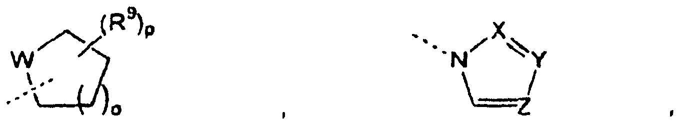 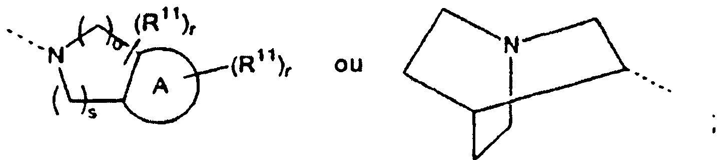
R⁷ et R⁸, indépendamment l'un de l'autre, sont choisis
parmi n H,
alkyle en C₁ à C₆,
alcényle en C₂ à C₆,
alcynyle en C₂ à C₆, et
(alkylène en C₂ à C₆)-O-alkyle en C₁ à C₆,
où chaque alkyle, alcényle et alcynyle est éventuellement substitué par un ou plusieurs atomes d'halogène, CN ou OH ;
R⁹ est indépendamment choisi parmi
les halogènes,
CN,
OH,
alkyle en C₁ à C₆, éventuellement substitué par 1 à 3 substituants choisis parmi les halogènes, CN et OH,
et
O-alkyle en C₁ à C₆, éventuellement substitué par 1 à 3 substituants choisis parmi les halogènes, CN et OH, (alkylène en C₁ à C₆) -O-alkyle en C₁ à C₆, éventuellement substitué par 1 à 3 substituants
R¹⁰ est choisis parmi les halogènes, CN et OH, ou
NR¹²R¹³;
H, ou
alkyle en C₁ à C₆ ;
R¹¹ est indépendamment choisi parmi
les halogènes,
CN,
OH,
alkyle en C₁ à C₆, éventuellement substitué par 1 à 3 substituants choisis parmi les halogènes, CN et OH,
alcényle en C₂ à C₆,
alcynyle en C₂ à C₆,
O-alkyle en C₁ à C₆, éventuellement substitué par 1 à 3 substituants choisis parmi les halogènes, CN et OH,
(alkylène en C₁ à C₆) -O-alkyle en C₁ à C₆,
éventuellement substitué par 1 à 3 substituants choisis parmi les halogènes, CN et OH,
(alkyle en C₀ à C₆) -cycloalkyle en C₃ à C₆,
-OC(O)-alkyle en C₁ à C₆,
-NH₂,
-NH(alkyle en C₁ à C₆), et
-N (alkyle en C₁ à C₆)₂ ;
R¹² R¹³, et indépendamment l'un de l'autre, sont choisis parmialkyle en C₁ à C₆, éventuellement substitué par OH,
alcényle en C₂ à C₆,
alcynyle en C₂ à C₆,
(alkylène en C₂ à C₆) -O-alkyle en C₁ à C₆, et
(alkylène en C₂ à C₆)-N-(alkyle en C₁ à C₆)₂ ;
W est CH, O ou NR¹⁰ ;
X est CH ou N ;
Y est CH ou N ;
Z est CH ou N ;
A est un cycle saturé, insaturé ou aromatique, à 3 à 7 chaînons, contenant 0 à 2 atomes d'azote ;
B est CR² ou N ;
G est CR² ou N ;
D est CR² ou N ;
E est CR² ou N ;
à la condition qu'une ou deux des variables B, G, D et E doivent être N ;
R² est indépendamment choisi parmi H, F, Cl, CH₃, OCH₃, et CF₃ ;
R³ est H, Cl, F, ou CH₃ ;
R⁴ est Cl, F ou CH₃ ;
R⁵ est 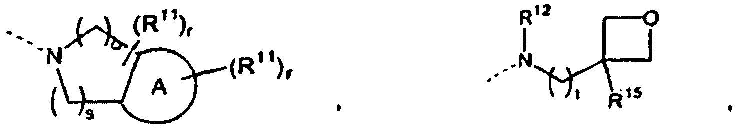 morpholine, éventuellement substituée par 1 à 3 substituants R¹⁴ identiques ou différents,
un hétérocycle saturé ou partiellement insaturé à 4 à 7 chaînons contenant dans le cycle un atome d'azote et éventuellement un autre hétéroatome choisi parmi O, N et S, lequel hétérocycle est éventuellement substitué par 1 à 4 substituants R¹¹ identiques ou différents, ou
NR¹²R¹³ ;
R¹⁴ est
alkyle en C₁ à C₆,
(alkylène en C₁ à C₆)-O-alkyle en C₁ à C₆,
(alkylène en C₁ à C₆)-OH,
(alkylène en C₁ à C₆)-NH₂,
(alkylène en C₁ à C₆)-NH-alkyle en C₁ à C₆, ou
(alkylène en C₁ à C₆)-N(alkyle en C₁ à C₆)₂ ;
R¹⁵ est
H ou
alkyle en C₁ à C₆ ;
l vaut 0, 1, 2, 3 ou 4 ;
m vaut 0, 1, 2, 3 ou 4 ;
o vaut 0 , 1 ou 2 ;
p vaut 0, 1, 2, 3 ou 4 ;
r vaut 0, 1, 2, 3 ou 4 ;
s vaut 1 ou 2 et
t vaut 0 ou 1.

2. Composé selon la revendication 1, dans lequel
R¹ est
-N(R¹⁰)-(C(R⁶)₂)ₘ-T
-(C(R⁶)₂)ₗ-T, ou
-O-(C(R⁶)₂)ₘ-T ;
R⁶ est indépendamment choisi parmi
H,
F,
OH,
OCH₃,
alkyle en C₁ à C₆, éventuellement substitué par 1 à 3 substituants choisis parmi les halogènes, CN, OH et OCH₃, et
cycloalkyle en C₃ à C₆, éventuellement substitué par 1 à 3 substituants choisis parmi les halogènes, CN, OH et OCH₃ ;
T est NR⁷R⁸,
morpholine, 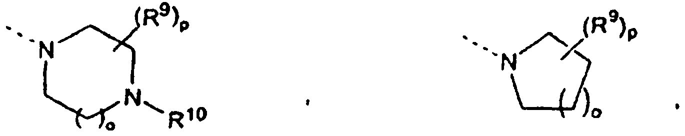 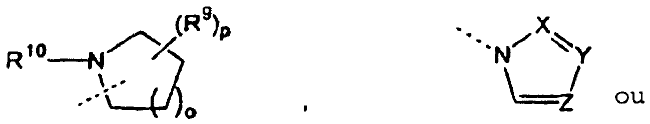 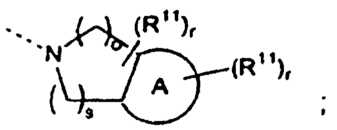
R⁷ et R⁸, indépendamment l'un de l'autre, sont choisis parmiH,
alkyle en C₁ à C₆,
alcényle en C₂ à C₆,
alcynyle en C₂ à C₆, et
(alkylène en C₂ à C₆)-O-alkyle en C₁ à C₆,
où chaque alkyle, alcényle et alcynyle est éventuellement substitué par un ou plusieurs atomes d'halogène, CN ou OH ;
R⁹ est indépendamment choisi parmi les halogènes,
CN,
OH,
alkyle en C₁ à C₆, éventuellement substitué par 1 à 3 substituants choisis parmi les halogènes, CN et OH,
et
O-alkyle en C₁ à C₆, éventuellement substitué par 1 à 3 substituants choisis parmi les halogènes, CN et OH,
(alkylène en C₁ à C₆) -O-alkyle en C₁ à C₆ éventuellement substitué par 1 à 3 substituants choisis parmi les halogènes, CN et OH ;
R¹⁰ est
H, ou
alkyle en C₁ à C₆ ;
R¹¹ est indépendamment choisi parmi les halogènes,
CN,
OH,
alkyle en C₁ à C₆ éventuellement substitué par 1 à 3 substituants choisis parmi les halogènes, CN et OH,
O-alkyle en C₁ à C₆ éventuellement substitué par 1 à 3 substituants choisis parmi les halogènes, CN et OH,
(alkylène en C₁ à C₆) -O-alkyle en C₁ à C₆ éventuellement substitué par 1 à 3 substituants choisis parmi les halogènes, CN et OH,
-NH₂,
-NH (alkyle en C₁ à C₆), et
-N (alkyle en C₁ à C₆)₂ ; X est CH ou N ;
Y est CH ou N ;
Z est CH ou N ;
A est un cycle saturé, insaturé ou aromatique, à 3 à 7 chaînons, contenant 0 à 2 atomes d'azote ;
B est CR² ou N ;
G est CR² ou N ;
D est CR² ou N ;
E est CR² ou N ;
à la condition qu'une ou deux des variables B, G, D et E doivent être N ;
R² est indépendamment choisi parmi H,
F,
Cl,
CH₃,
OCH₃, et CF₃ ;
R³ est
H,
Cl,
F, ou
CH₃ ;
R⁴ est Cl ou F ;
R⁵ est 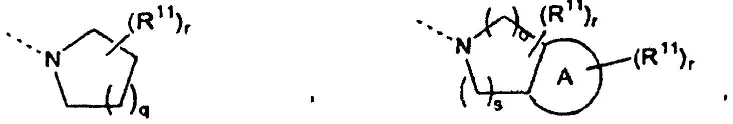 morpholine, éventuellement substituée par 1 à 3 substituants R¹⁴ identiques ou différents, ou NR¹²R¹³;
R¹² et R¹³, indépendamment l'un de l'autre, sont choisis parmialkyle en C₁ à C₆,
alcényle en C₂ à C₆,
alcynyle en C₂ à C₆,
(alkylène en C₂ à C₆)-O-alkyle en C₁ à C₆, et
(alkylène en C₂ à C₆)-N-(alkyle en C₁ à C₆)₂;
R¹⁴ est
alkyle en C₁ à C₆,
(alkylène en C₁ à C₆) -O-alkyle en C₁ à C₆,
(alkylène en C₁ à C₆)-OH,
(alkylène en C₁ à C₆)-NH₂,
(alkylène en C₁ à C₆)-NH-alkyle en C₁ à C₆, ou
(alkylène en C₁ à C₆)-N(alkyle en C₁ à C₆)₂ ;
l vaut 0, 1, 2, 3 ou 4 ;
m vaut 0, 1, 2, 3 ou 4 ;
o vaut 0 , 1 ou 2 ;
p vaut 0, 1, 2, 3 ou 4 ;
q vaut 0, 1, 2 ou 3 ;
r vaut 0, 1, 2, 3 ou 4 ; et
s vaut 1 ou 2.

3. Composé selon la revendication 1 ou 2 de formule (I') : 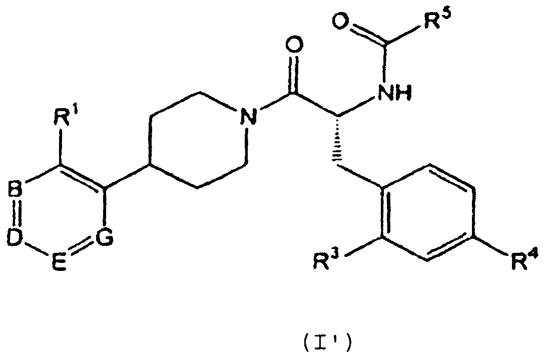 dans laquelle B, G, D, E, R¹, R³, R⁴ et R⁵ sont tels que définis dans la revendication 1 ou 2.

4. Composé selon l'une quelconque des revendications 1 à 3, dans lequel au moins l'un de R⁷ et R⁸ est choisi parmi
alcényle en C₂ à C₆,
alcynyle en C₂ à C₆, et
(alkylène en C₁ à C₆)-O-alkyle en C₁ à C₆.

5. Composé selon l'une quelconque des revendications 1 à 4, dans lequel R² est choisi parmi H, F, Cl et CH₃.

6. Composé selon l'une quelconque des revendications 1 à 5, dans lequel
1 vaut 2 ou 3, ou
m vaut 2 ou 3.

7. Composé selon l'une quelconque des revendications 1 à 6, en tant que médicament.

8. Composé selon l'une quelconque des revendications 1 à 6, pour le traitement ou la prophylaxie d'une cachexie cancéreuse, une atrophie musculaire, une anorexie, une sclérose latérale amyotrophique (SLA), une anxiété et/ou une dépression.

9. Composé selon l'une quelconque des revendications 1 à 6, pour le traitement ou la prophylaxie de l'obésité, du diabète sucré, d'un dysfonctionnement sexuel mâle ou femelle et/ou d'un dysfonctionnement érectile.

10. Utilisation du composé de l'une quelconque des revendications 1 à 6 pour la préparation d'un médicament pour le traitement ou la prophylaxie d'une cachexie cancéreuse, une atrophie musculaire, une anorexie, une sclérose latérale amyotrophique (SLA), une anxiété et/ou une dépression.

11. Utilisation du composé de l'une quelconque des revendications 1 à 6 pour la préparation d'un médicament pour le traitement ou la prophylaxie de l'obésité, du diabète sucré, d'un dysfonctionnement sexuel mâle ou femelle et/ou d'un dysfonctionnement érectile.

12. Composition pharmaceutique comprenant un composé de l'une quelconque des revendications 1 à 6 et un véhicule pharmaceutiquement acceptable.
